(19) Europäisches Patentamt
European Patent Office
Office européen des brevets

(11) Publication number: 0 276 065 B1

(12) # EUROPEAN PATENT SPECIFICATION

(45) Date of publication of the patent specification: **06.06.90**

(21) Application number: **88300163.8**

(22) Date of filing: **11.01.88**

(51) Int. Cl.5: **C07C 49/84,** C07C 59/92, C07C 65/40, C07C 69/738, C07C 69/76, C07C 217/02, C07C 235/18, C07C 235/28, C07C 245/00, C07C 255/11

(54) **Anti-inflammatory agents.**

(30) Priority: **12.01.87 US 2542**

(43) Date of publication of application: **27.07.88 Bulletin 88/30**

(45) Publication of the grant of the patent: **06.06.90 Bulletin 90/23**

(84) Designated Contracting States: **AT BE CH DE ES FR GB GR IT LI LU NL SE**

(56) References cited:
**EP-A- 0 051 819**
**EP-A- 0 108 592**
**DE-A- 1 493 729**
**DE-A- 2 244 324**
**DE-A- 3 312 675**
**US-A- 4 166 819**

(73) Proprietor: **ELI LILLY AND COMPANY, Lilly Corporate Center, Indianapolis Indiana 46285(US)**

(72) Inventor: **Bollinger, Nancy Grace, 7601 East 80th Street, Indianapolis Indiana 46256(US)**
Inventor: **Goodson, Theodore, Jr., 4045 Devon Drive, Indianapolis Indiana 46226(US)**
Inventor: **Herron, David Kent, 5945 Andover Road, Indianapolis Indiana 46220(US)**

(74) Representative: **Tapping, Kenneth George et al, Erl Wood Manor, Windlesham Surrey, GU20 6PH(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

This invention relates to novel leukotriene antagonists. The present leukotriene antagonists possess an unusual 2,4,5-substitution pattern (versus the standard 2,3,4 pattern) on the phenolic ring. The present antagonist compounds are not only effective as antagonists of the SRS-A leukotrienes ($LTC_4$, $LTD_4$, and LTE) but also leukotriene $LTB_4$. $LTB_4$ has been indicated as a causitive agent in inflammatory diseases such as psoriasis and inflammatory bowel disease.

Research in the area of allergic reactions of the lung has provided evidence that arachidonic acid derivatives formed by the action of lipoxygenases are related to various disease states. Some of these arachidonic acid metabolites have been classified as members of a family of eicosatetraenoic acids termed leukotrienes. Three of these substances (i.e., leukotriene $C_4$, $D_4$ and $E_4$) are currently thought to be major components of what has been previously called slow reacting substance of anaphylaxis (SRS-A).

Leukotriene $B_4$ ($LTB_4$) is a proinflammatory lipid which has been implicated in the pathogenesis of psoriasis, arthritis, chronic lung diseases, inflammatory bowel diseases, and other inflammatory states characterized by the infiltration and aggregation of polymorphonuclear leukocytes. Thus aggregated, the polymorphonuclear leukocytes liberate tissue-degrading enzymes and reactive chemicals causing the inflammation.

Antagonism of $LTB_4$ should therefore provide a novel therapeutic approach to treatment of these conditions.

There are many compounds that antagonize the effects of the sulfidopeptidoleukotriene $LTC_4$, $LTD_4$ and $LTE_4$. See, for example, Marshall et al., U.S. Patent No. 4 661 505, and R.D. Dillard, European Patent Application No. 132 366. The compounds of these references, as well as the many other compounds in this field, are limited to or clearly prefer a 2,3,4-substitution pattern on the phenol ring. In fact, substitution on the 5-position of the phenol ring was thought to greatly diminish or eliminate the $LTD_4$ antagonist activity of the compounds.

Much to our surprise, we have found that the present 2,4,5-substituted phenolic compounds not only $LTD_4$ antagonists, but a good many of the claimed compounds are also $LTB_4$ antagonists. $LTB_4$ is an important causitive agent in chronic inflammatory disease such as psoriasis and inflammatory bowel disease (IBD). Adequate therapy presently does not exist for either psoriasis or IBD.

Accordingly, the novel 2,4,5-substituted phenolic leukotriene antagonists of the present invention can be used in the treatment of inflammation.

Also, most of the compounds are $LTB_4$ antagonists and should therefore also be useful in the treatment of conditions such as psoriasis, inflammatory bowel disease, and allergic disorders such as asthma, where leukotrienes are thought to be causal mediators.

Specifically, this invention provides compounds of Formula I

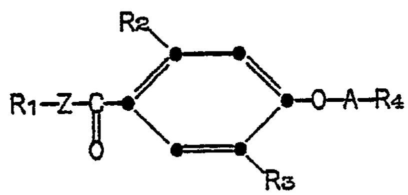

I

and pharmaceutically acceptable salts thereof, wherein

$R_1$ is hydrogen or R'OOC–;

Z is $-(CH_2)_n-$ or phenylene;

n is 1–8;

$R_2$ is hydroxy, halo, or $-O-(CH_2)_m-Y$;

$R_3$ is $C_1-C_6$ alkyl, $C_1-C_6$ alkanoyl, $C_2-C_4$ alkenyl, $C_1-C_4$ alkoxy, hydroxy-substituted $C_1-C_3$ alkyl, or $-CH_2-D$;

A is a bond or straight or branched chain $C_1-C_{10}$ alkylidene;

$R_4$ is $C_1-C_6$ alkyl, $C_2-C_6$ alkenyl, $C_2-C_6$ alkynyl, 1,2,4-triazol-1-yl, hydroxy, –CN, halo, $-N_3$, $-NR_5R_6$, $-COR_7$, $-S(O)_p-(C_1-C_4$ alkyl), 5-tetrazolyl optionally substituted with a $C_1-C_4$ alkyl group or $-(CH_2)_q-$COOR', or phenyl optionally substituted with one or two groups selected from halo, cyano, $C_1-C_3$ alkyl, trifluoromethyl, $-CH_2CN$, $-CH_2Br$, $C_1-C_4$ alkoxy, $-S(O)_p-(C_1-C_4$ alkyl), acetyl, –COOR', 5-tetrazolyl, or 5-tetrazolyl substituted with $C_1-C_4$ alkyl or $-(CH_2)_q-$COOR';

where each R' is independently hydrogen or $C_1-C_4$ alkyl;

m is 1–4;

q is 1–4;

Y is hydrogen or –CN;

D is halo, $C_1-C_4$ alkoxy, or $-S-(C_1-C_4$ alkyl);

$R_5$ and $R_6$ are independently hydrogen, $C_1-C_3$ alkyl, or $C_2-C_4$ alkanoyl, or when taken together with the

nitrogen atom to which they are attached form a morpholino ring;
$R_7$ is hydroxy, $C_1$–$C_4$ alkoxy, halo, $-NR_5R_6$, –NHOH,

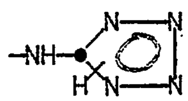
, or $C_1$-$C_3$ alkyl; and

each p is 0, 1, or 2, provided that when A is a bond, $R_4$ must be $C_1$–$C_6$ alkyl or an optionally substituted phenyl group, and further provided that when one of $R_5$ and $R_6$ is $C_2$–$C_4$ alkanoyl, the other of $R_5$ and $R_6$ is hydrogen.

A second aspect of this invention is a pharmaceutical formulation which comprises as an active ingredient a compound of Formula I as defined above, or a pharmaceutically acceptable salt thereof, associated with one or more pharmaceutically acceptable carrier or excipients therefor.

A preferred group of compounds are the compounds of Formula Ia:

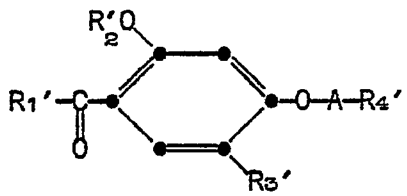
Ia

and pharmaceutically acceptable salts thereof, wherein
$R_1'$ is methyl or ethyl;
$R_2'$ is hydrogen or methyl;
$R_3'$ is ethyl, propyl, $-CH_2$-S-$CH_3$, or allyl; and
$R_4'$ is hydroxy, cyano, -COOR', $-CONR_5R_6$, -CO($C_1$-$C_3$ alkyl), $-S(O)_p$-($C_1$-$C_4$ alkyl), 5-tetrazolyl optionally substituted with a $C_1$-$C_4$ alkyl group, or phenyl optionally substituted in the meta position with one of the groups listed above, particularly cyano.

The following definitions refer to the various terms used throughout this disclosure.

The term "$C_1$-$C_6$ alkyl" refers to the straight and branched aliphatic radicals of 1 to 6 carbon atoms, such as methyl, ethyl, propyl, isopropyl, butyl, tert-butyl, sec-butyl, isobutyl, pentyl, hexyl, and the like. Within this term are included the terms "$C_1$-$C_3$ alkyl" and "$C_1$-$C_4$ alkyl". The term "$C_1$-$C_4$ alkoxy" refers to methoxy, ethoxy, propoxy, isopropoxy, butoxy, sec-butoxy, isobutoxy, and tert-butoxy. The term "$C_1$-$C_4$ alkoxy" includes within its definition the term "$C_1$-$C_3$ alkoxy". The term "halo" refers to fluoro, chloro, bromo, and iodo.

The term "$C_2$-$C_6$ alkenyl" refers to groups such as vinyl, allyl, butenyl, hexenyl, and the like. The term "$C_2$-$C_6$ alkynyl" refers to groups such as acetenyl, propargyl, butynyl, hexynyl, and the like. The term "$C_1$-$C_6$ alkanoyl" refers to the straight and branched aliphatic acyl radicals of 1 to 6 carbon atoms such as formyl, acetyl, propionyl, butyryl, 2-methylpropionyl, pentanoyl, hexanoyl, and the like.

The terms "$C_1$-$C_{10}$ alkylidene" and "phenylene" are divalent radicals derived from a $C_1$-$C_{10}$ alkane or benzene, respectively.

When any of the substituents in Formula I are carboxylic acid or 5-tetrazolyl moieties, the compounds of this invention include the pharmaceutically acceptable base addition salts thereof. Such salts include those derived from inorganic bases, such as ammonium and alkali and alkaline earth metal hydroxides, carbonates, bicarbonates, and the like, as well as salts derived from basic organic amines, such as aliphatic and aromatic amines, aliphatic diamines, hydroxy alkylamines, and the like. Such bases useful in preparing the salts of this invention thus include ammonium hydroxide, potassium carbonate, sodium bicarbonate, calcium hydroxide, methyl amine, diethyl amine, ethylene diamine, cyclohexylamine, ethanolamine, and the like. The potassium and sodium salt forms are particularly preferred.

It is recognized that when any of the alkyl groups are branched, various stereoisomeric products may exist. This invention is not limited to any particular stereoisomer but includes all possible individual isomers and mixtures thereof.

The compounds of this invention may be prepared according to standard methods known in the art. Thus, a further aspect of the instant invention consists of the following processes for preparing the compounds of Formula I. For example, final products and intermediates thereto can be prepared by acylating a benzene derivative as summarized in Scheme I:

Scheme I

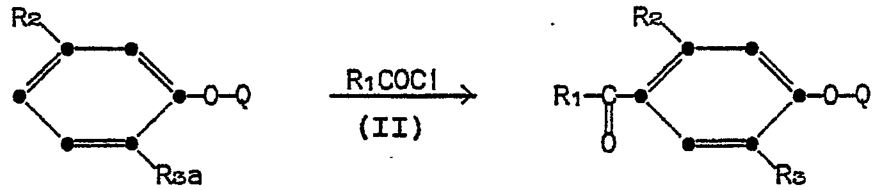


III IV

wherein:

$R_{3a}$ is $R_3$ or hydrogen, and Q is -A-$R_4$ or hydrogen. According to this scheme, an acid chloride of formula II is reacted with benzene derivative III under Friedel-Crafts acylation conditions to provide the acyl derivative IV. Any of a number of conditions to effect this transformation are known in the art and are operable. A preferred set of conditions comprises the reaction of II and III with a Lewis acid such as aluminum chloride in the presence of a non-reactive solvent, preferably dichloromethane. The reaction is best carried out at temperatures from about 0 to about 25°C and is generally complete within 2–4 hours. Alternatively, an acid anhydride or other acid halide may be employed in place of acid chloride II. When $R_{3a}$ is hydrogen, diacylated compounds can be obtained. If two different acyl moieties are desired, such acylations can be performed sequentially.

Typically, the acylation is effected using an acylating agent of formula

$R_1$–Z–CO–LG

where LG is a leaving group such as chloro, bromo or iodo or a group forming an acid anhydride.

When Q is hydrogen, the phenol, either before or after acylation, can be alkylated by standard methods.

The phenol is alkylated under typical bimolecular nucleophilic substitution ("$S_N2$") conditions – polar, aprotic solvents, (such as ketones (acetone, methylethyl ketone); amides (dimethylformamide), hexamethylphosphoramide, acetonitrile, and dimethylsulfoxide); preferably with an alkylating agent of the formula

X–A–$R_4$

wherein X is a good leaving group-such as a halo atom (chloro, bromo, iodo) (i.e., the Williamson synthesis); a sulfonate ester group (p-toluene-sulfonate, methylsulfonate, trifluoromethanesulfonate); a sulfonium group (dimethylsulfonium) and the like. It is preferred that the alkylation be carried out in the presence of non-nucleophilic bases with a pKa sufficient to deprotonate the hydroxy group of the phenol, same as sodium hydride. It is further preferred that any other reactive functionality on the alkylating reagent, such as a carboxylic acid or a tetrazole group, be first protected - such as by converting the carboxy group to a corresponding ester group. Also, newer alkylating reactions, such as using the methyl, ethyl or di-isopropyl azodicarboxylates and an alkyl- or arylphosphine to utilize the coupling of the phenol with an alkanol of the formula

HO-A-$R_4$

in an aromatic hydrocarbon or ethereal solvent. A similar process is described in U.S. Patent No. 4,604,386. A similar process is used to introduce the -$(CH_2)_m$-Y functionality at the $R_2$ position when $R_2$ is hydroxy.

Derivatives of Formula IV (or I) which contain an ester or nitrile functionality can be transformed to the corresponding acid and/or tetrazole compounds of the invention according to standard methods. For example, hydrolysis of esters of may be accomplished by any of a variety of acidic or basic conditions, preferably under aqueous conditions. Two preferred methods involve the use of lithium hydroxide in a solvent mixture of acetone/water or potassium hydroxide in a mixture of methanol/water. Under the former conditions, hydrolysis is generally complete in about 12-18 hours at temperatures from about 20-30°C whereas the latter reaction is usually complete in one hour at 20-30°C.

Similarly, transformation of the nitriles of this invention to the corresponding tetrazoles can be accomplished by any of a variety of standard methods. Generally, the nitrile is reacted with an azide reagent in a non-reactive solvent. Preferred conditions include the use of ammonium azide in dimethylformamide or tri(n-butyl)stannylazide in a non-reactive solvent such as dimethoxyethane or tetrahydrofuran. Under the latter conditions, the reaction is generally heated at or near the reflux temperature of the reaction mixture. The transformation is generally complete under these conditions in 2-3 days.

It is generally preferred, in compounds containing both a nitrile and an ester functionality, that the nitrile group be transformed into a tetrazole before hydrolysis of the ester.

When A-$R_4$ or $-(CH_2)_m$-Y are methyl, the corresponding phenols may be obtained via standard demethylation procedures. Standard methods for this transformation include the use of hydrobromic acid in acetic acid or treatment with molten pyridine hydrochloride. These methods usually also transform a carboxylic acid ester to the corresponding free carboxylic acid.

Other intraconversions of compounds are readily apparent to skilled artisans. For example, when $R_4$ is halo, compounds treated with cyanide, such as potassium or sodium cyanide, in a non-reactive solvent such as dimethylformamide, are transformed into cognates wherein $R_4$ is -CN. The use of a catalytic amount of iodide is employed to speed the reaction. Such nitriles can be converted into tetrazoles as described above, or hydrolyzed in the presence of a base, such as sodium or potassium hydroxides, in alcoholic water to provide the corresponding carboxylic acids. An alternate process for converting halides into nitriles involves the displacement by carbon anions in sodium amide and liquid ammonia as described in Example 68 which follows.

In other intraconversions, the halide derivative of Formula I ($R_4$ is halo) is allowed to react with an azide (e.q., sodium azide) amine (e.q., $R_5R_6NH$), or thiol (e.g., ($C_1$-$C_4$ alkyl)-SH), to provide the analogous compounds wherein $R_4$ is $-N_3$, $-NR_5R_6$, and -S-($C_1$-$C_4$ alkyl), respectively. The appropriate reagent is usually employed in the presence of a non-reactive solvent, such as dimethylformamide, and the transformation is generally complete within about 12-18 hours when kept at approximately 25°C. When a thiol reagent is used, a strong base, such as sodium hydride, is preferably also added. The primary amine compounds (e.g., $R_4$ is $-NH_2$) can also be obtained by reducing the corresponding azide, e.g., through catalytic hydro genation. The primary amine produced by any of these methods may be acylated by standard means, such as upon treatment with an alkanoyl halide or anhydride in the presence of a non-reactive acid scavenger.

Other transformations are also well known. Carboxylic acids can be esterified by standard means, or converted to acid halides which are then reacted with amines of the formula $R_5R_6NH$ to provide the corresponding amides. Similarly, esters, amides, and nitriles may be hydrolyzed to the carboxylic acid by means as described previously. Nitriles can also be hydrolyzed to the primary amide by treatment with aqueous base.

Alkylation of tetrazoles, such as with a $C_1$-$C_4$ alkyl halide $X-(CH_2)_q-COOR'$, in the presence of an acid scavenger, such as potassium carbonate, and an inert solvent such as dimethylformamide, provides both 1- and 2-substituted tetrazol-5-yl derivatives which may be separated by standard methods, for example, by employing high pressure liquid chromatography.

Although many of the compounds wherein $R_4$ is hydroxy can be made directly via alkylation of the desired phenol with a halo alkanol, some carbinols may also be obtained by reducing the corresponding ester or ketone, or by alkylating an acid halide derivative as found in Examples 102 and 103 which follow.

Similar transformations may be made with substituents on the optionally substituted phenyl ring of the $R_4$ moiety, and also various substituents accorded to $R_3$. For example, when $R_3$ is alkenyl, the double bond can be oxidized with a peracid to the corresponding epoxide intermediate which, upon catalytic hydrogenation, can be transformed into a hydroxyalkyl derivative. Reduction of an alkanoyl derivative also provides a carbinol analog. Hydrogenation of an alkene derivative or further reduction of the carbinol provides the alkyl substituted compound. In the special case of a benzaldehyde being transformed into a methanol, treatment of the methanol with lithium chloride and collidine in dimethylformamide provides the versatile chloromethyl derivative which, upon treatment with an alkanethiol or methanol/silver perchlorate, serves as an intermediate to the alkylthiomethyl and methoxymethyl compounds of this invention, respectively.

The thio derivatives and intermediates of this invention (p is 0) may be transformed into the corresponding sulfoxide (p is 1) compounds upon treatment with a mild oxidizing agent, such as hydrogen peroxide in methanol, meta-chloroperbenzoic acid (MCPBA) in methylene chloride at 0°C., or an alkali metal periodate in aqueous alcohol. The corresponding sulfones (p is 2) are prepared from the thio or sulfoxide compounds on treatment with a strong oxidizing agent such as hydrogen peroxide in acetic acid or m-chloroperbenzoic acid in methylene chloride at 20-30°C. In addition, various compounds of Formula I can be prepared from other compounds, precursors, or intermediates of Formula I by standard methods such as hydrolysis, esterification, alkylation, oxidation, reduction, and the like, as are well known to those skilled in the art.

A preferred process of the present invention is set forth in Scheme II:

## Scheme II

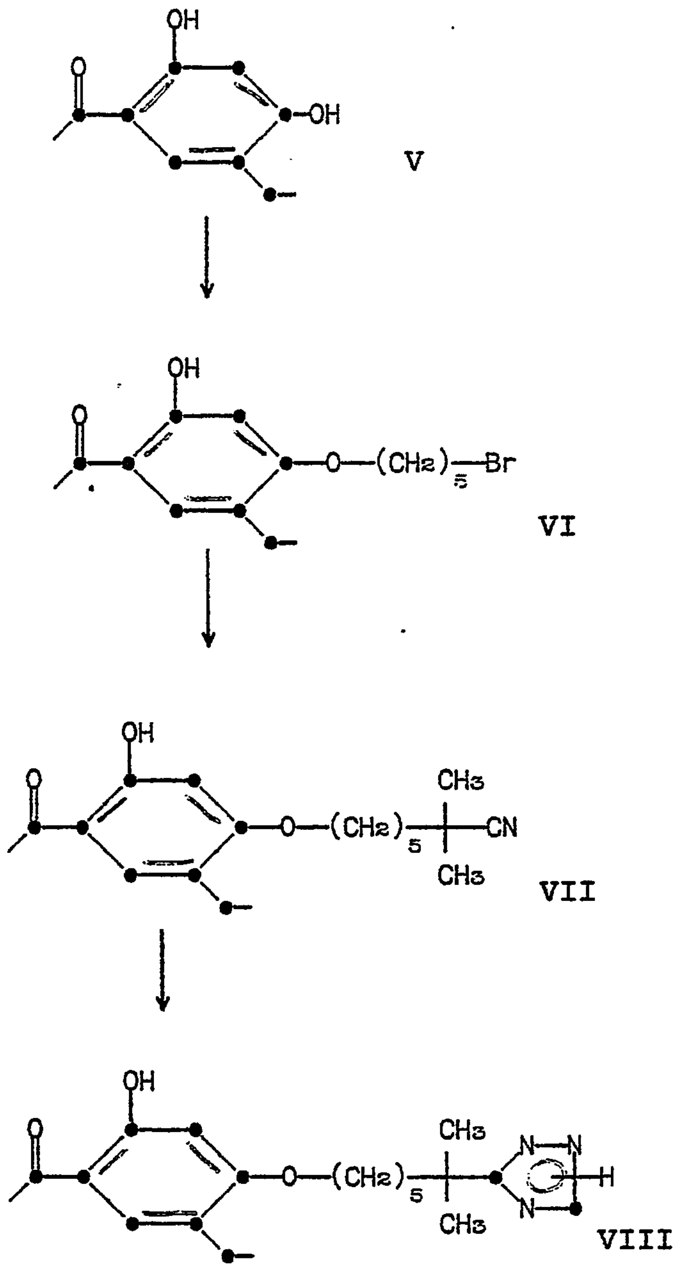

V

VI

VII

VIII

In the above Scheme II, diphenol V is alkylated employing an approximately 1:1 stoichiometry with 1,5-dibromopentane to the hydroxyacetophenone VI. The hydroxyacetophenone is in turn reacted with sodium 2-cyanopropan-2-ate (formed in situ in a sodium in ammonia mixture) to give the cyano compound VII. The cyano compound is reacted with an azide reagent (such as tri(n-butyl)tin azide) to yield the tetrazole compound VIII.

Intermediate compounds II and III and any other necessary reagents are either commercially available, known in the literature, or can be prepared according to methods known in the art as exemplified by the examples which follow. The following examples further illustrate the preparation of the intermediates and compounds of this invention. The examples are illustrative only and are not intended to limit the scope of the invention in any way. Where structures were confirmed by infra-red, proton nuclear magnetic resonance, or mass spectral analysis, the compound is so designated by "IR", "NMR", or "MS", respectively.

Preparation 1

2-allyl-4-acetyl-5-methoxyphenol

A. Preparation of 4-allyloxy-2-hydroxyacetophenone.

A mixture of 60.8 g of 2,4-dihydroxyacetophenone, 38.0 ml of allyl bromide, 60.7 g of potassium carbonate, 5 g of potassium iodide, and 500 ml of methyl ethyl ketone was heated at reflux for 24 hours. The mixture was filtered and the filtrate concentrated invacuo. The residue was dissolved in ethyl acetate/Skelly B, washed successively with potassium carbonate and sodium chloride solutions, dried over sodium sulfate, filtered; and concentrated in vacuo to provide 69.4 g of the subtitle intermediate as an oil which was used without further purification.

B. Preparation of 4-allyloxy-2-methoxyacetophenone.

To a mixture of 10.6 g of a 50% sodium hydride dispension in oil and 18.7 ml of methyl iodide in 150 ml of dimethylformamide cooled to 0°C. by means of an external ice bath was added a solution of 38.4 g of 4-allyloxy-2-hydroxyacetophenone in 100 ml of dimethylformamide over a 30 minute interval. The mixture was allowed to warm to room temperature and then heated for 5 hours at 50-60°C. After cooling, the reaction mixture was added to ethyl acetate and dilute hydrochloric acid. The layers were separated, and the organic layer was washed with a saturated sodium chloride solution, dried over sodium sulfate, filtered, and concentrated invacuo. The residue was dissolved in a minimum of hot heptane, and on subsequent cooling to room temperature, 13.63 g of the desired subtitle intermediate were recovered, m.p. <25°C.
Analysis for $C_{12}H_{14}O_3$:
Calculated: C, 70.23; H, 6.38;
Found: C, 70.02; H, 6.56.

C. Preparation of 2-allyl-4-acetyl-5-methoxyphenol.

Under a nitrogen atmosphere, 7.4 g of 4-allyloxy-2-methoxyacetophenone were heated with stirring at 210°C. After cooling, the residue was dissolved in methylene chloride/heptane and cooled overnight in a freezer (ca -20°C). The crystals which formed were recovered by filtration affording 1.78 g of the title intermediate. NMR.
Analysis for $C_{12}H_{14}O_3$:
Calculated: C, 69.89; H, 6.84;
Found: C, 68.81; H, 6.42.

Preparation 2

5-allyl-2,4-dihydroxyacetophenone

To 106 mg of 2-allyl-4-acetyl-5-methoxyphenol in 15 ml of methylene chloride at -78°C under a nitrogen atmosphere were added 1.5 ml of a 1.0 M solution of boron tribromide in methylene chloride. The mixture was allowed to warm to -45°C over the next 30 minutes and then added to ethyl acetate and a saturated sodium chloride solution. The layers were separated and the organic layer dried over sodium sulfate, filtered, and concentrated in vacuo. The residue was purified by preparative thin layer chromatography (silica gel) eluting with 40% ethyl acetate in hexane providing 51 mg of the desired title intermediate. Crystallization from diethyl ether/hexane provided material with a melting point of 74-76°C. NMR.

Preparation 3

6-(5-Ethyl-2,4-dihydroxyphenyl)-6-oxohexanoic acid, methyl ester

To a mixture of 50 ml of a 1M solution of boron tribromide in methylene chloride cooled to 0°C were added 3.08 g of 6-(5-ethyl-2,4-dimethoxyphenyl)-6-oxohexanoic acid, methyl ester (See example 6). While allowing the temperature to warm to room temperature, the mixture was stirred for 24 hours. An additional 25 ml of boron tribromide were added and the mixture was again stirred overnight. The reaction mixture was partitioned between a cold ammonium chloride solution and ethyl acetate. The layers were separated and the organic layer was again washed with cold ammonium chloride solution. The organic layer was dried over sodium sulfate, filtered, and concentrated in vacuo. The residue was dissolved in 200 ml of methanol. Under a nitrogen atmosphere, two milliliters of sulfuric acid were added, and the mixture heated at reflux for three hours. After removing the methanol in vacuo, the residue was dissolved in ethyl acetate, washed twice with a saturated sodium chloride solution, dried over sodium sulfate, filtered, and concentrated in vacuo. The resulting residue was purified by high pressure liquid chromatography over silica gel eluting with a 0-40% ethyl acetate in hexane gradient. The appropriate fractions

were combined and concentrated in vacuo to provide 0.36 g of the desired title intermediate, m.p. = 62-64°C.

According to the same procedure (in some cases less the step reforming the ester), the following intermediates were prepared from the corresponding dimethoxy compounds of Examples 1-5, and 7-9.

3-(5-Ethyl-2,4-dihydroxybenzoyl)benzoic acid, methyl ester, 43% yield, m.p. = 146-147°C.

Analysis for $C_{17}H_{16}O_5$:
Calculated: C, 67.99; H, 5.37;
Found: C, 66.77; H, 5.56.

4-(5-Ethyl-2,4-dihydroxybenzoyl)benzoic acid, methyl ester, 21% yield, m.p. = 150-153°C.

2-(5-Ethyl-2,4-dihydroxybenzoyl)benzoic acid, methyl ester, 39% yield.
5-Butyl-2,4-dihydroxyacetophenone, 64% yield
5-Pentyl-2,4-dihydroxyacetophenone, 53% yield
5-Methyl-2,4-dihydroxyacetophenone, 48% yield
5-(5-Ethyl-2,4-dihydroxyphenyl)-5-oxopentanoic acid, methyl ester, 49% yield
5-Ethyl-2,4-dihydroxyacetophenone, 36% yield
5-Hexyl-2,4-dihydroacetophenone, 70% yield

Preparation 4

4,6-Diacetoresorcinol

A mixture of 3 g of 4,6-diacetoresorcinol, dimethyl ether (see Example 10), 50 ml of acetic acid, and 50 ml of 48% hydrobromic acid was refluxed for 2 hours. After concentration in vacuo, the residue was partitioned between ethyl acetate and a saturated sodium chloride solution. The layers were separated and the organic solution was washed with a saturated sodium bicarbonate solution. Evaporation of the solvent provided crude product which was purified by high pressure liquid chromatography over silica gel eluting with a 0-50% ethyl acetate in hexane gradient. The appropriate fractions were combined and concentrated in vacuo to provide 0.9 g of the desired title intermediate, m.p. = 182-184°C.

Analysis for $C_{10}H_{10}O_4$:
Calculated: C, 61.85; H, 5.19;
Found: C, 62.74; H, 5.45.

Preparation 5

5-Acetyl-2,4-dihydroxybenzaldehyde

To a mixture of 49.8 g of 2,4-dimethoxybenzaldehyde, 23.6 ml of acetyl chloride, and 1000 ml of methylene chloride cooled to approximately 0°C were added 119.7 g of aluminum chloride with stirring under a nitrogen atmosphere. The reaction was allowed to warm to room temperature while stirring overnight. The mixture was added to a slurry of ice and concentrated hydrochloric acid. The organic layer was separated and washed with a saturated sodium chloride solution, dried over sodium sulfate, and concentrated in vacuo. The residue was purified by high pressure liquid chroma tography over silica gel eluting with a 0-40% ethyl acetate in hexane gradient. The appropriate fractions were pooled and concentrated to provide 5.85 g of the desired title intermediate, m.p. = 142-143°C.

Preparation 6

5-Acetyl-2,4-dihydroxybenzyl alcohol

One gram of 5-acetyl-2,4-dihydroxybenzaldehyde was hydrogenated in the presence of 1 g of 10% palladium on carbon in 150 ml of ethanol. After about 1 hour, hydrogen uptake ceased and hydrogenation was terminated. The reaction mixture was filtered and concentrated invacuo. The residue was dissolved in approximately 30 ml of ethyl acetate and cooled in the refrigerator. The resulting solid was recovered by filtration affording 0.66 g of the desired title intermediate, m.p. = 151-152°C. The same reaction when run on a 5-fold scale provide 2.76 g of material.

Analysis for $C_9H_{10}O_4$:
Calculated: C, 59.34; H, 5.53;
Found: C, 59.29; H, 5.74.

Example 1

5-Butyl-2,4-dimethoxyacetophenone

A. Preparation of 2,4-dimethoxybutyrophenone.

One hundred grams of meta-dimethoxybenzene and 84.86 g of butyryl chloride were added to 1000 ml of methylene chloride. The solution was cooled to 0°C by means of an external acetone/ice bath. With stirring, 144.76 g of aluminum chloride were added in portions over 1 hour. After all of the aluminum chloride was added, the mixture was stirred at 0°C for 2.5 hours. The mixture was poured into a mixture of ice and concentrated hydrochloric acid. The layers were separated and the organic layer was washed sequentially with a saturated potassium carbonate solution and a saturated sodium chloride solution, dried over magnesium sulfate, and concentrated in vacuo providing 142.3 g of a crude oil which was used in the subsequent reaction without further purification.

B. Preparation of 1-(2,4-dimethoxyphenyl)-1-butanol.

The 142.3 g of crude product from Example 1A were dissolved in 800 ml of methanol. After cooling to approximately 0°C, 31.02 g of sodium borohydride were added in portions. The reaction mixture was allowed to warm to room temperature and stirred overnight. An additional 6.46 g of sodium borohydride were added and the reaction was stirred for 2 days at room temperature. The reaction mixture was heated at reflux for three hours and then concentrated invacuo. The residue was triturated with cold 1N hydrochloric acid and extracted into ethyl acetate. The organic layer was washed with water, dried over magnesium sulfate, and concentrated in vacuo to provide 137.5 g of the subtitle intermediate which was used without further purification.

C. Preparation of 1-butyl-2,4-dimethoxybenzene.

In five portions, 137.5 g of the intermediate of Example 1B were catalytically hydrogenated in glacial acetic acid. Each portion employed about 5 g of 5% palladium on carbon as the catalyst. Hydrogenation of each portion was complete within about 30-40 minutes. The reactions were combined, filtered, and concentrated in vacuo. The residue was triturated with cold 1N hydrochloric acid and treated with ethyl acetate. The layers were separated and the organic layer was washed with a sodium bicarbonate solution followed by a sodium chloride solution. After drying over magnesium sulfate, the solution was concentrated in vacuo to provide 106.1 g of the desired subtitle intermediate which was used in the subsequent step without further purification.

D. Preparation of 5-butyl-2,4-dimethoxyacetophenone.

The 106.1 g of intermediate from Example 1C and 42.87 g of acetyl chloride were dissolved in 1000 ml of methylene chloride. The reaction mixture was cooled to -15°C by means of an external ethylene glycol/dry ice bath. In portions, 80.06 g of aluminum chloride were added over 90 minutes at a rate to maintain the temperature between -12° and -18°C. The reaction mixture was poured into a mixture of ice and concentrated hydrochloric acid. The layers were separated and the organic layer was washed with a potassium carbonate solution followed by a saturated sodium chloride solution. After drying over magnesium sulfate, the organic solution was concentrated in vacuo to provide 120.1 g of an oil which solidified upon standing. Twenty grams of this material was purified by high pressure liquid chromatography over silica gel eluting with a 0-100% ethyl acetate and hexane step gradient to provide 8.42 g of the purified title product.

Examples 2-9

Following the procedure of Example 1D, the following compounds were prepared from the appropriate benzene derivative and the corresponding acid chloride or acid anhydride.

2. 5-Hexyl-2,4-dimethoxyacetophenone, 39% yield.
3. 5-Pentyl-2,4-dimethoxyacetophenone, 19% yield.
4. 5-Methyl-2,4-dimethoxyacetophenone, 32% yield.
5. 3-(5-Ethyl-2,4-dimethoxybenzoyl)benzoic acid, methyl ester, 63% yield, m.p. = 116-118°C.

Analysis for $C_{19}H_{20}O_5$:
Calculated: C, 69.50; H, 6.14;
Found: C, 67.59; H, 5.43.

6. 6-(5-Ethyl-2,4-dimethoxyphenyl)-6-oxo-hexanoic acid, methyl ester, 56% yield, m.p. = 57-58°C.

Analysis for $C_{17}H_{24}O_5$:

Calculated: C, 66.21; H, 7.85;
Found: C, 63.09; H, 6.57.

7. 4-(5-Ethyl-2,4-dimethoxybenzoyl)benzoic acid, methyl ester, 53% yield, m.p. = 128-130°C.
Analysis for $C_{19}H_{20}O_5$:
Calculated: C, 69.50; H, 6.14;
Found: C, 67.62; H, 7.38.

8. 2-(5-Ethyl-2-hydroxy-4-methoxybenzoyl)-benzoic acid, 24% yield, m.p. = 194-197°C.
Analysis for $C_{17}H_{16}O_5$:
Calculated: C, 67.99; H, 5.37;
Found: C, 67.70; H, 5.65.

9. 5-(5-Ethyl-2-hydroxy-4-methoxyphenyl)-5-oxopentanoic acid, 29% yield, m.p. = 124-128°C.
Analysis for $C_{14}H_{18}O_5$:
Calculated: C, 63.14; H, 6.81;
Found: C, 64.48; H, 7.22.

## Example 10

4,6-Diacetoresorcinol, dimethyl ether

Following the general procedure of Example 1D, 100 g of meta-dimethoxybenzene, 62.5 g of acetyl chloride, and 106.16 g of aluminum chloride were reacted in 1000 ml of dichloromethane. After workup, the material was crystallized from ethyl acetate/hexane to provide 89.8 g of crude material. Forty-five grams of this material was purified by high pressure liquid chromatography over silica gel eluting with a 0-20% ethyl acetate in hexane gradient. The less polar material was recovered and identified as 2,4-dimethoxyacetophenone. The material remaining on the chromatography column was eluted with ethyl acetate and provided 10 g of the desired title product.

## Examples 11-15

The following compounds were prepared from the appropriate benzene derivative and corresponding acid chloride or acid anhydride according to the procedure of Example 1D.

11. 5-Acetyl-2,4-dihydroxybenzaldehyde, 12.8% yield, m.p. = 134-136°C.
Analysis for $C_9H_8O_4$:
Calculated: C, 60.00; H, 4.98; O, 35.52;
Found: C, 59.87; H, 4.55; O, 35.61.

12. 5-[4-(4-Cyanobutoxy)-5-allyl-2-methoxyphenyl]-5-oxopentanoic acid, 8.4% yield.

13. 5-(2-Allyl-4-propanoyl-5-methoxyphenoxy)pentanenitrile, 22.8% yield.
Analysis for $C_{18}H_{23}NO_3$:
Calculated: C, 71.73; H, 7.69; N, 4.65;
Found: C, 71.56; H, 7.59; N, 4.44.

14. 5-(2-Allyl-4-benzoyl-5-methoxyphenoxy)pentanenitrile, 11.2% yield.

15. 5-(4-Acetyl-5-chloro-2-propylphenoxy)pentanenitrile, 40.8% yield, oil.
Analysis for $C_{16}H_{20}ClNO_2$:
Calculated: C, 65.41; H, 6.86; N, 4.77; Cl, 12.07;
Found: C, 66.26; H, 7.06; N, 5.04; Cl, 11.84.

## Example 16

5-(2,4-Diacetyl-5-hydroxyphenoxy)pentanenitrile

A mixture of 582 mg of 4,6-diacetylresorcinol, 460 mg of potassium carbonate, 530 mg of 5-bromopentanenitrile and 0.2 g of potassium iodide was heated at reflux overnight. The mixture was concentrated in vacuo and partitioned between ethyl acetate and dilute hydrochloric acid. The organic layer was separated, dried over sodium sulfate, and concentrated in vacuo The residue was purified by preparative thin layer chromatography on silica gel eluting with 40% ethyl acetate in hexane to provide 200 mg of the desired title product, m.p. = 105-106°C.
Analysis for $C_{15}H_{17}NO_4$:
Calculated: C, 65.44; H, 6.22; N, 5.09;
Found: C, 65.61; H, 6.28; N, 5.33.

Examples 17-67

The following compounds were prepared according to the procedure of Example 16 employing the appropriate phenol and the corresponding halo alkane derivative.

17. 5-[4-Acetyl-5-hydroxy-2-(hydroxymethyl)phenoxy]pentanenitrile, 44% yield, m.p. = 72-74°C.
Analysis for $C_{14}H_{17}NO_4$:
Calculated: C, 63.87; H, 6.51; N, 5.32;
Found: C, 63.67; H, 6.34; N, 5.31.

18. 5-(4-Acetyl-5-hydroxy-2-formylphenoxy)pentanenitrile, 3% yield.
Analysis for $C_{14}H_{15}NO_4$:
Calculated: C, 64.36; H, 5.79;
Found: C, 63.04; H, 6.02.

19. 5-(4-Acetyl-2-ethyl-5-hydroxyphenoxy)pentanenitrile, 50% yield, m.p. = 83-84.5°C.
Analysis for $C_{15}H_{19}NO_3$:
Calculated: C, 68.94; H, 7.33; N, 5.36;
Found: C, 69.74; H, 6.94; N, 5.17.

20. 7-(4-Acetyl-2-ethyl-5-hydroxyphenoxy)heptanenitrile, 50% yield, m.p. = 56-57°C.
Analysis for $C_{17}H_{23}NO_3$:
Calculated: C, 70.56; H, 8.01; N, 4.84;
Found: C, 70.60; H, 8.31; N, 4.70.

21. 6-(4-Acetyl-5-hydroxy-2-allylphenoxy)hexanenitrile, 45.8% yield, m.p. = 70-71°C.
Analysis for $C_{17}H_{21}NO_3$:
Calculated: C, 71.06: H, 7.37; N, 4.07:
Found: C, 70.B7: H, 7.47: N, 4.70.

22. 5,5'-[(4-Acetyl-6-formyl-1,3-phenylene)bis(oxy)]bis[pentanenitrile], 75% yield, m.p. = 86-88°C.
Analysis for $C_{19}H_{22}N_2O_4$:
Calculated: C, 66.65; H, 6.48; N, 8.18;
Found: C, 66.98; H, 6.25; N, 7.96.

23. 9-(4-Acetyl-5-hydroxy-2-allylphenoxy)nonanenitrile, 6% yield, m.p. = 50-52°C.
Analysis for $C_{20}H_{27}NO_3$:
Calculated: C, 72.92; H, 8.26; N, 4.25;
Found: C, 72.65; H, 8.49; N, 4.00.

24. 7-(4-Acetyl-5-hydroxy-2-allylphenoxy)heptanenitrile, 39% yield, m.p. = 54-55°C.
Analysis for $C_{18}H_{23}NO_3$:
Calculated: C, 71.73; H, 7.69; N, 4.65;
Found: C, 71.85; H, 7.66; N, 4.51.

25. (4-Acetyl-5-hydroxy-2-allylphenoxy)acetonitrile, 45% yield, m.p. = 66-68°C.
Analysis for $C_{13}H_{13}NO_3$:
Calculated: C, 67.52; H, 5.67; N, 6.06;
Found: C, 67.31; H, 5.39; N, 5.92.

26. 4-(4-Acetyl-5-hydroxy-2-allylphenoxy)butanenitrile, 7% yield, m.p. = 92-94°C.
Analysis for $C_{15}H_{17}NO_3$:
Calculated: C, 69.48; H, 6.61; N, 5.40;
Found: C, 69.96; H, 7.74; N, 6.46.

27. 5-[4-(4-Cyanobutoxy)-5-ethyl-2-hydroxybenzene]-5-oxopentanoic acid, methyl ester, 79% yield, m.p. = 50-52°C.
Analysis for $C_{19}H_{25}NO_5$:
Calculated: C, 65.69: H, 7.25: N, 4.03;
Found: C, 65.82; H, 7.48; N, 4.26.

28. 5-(4-Acetyl-2-methyl-5-hydroxyphenoxy)pentanenitrile, 71% yield, m.p. = 86-87°C.
Analysis for $C_{14}H_{17}NO_3$:
Calculated: C, 68.00; H, 6.93; N, 5.66;
Found: C, 67.77; H, 6.84; N, 5.38.

29. 5-(4-Acetyl-2-hexyl-5-hydroxyphenoxy)pentanenitrile, 75% yield, m.p. = 45-49°C.
Analysis for $C_{19}H_{27}NO_3$:
Calculated: C, 71.89; H, 8.57; N, 4.41;
Found: C, 71.82; H, 8.74; N, 4.29.

30. 5-(4-Acetyl-2-butyl-5-hydroxyphenoxy)pentanenitrile, 73% yield, m.p. 57-60°C.
Analysis for $C_{17}H_{23}NO_3$:
Calculated: C, 70.56; H, 8.01; N, 4.84;
Found: C, 70.79; H, 7.79; N, 4.89.

31. 5-(4-Acetyl-5-hydroxy-2-pentylphenoxy)pentanenitrile, 58% yield, m.p. = 34-37°C.
Analysis for $C_{18}H_{25}NO_3$:
Calculated: C, 71.26; H, 8.31; N, 4.62;

Found: C, 71.49; H, 8.41; N, 4.70.

32. 6-[4-(4-Cyanobutoxy)-5-ethyl-2-hydroxybenzene]-6-oxohexanoic acid, methyl ester, 77.8% yield, m.p. = 90-94°C.

33. 2-[4-(4-Cyanobutoxy)-5-ethyl-2-hydroxybenzoyl]benzoic acid, methyl ester, 31.5% yield.

34. 3-[4-(4-Cyanobutoxy)-5-ethyl-2-hydroxybenzoyl]benzoic acid, methyl ester, 85.7% yield, m.p. = 88-90°C.

35. 4-[4-(4-Cyanobutoxy)-5-ethyl-2-hydroxybenzoyl]benzoic acid, methyl ester, 31.5% yield.

36. 7-[4-Acetyl-5-hydroxy-2-(hydroxymethyl)phenoxy]heptanenitrile, 69% yield, m.p. = 58-60°C.
Analysis for $C_{16}H_{21}NO_4$:
Calculated: C, 65.96; H, 7.27; N, 4.81;
Found: C, 65.96; H, 7.04; N, 4.70.

37. 4-(Heptyloxy)-2-hydroxy-5-allylacetophenone, 40% yield, oil.
Analysis for $C_{18}H_{26}O_3$:
Calculated: C, 74.45; H, 9.02;
Found: C, 74.31; H, 8.79.

38. 4-(Octyloxy)-5-allyl-2-hydroxyacetophenone, 21% yield, oil.
Analysis for $C_{19}H_{28}O_3$:
Calculated: C, 74.96; H, 9.27;
Found: C, 74.99; H, 9.00.

39. 4-(Hexyloxy)-2-hydroxy-5-allylacetophenone, 36% yield, m.p. = 42-44°C.
Analysis for $C_{17}H_{24}O_6$:
Calculated: C, 73.88; H, 8.75;
Found: C, 73.96; H, 8.93.

40. 4-Butoxy-2-hydroxy-5-allylacetophenone, 60% yield, oil.
Analysis for $C_{15}H_{20}O_3$:
Calculated: C, 72.55; H, 8.12:
Found: C, 72.60: H, 7.83.

41. 4-Pentyloxy-2-hydroxy-5-allylacetophenone, 44% yield, oil.
Analysis for $C_{16}H_{22}O_3$:
Calculated: C, 73.25; H, 8.45;
Found: C, 73.52; H, B.41.

42. 2-Hydroxy-4-methoxy-5-allylacetophenone, 41% yield.
Analysis for $C_{12}H_{14}O_6$:
Calculated: C, 69.89; H, 6.84;
Found: C, 69.87; H, 6.72.

43. 2,4-Dimethoxy-5-allylacetophenone, 2% yield.
Analysis for $C_{13}H_{16}O_3$:
Calculated: C, 70.89; H, 7.32;
Found: C, 70.14; H, 7.00.

44. 4-(4-Hydroxybutoxy)-5-allyl-2-hydroxyacetophenone, 5% yield, m.p. = 117-119°C.
Analysis for $C_{15}H_{20}O_4$:
Calculated: C, 68.16; H, 7.63;
Found: C, 68.38; H, 7.85.

45. 4-(3-Butenyloxy)-2-hydroxy-5-allylacetophenone, 26% yield, m.p. = <25°C.
Analysis for $C_{15}H_{18}O_3$:
Calculated: C, 73.15; H, 7.37:
Found: C, 72.88; H, 7.45.

46. 4-(5-Hexenyloxy)-2-hydroxy-5-allylacetophenone, 30% yield, m.p. = <25°C.
Analysis for $C_{17}H_{22}O_3$:
Calculated: C, 74.42; H, 8.08;
Found: C, 74.16; H, 8.13.

47. 2-Hydroxy-4-benzyloxy-5-allylacetophenone, 60% yield, m.p. = 86°C.
Analysis for $C_{18}H_{18}O_3$:
Calculated: C, 76.57; H, 6.43;
Found: C, 76.47; H, 6.19.

48. 4-[(4-Acetyl-5-hydroxy-2-allylphenoxy)methyl]benzonitrile, 63% yield, m.p. = 158.5-159°C.
Analysis for $C_{19}H_{17}NO_3$:
Calculated: C, 74.25; H, 5.58; N, 4.56;
Found: C, 74.48; H, 5.80; N, 4.67.

49. 2-[(4-Acetyl-5-hydroxy-2-allylphenoxy)methyl]benzonitrile, 71% yield, m.p. = 159°C.
Analysis for $C_{19}H_{17}NO_3$:
Calculated: C, 74.25; H, 5.58; N, 4.56;
Found: C, 74.37; H, 5.86; N, 4.39.

50. 3-[(4-Acetyl-5-hydroxy-2-allylphenoxy)methyl]benzonitrile, 51% yield, m.p. = 131.5°C.
Analysis for $C_{19}H_{17}NO_3$:

Calculated: C, 74.25; H, 5.58; N, 4.56;
Found: C, 74.39; H, 5.55; N, 4.28.

51. 2-Hydroxy-4-(3-methylbenzyloxy)-5-allylacetophenone, 50% yield, m.p. = 87-88°C.
Analysis for $C_{19}H_{20}O_3$:
Calculated: C, 77.00; H, 6.80;
Found: C, 77.03; H, 7.05.

52. 4-[4-(Bromomethyl)benzyloxy]-2-hydroxy-5-allylacetophenone, 18% yield, oil.
Analysis for $C_{19}H_{19}BrO_3$:
Calculated: C, 60.81; H, 5.10; Br, 21.29;
Found: C, 60.74; H, 5.11; Br, 21.57.

53. 2-Hydroxy-4-(3-phenylpropoxy)-5-propylacetophenone, 26% yield, m.p. = 60°C.
Analysis for $C_{20}H_{24}O_3$:
Calculated: C, 76.89; H, 7.74;
Found: C, 76.68; H, 7.69.

54. 5-Ethyl-4-(3-fluorobenzyloxy)-2-hydroxyacetophenone, 18% yield, m.p. = 104-105°C.
Analysis for $C_{17}H_{17}FO_3$:
Calculated: C, 70.82; H, 5.94;
Found: C, 70.54; H, 6.10.

55. 3-[(4-Acetyl-2-ethyl-5-hydroxyphenoxy)methyl]benzonitrile, 80% yield, m.p. = 155-155.5°C.
Analysis for $C_{18}H_{17}NO_3$:
Calculated: C, 73.20; H, 5.80; N, 4.74;
Found: C, 72.92; H, 5.98; N, 4.68.

56. 4-(3-Chlorobenzyloxy)-5-ethyl-2-hydroxyacetophenone, 47% yield.
Analysis for $C_{17}H_{17}ClO_3$:
Calculated: C, 67.00; H, 5.62;
Found: C, 67.13; H, 5.33.

57. 4-(5-Hexynyloxy)-2-hydroxy-5-allylacetophenone, 20% yield, m.p. = <25°C. NMR.

58. 5-Ethyl-2-hydroxy-4-(3-trifluoromethylbenzyloxy)acetophenone, 11% yield.
Analysis for $C_{18}H_{17}F_3O_3$:
Calculated: C, 63.90: H, 5.06:
Found: C, 62.9; H, 5.53.

59. 4-(3-Methylmercaptobenzyloxy)-5-ethyl-2-hydroxyacetophenone, 18.5% yield, m.p. = 89°C. NMR.

60. 3-[(4-Acetyl-5-hydroxy-2-allylphenoxy)methyl]benzeneacetonitrile. NMR.

61. 4-(2-Bromoethoxy)-5-ethyl-2-hydroxyacetophenone, 42.5% yield, m.p. = 58-59°C. NMR.

62. 4-(3-Bromopropoxy)-5-ethyl-2-hydroxyacetophenone, 80.3% yield, m.p. = 126-127°C. NMR.

63. 4-(5-Bromopentoxy)-5-ethyl-2-hydroxyacetophenone, 59.7% yield, m.p. = 60-62°C. NMR.

64. 4-(10-Bromodecyloxy)-5-allyl-2-hydroxyacetophenone, 18% yield, m.p. = <25°C. NMR.
Analysis for $C_{21}H_{31}BrO_3$:
Calculated: C, 61.31; H, 7.60; Br, 19.42;
Found: C, 58.76; H, 6.59; Br, 21.02.

65. 4-(7-Bromoheptyloxy)-5-allyl-2-hydroxyacetophenone, 70% yield, oil.
Analysis for $C_{18}H_{25}BrO_3$:
Calculated: C, 58.54; H, 6.82; Br, 21.64;
Found: C, 56.92; H, 6.33; Br, 22.32.

66. 4-(6-Bromohexyloxy)-5-allyl-2-hydroxyacetophenone, 42% yield.
Analysis for $C_{17}H_{23}BrO_3$:
Calculated: C, 57.47; H, 6.53; Br, 22.49;
Found: C, 57.37; H, 6.63; Br, 22.60.

67. 4-(4-Bromobutoxy)-5-allyl-2-hydroxyacetophenone, 56% yield, m.p. = <25°C.
Analysis for $C_{15}H_{19}BrO_3$:
Calculated: C, 55.03; H, 5.85; Br, 24.42;
Found: C, 54.43; H, 5.87; Br, 24.93.

Example 68

4-(6-Cyano-6-methßylheptyloxy)-5-ethyl-2-hydroxyacetophenone

Approximately 300 ml of ammonia were condensed in a 1 liter flask fitted with a mechanical stirrer and reflux condenser. A pinch of ferric chloride was added to the solution with stirring followed by the piecewise addition of 0.69 g of sodium metal. A solution of 1.36 ml of isobutyronitrile in 30 ml of diethyl ether was added dropwise followed by a slurry of 4.935 g of 4-(5-bromopentoxy)-5-ethyl-2-hydroxyacetophenone in diethyl ether. The reaction was stirred overnight during which time the ammonia evaporated leaving an oil. The oil was partitioned between ethyl acetate and dilute cold hydrochloric acid. The organic layer was separated, dried over sodium sulfate, filtered, and concentrated invacuo. The residue was purified by high pressure liquid chromatography over silica gel eluting with a 0-25% ethyl acetate in hexane gradient providing 2.91 g of the desired title product, m.p. = 168-169°C.
Analysis for $C_{19}H_{27}NO_3$:
Calculated: C, 71.89; H, 8.57; N, 4.41;
Found: C, 71.58; H, 8.70; N, 4.08.

Example 69

8-(4-Acetyl-5-hydroxy-2-allylphenoxy)octanenitrile

A mixture of 1.845 g of 4-(7-bromoheptyloxy-5-allyl-2-hydroxyacetophenone, 0.651 g of potassium cyanide, 0.2 g of potassium iodide, and 25 ml of dimethylformamide was heated at 70°C for approximately 6 days. The mixture was then partitioned between ethyl acetate and cold dilute hydrochloric acid. The layers were separated and the organic layer was dried over sodium sulfate, filtered, and concentrated in vacuo. The residue was purified by preparative thin layer chromatography on silica gel. Purification of the resulting material by crystallization from diethyl ether/hexane provided 190 mg of the desired title product, m.p. = 46-48°C.
Analysis for $C_{19}H_{25}NO_3$:
Calculated: C, 72.35; H, 7.99; N, 4.44;
Found: C, 72.29; H, 7.72; N, 4.64.

Examples 70 and 71

The following compounds are prepared according to the procedure of Example 69 from the appropriate bromide derivative and potassium or sodium cyanide.

70. 11-(4-Acetyl-5-hydroxy-2-allylphenoxy)undecanenitrile, 3.9% yield.
Analysis for $C_{22}H_{31}NO_3$:
Calculated: C, 73.92; H, 8.74; N, 3.92;
Found: C, 73.83; H, 8.53; N, 3.93.

71. 4-[(4-Acetyl-5-hydroxy-2-allylphenoxy)methyl]benzeneacetonitrile, 20% yield, m.p. = 142-144°C. NMR.

Example 72

4-(3-Azidopropoxy)-5-ethyl-2-hydroxyacetophenone

A mixture of 2.107 g of 4-(3-bromopropoxy)-5-ethyl-2-hydroxyacetophenone and 0.5 g of sodium azide in 30 ml of dimethylformamide was stirred together overnight at room temperature. The mixture was partitioned between ethyl acetate and dilute hydrochloric acid. The layers were separated and the organic layer was dried over sodium sulfate, filtered, and concentrated in vacuo providing the desired title product. The proton NMR and mass spectral analyses were consistent with the structure of the desired product.

Example 73

4-(2-Azidoethoxy)-5-ethyl-2-hydroxyacetophenone

The title product was prepared from 2 g of 4-(2-bromoethoxy)-5-ethyl-2-hydroxyacetophenone according to the procedure of Example 72. The proton nmr and mass spectral analyses were consistent with the structure of the desired product.

Example 74

4-(4-Aminobutoxy)-2-hydroxy-5-propylacetophenone hydrochloride

A mixture of 1.64 g of 4-(4-bromobutoxy)-2-hydroxy-5-allylacetophenone and 0.325 g of sodium azide and 25 ml of dimethylformamide was reacted according to the procedure of Example 72. After workup, the resulting product was hydrogenated over 1 g of 5% palladium on carbon in 100 ml of 2B ethanol for 4 hours. The reaction mixture was filtered and concentrated invacuo. The residue was dissolved in diethyl ether and extracted into acid. The acidic portion was made basic and extracted into diethyl ether. The organic solution was dried over sodium sulfate and concentrated in vacuo. The residue was dissolved in diethyl ether and hydrogen chloride gas was bubbled through the solution. Evaporation of the solvent and

crystallation of the residue from ethanol/diethyl ether provided 0.36 g of the desired product, m.p. = 87-88°C.
Analysis for $C_{15}H_{23}NO_3 \cdot HCl$:
Calculated: C, 60.10; H, 7.40; N, 4.67;
Found: C, 60.22; H, 7.60; N, 4.68.

Example 75

2-Hydroxy-4-[4-(4-morpholino)butoxy]-5-allylacetophenone hydrochloride

A mixture of 1.64 g of 4-(4-bromobutoxy)-5-allyl-2-hydroxyacetophenone and 15 ml of morpholine were stirred overnight. Diethyl ether was added and the solution was washed twice with a saturated sodium chloride solution. The organic layer was extracted with cold dilute hydrochloric acid. The acid solution was made basic with potassium carbonate and extracted with diethyl ether. The organic layer was dried over sodium sulfate, filtered, and concentrated in vacuo. The product was treated with hydrogen chloride gas in diethyl ether. Crystallization from ethanol/diethyl ether provided 1.28 g of the desired title product, m.p. = 140-141°C.
Analysis for $C_{19}H_{27}NO_4 \cdot HCl$:
Calculated: C, 61.70; H, 7.63; N, 3.79;
Found: C, 60.14; H, 7.06; N, 3.55.

Example 76

4-[4-(Dimethylamino)butoxy]-2-hydroxy-5-allylacetophenone hydrochloride

Following the procedure of Example 75, 1.32 g of the desired title product was recovered when 100 g of dimethylamine were substituted for morpholine, m.p. = 88-90°C.
Analysis for $C_{17}H_{25}NO_3 \cdot HCl$:
Calculated: C, 62.28; H, 7.99; N, 4.27;
Found: C, 61.06; H, 9.19; N, 5.53.

Example 77

N-[4-(4-Acetyl-5-hydroxy-2-propylphenoxy)butyl]acetamide

Approximately 100 milligrams of 4-(4-aminobutoxy)-5-propyl-2-hydroxyacetophenone hydrochloride were treated with acetyl chloride and sodium bicarbonate in acetone. After stirring 48 hours, the mixture was concentrated in vacuo and partitioned between ethyl acetate and saturated sodium chloride solution. The organic layer was separated, washed with dilute hydrochloric acid followed by a saturated potassium carbonate solution, and concentrated to dryness. Preparative thin layer chromatography over silica gel eluting with 1:1 ethyl acetate/hexane provided 10 mg of the desired title product.
Analysis for $C_{17}H_{25}NO_4$:
Calculated: C, 66.43; H, 8.20; N, 4.56;
Found: C, 65.73; H, 7.60; N, 3.83.

Example 78

2-Hydroxy-4-[6-(methylthio)hexyloxy]-5-allylacetophenone

Ten grams of methane thiol in 25 ml of dimethylformamide were treated with 0.83 g of a 50% sodium hydride dispersion in oil at 0°C under a nitrogen atmosphere. The reaction was brought to room temperature, and, after stirring two hours, a mixture of 1.5 g of 4-(6-bromohexyloxy)-5-allyl-2-hydroxyacetophenone in 25 ml of dimethylformamide was added. The mixture was heated under a nitrogen atmosphere at 70°C for 24 hours. After cooling to room temperature, the mixture was partitioned between ethyl acetate and dilute hydrochloric acid. The organic layer was separated, dried over sodium sulfate, and concentrated in vacuo. The residue was purified by preparative thin layer chromatography over silica gel providing 0.73 g of the desired title product, m.p. = 42°C.
Analysis for $C_{18}H_{26}O_3S$:
Calculated: C, 67.04; H, 8.13; S, 9.94;
Found: C, 67.13; H, 8.39; S, 10.16.

Examples 79 and 80

2-Hydroxy-4-[6-(methylsulfinyl)hexyloxy]-5-allylacetophenone and 2-hydroxy-4-[6-(methylsulfonyl)-hexyloxy]-5-allylacetophenone

A mixture of 0.322 g of 2-hydroxy-4-[6-(methylthio)hexyloxy]-5-allylacetophenone in 50 ml of methylene chloride was cooled to 0°C. A solution of 0.216 g of 80% meta-chloroperbenzoic acid in 50 ml of methylene chloride was added over a 30 minute period. The reaction mixture was allowed to warm to room temperature and stirred overnight. The reaction mixture was concentrated in vacuo and partitioned between ethyl acetate and a sodium bicarbonate solution. The organic layer was separated, dried over sodium sulfate, and concentrated in vacuo. The residue was purified by preparative thin layer chromatography eluting with 1:1 ethyl acetate/hexane providing 30 mg of the title sulfoxide and 70 mg of the title sulfone.

79. 2-Hydroxy-4-[6-(methylsulfinyl)hexyloxy]-5-allylacetophenone.
Analysis for $C_{18}H_{26}O_4S$:
Calculated: C, 63.88: H, 7.74: S, 9.47:
Found: C, 62.29: H, 7.45: S, 10.42.
80. 2-Hydroxy-4-[6-(methylsulfonyl)hexyloxy]-5-allylacetophenone.
Analysis for $C_{18}H_{26}O_5S$:
Calculated: C, 60.99; H, 7.39; S, 9.05;
Found: C, 60.75; H, 7.25; S, 9.17.

Examples 81-83

The following compounds were prepared from the corresponding bromo derivative according to the procedures of Examples 78-80.

81. 2-Hydroxy-4-[6-(methylthio)hexyloxy]-5-ethylacetophenone, 77.8% yield, m.p. = 52-53°C. NMR.
82. 2-Hydroxy-4-[6-(methylsulfinyl)hexyloxy]-5-ethylacetophenone, 17% yield, m.p. = 87-90°C.
Analysis for $C_{17}H_{26}O_4S$:
Calculated: C, 62.55; H, 8.03; S, 9.82;
Found: C, 62.46; H, 7.78; S, 9.65.
83. 2-Hydroxy-4-[6-(methylsulfonyl)hexyloxy]-5-ethylacetophenone, 70% yield, m.p. = 124-126°C.
Analysis for $C_{17}H_{26}O_5S$:
Calculated: C, 59.62; H, 7.65; S, 9.36;
Found: C, 59.66; H, 7.57; S, 9.42.

Example 84

3-[(4-Acetyl-5-hydroxy-2-ethylphenoxy)methyl]benzoic acid

A mixture of 1.5 g of 3-[(4-acetyl-5-hydroxy-2-ethylphenoxy)methyl]benzonitrile, 1.14 g of potassium hydroxide, 75 ml of ethanol and 75 ml of water was heated at reflux overnight. The mixture was acidified with hydrochloric acid and extracted with ethyl acetate. The organic layer was extracted with 1N sodium hydroxide. The base layer was acidified with hydrochloric acid and extracted with ethyl acetate. The organic layer was dried over sodium sulfate and concentrated in vacuo providing 1.06 g of the desired title product, m.p. = 161-164°C.
Analysis for $C_{18}H_{18}O_5$:
Calculated: C, 68.78; H, 5.77;
Found: C, 68.62; H, 5.65.

Examples 85-87

The following acids were prepared from the corresponding nitriles according to the procedure of Example 84.

85. 7-(4-Acetyl-2-ethyl-5-hydroxyphenoxy)heptanoic acid, 89% yield, m.p. = 86-88°C.
Analysis for $C_{17}H_{24}O_5$:
Calculated: C, 66.21; H, 7.85;
Found: C, 65.31; H, 8.00.
86. 6-(4-Acetyl-2-allyl-5-hydroxyphenoxy)hexanoic acid, 73% yield, m.p. = 90-92°C. NMR.
87. 7-(4-Acetyl-2-ethyl-5-hydroxyphenoxy)-2,2-dimethylheptanoic acid, 28% yield, m.p. = 110-112°C.
Analysis for $C_{19}H_{28}O_5$:
Calculated: C, 67.83; H, 8.39;
Found: C, 66.68; H, 8.41.

Example 88

5-[(4-Cyanobutoxy)-5-ethyl-2-hydroxybenzene]-5-oxopentanoic acid

To a solution of 347 mg of 5-[(4-cyanobutoxy)-5-ethyl-2-hydroxybenzene]-5-oxopentanoic acid, methyl ester in 6 ml of acetone were added 1.5 ml of water followed by 50 mg of lithium hydroxide. After stirring for 1 hour, 2 ml of water were added and the reaction was stirred an additional 3 hours. The solution was concentrated in vacuo, diluted with water to 30 ml, and treated with dilute hydrochloric acid. The resulting precipitate was shaken with ethyl acetate. The organic layer was separated and extracted into a potassium carbonate solution. The potassium carbonate layer was treated with dilute hydrochloric acid and the resulting precipitate was extracted into ethyl acetate. The organic layer was dried over sodium sulfate, filtered, and concentrated in vacuo. The resulting solid was crystallized from diethyl ether providing the 0.19 g of the desired title product, m.p. = 140-142°C.
Analysis for $C_{18}H_{23}NO_5$:
Calculated: C, 64.85; H, 6.95; N, 4.20;
Found: C, 64.73; H, 7.18; N, 4.46.

Examples 89-92

The following acids were prepared according to the procedure of Example 88 from the corresponding esters.

89. 2-[4-(4-Cyanobutoxy)-5-ethyl-2-hydroxybenzoyl]benzoic acid, 14% yield, m.p. = 162-166°C.
Analysis for $C_{21}H_{21}NO_5$:
Calculated: C, 68.65; H, 5.76; N, 3.81;
Found: C, 68.38; H, 5.51; N, 4.10.
90. 6-[(4-Cyanobutoxy)-5-ethyl-2-hydroxybenzene]-6-oxohexanoic acid, 63% yield, m.p. = 120–121°C.
Analysis for $C_{19}H_{25}NO_5$:
Calculated: C, 65.69; H, 7.25; N, 4.03;
Found: C, 65.70; H, 6.99; N, 3.77.
91. 4-[4-(4-Cyanobutoxy)-5-ethyl-2-hydroxybenzoyl]benzoic acid, 2% yield. NMR.
92. 3-[4-(4-Cyanobutoxy-5-ethyl-2-hydroxybenzoyl]benzoic acid, 49% yield, m.p. = 136–139°C.
Analysis for $C_{21}H_{21}NO_5$:
Calculated: C, 68.65; H, 5.76; N, 3.81;
Found: C, 68.87; H, 5.91; N, 3.53.

Example 93

3-[(4-Acetyl-2-ethyl-5-hydroxyphenoxy)methyl]-benzoic acid, methyl ester
A mixture of 0.25 g of 3-[(4-acetyl-2-ethyl-5-hydroxyphenoxy)methyl]benzoic acid, six drops of sulfuric acid, and 50 ml of methanol were heated at reflux overnight. The solvent was removed in vacuo and the residue was dissolved in ethyl acetate. The organic solution was washed with base, and concentrated in vacuo. The residue was crystallized from acetone providing 210 mg of the desired title product, m.p. 132.5°C.
Analysis for $C_{19}H_{20}O_5$:
Calculated: C, 69.5; H, 6.14; N, O;
Found: C, 68.6; H, 6.18; N, 1.19.

Example 94

6-(4-Acetyl-5-hydroxy-2-allylphenoxy)hexanoic acid, methyl ester

The title product was prepared from the corresponding acid following the procedure of Example 93, 78.4% yield, m.p. = 64-65°C.
Analysis for $C_{18}H_{24}O_5$:
Calculated: C, 67.48; H, 7.55;
Found: C, 67.08; H, 6.98.

Example 95

6-(4-Acetyl-5-hydroxy-2-allylphenoxy)hexanoyl chloride

A mixture of 3.06 g of 6-(4-acetyl-5-hydroxy-2-allylphenoxy)hexanoic acid, 1.74 ml of oxalyl chloride, 100 ml of methylene chloride, and ten drops of dimethylformamide was mixed at 0°C and allowed to warm to room temperature. After stirring for 4 hours, the solvents were removed by evaporation to provide the

room temperature. After stirring for 4 hours, the solvents were removed by evaporation to provide the desired title product. NMR.

Example 96

6-(4-Acetyl-5-hydroxy-2-allylphenoxy)hexanamide

Ammonia gas was bubbled into a solution of approximately 3 millimoles of the acid chloride from Example 95 in 25 ml of methylene chloride. After 10 minutes, gas introduction was discontinued and the reaction was stirred overnight. The reaction mixture was concentrated in vacuo and then partitioned between ethyl acetate and dilute hydrochloric acid to which sodium chloride had been added. The organic layer was separated, washed with a potassium carbonate solution, dried over sodium sulfate, and concentrated to dryness. The residue was purified by preparative thin layer chromatography eluting with ethyl acetate to provide 110 mg of the desired title product, m.p. = 122-124°C.
Analysis for $C_{17}H_{23}NO_4$:
Calculated: C, 66.86; H, 7.59; N, 4.59;
Found: C, 67.13; H, 7.57; N, 4.88.

Examples 97-100

The following amide derivatives were prepared from the corresponding acid chloride and the appropriate amine following the procedure of Example 96.

97. 6-(4-Acetyl-5-hydroxy-2-allylphenoxy)-N,N-dimethylhexanamide, 21% yield, m.p. = 76°C.
Analysis for $C_{19}H_{27}NO_4$:
Calculated: C, 68.44; H, 8.16; N, 4.20;
Found: C, 69.65; H, 8.39; N, 4.36.

98. 7-(4-Acetyl-2-ethyl-5-hydroxyphenoxy)-N,N-dimethylheptanamide, 48% yield, m.p. = 127-129°C.
Analysis for $C_{19}H_{29}NO_4$:
Calculated: C, 68.03; H, 8.71 N, 4.18;
Found: C, 67.74; H, 8.70; N, 4.20.

99. 7-(4-Acetyl-2-ethyl-5-hydroxyphenoxy)-N-methylheptanamide, 6% yield, m.p. = 55-57°C.
Analaysis for $C_{18}H_{27}NO_4$:
Calculated: C, 67.26; H, 8.47; N, 4.36;
Found: C, 67.05; H, 8.23; N, 4.21.

100. 6-(4-Acetyl-5-hydroxy-2-allylphenoxy)-N-hydroxyhexanamide, 7% yield, m.p. = 90-94°C.
Analysis for $C_{17}H_{23}NO_5$:
Calculated: C, 63.54; H, 7.21; N, 4.36;
Found: C, 63.69; H, 7.05; N, 4.61.

Example 101

7-(4-Acetyl-2-ethyl-5-hydroxyphenoxy)-2,2-dimethylheptanamide

A mixture of 500 mg of 7-(4-acetyl-2-ethyl-5-hydroxyphenoxy)-2,2-dimethylheptanenitrile in 150 ml of 5N sodium hydroxide and 50 ml of ethanol was heated at reflux overnight. The ethanol was removed by evaporation, 100 ml of water were added, and the solution was extracted with diethyl ether. The organic layer was separated, dried, and concentrated in vacuo. The residue was purified by preparative thin layer chromatography over silica gel eluting with 1:1 ethyl acetate/hexane to provide 34 mg of the desired title product, m.p. = 73-74°C.

Proton nmr and mass spectral analyses were consistent with the structure of the desired product.

Examples 102 and 103

7-(4-Acetyl-5-hydroxy-2-allylphenoxy)-2-heptanone and 2-hydroxy-4-(6-hydroxy-6-methylheptyloxy)-5-allylacetophenone

A mixture of approximately 5 millimoles of 6-(4-acetyl-5-hydroxy-2-allylphenoxy)hexanoyl chloride in 100 ml of diethyl ether was cooled to -78°C by means of a dry ice/acetone bath. Under a nitrogen atmosphere, 8.56 ml of a 1.8N solution of methyllithium in diethyl ether was added. The reaction was allowed to warm to -50°C. over a one hour period. The mixture was poured into dilute hydrochloric acid and extracted with ethyl acetate. The organic layer was washed with a saturated sodium chloride solution, dried over sodium sulfate, and concentrated in vacuo. The residue was purified by preparative thin layer chromatography over silica gel eluting with 40% ethyl acetate in hexane providing 130 mg of the title carbinol and 30 mg of the title ketone.

102. 7-(4-Acetyl-5-hydroxy-2-allylphenoxy)-2-heptanone.
Analysis for $C_{18}H_{24}O_4$:
Calculated: C, 71.03; H, 7.95;
Found: C, 70.75; H, 7.86.

103. 2-Hydroxy-4-(6-hydroxy-6-methylheptyl-oxy)-5-allylacetophenone.
Analysis for $C_{18}H_{24}O_4$:
Calculated: C, 71.22; H, 8.81;
Found: C, 70.95; H, 8.95.

Example 104

5-(4-Benzoyl-5-hydroxy-2-allylphenoxy)pentanenitrile

A mixture of 0.22 g of 5-(4-benzoyl-5-methoxy-2-allylphenoxy)pentanenitrile, 2.52 ml of a 1M solution of boron tribromide in dichloromethane, and 50 ml of methylene chloride were stirred at -78°C for two hours. The solution was poured into a cold saturated ammonium chloride solution and ice. The mixture was extracted with ethyl acetate, the organic layer was washed with a cold saturated ammonium chloride solution followed by a saturated sodium chloride solution, dried and concentrated in vacuo. The residue was purified by preparative thin layer chromatography over silica gel eluting with 1:1 ethyl acetate/hexane providing 50 mg of the desired title product, m.p. = 60-62°C. NMR, MS.

Examples 105-107

The following compounds were prepared from the corresponding methoxy derivatives according to the procedure of Example 104.

105. 5-[5-Hydroxy-4-(1-oxopropyl)-2-allylphenoxy]pentanenitrile, 4% yield. NMR, MS.
106. 5-[4-(4-Cyanobutoxy)-2-hydroxy-5-allylphenyl)-5-oxopentanoic acid, 11% yield. NMR, MS.
107. 5-[5-Hydroxy-4-(1-oxodecyl)-2-allylphenoxy]pentanenitrile, 4% yield, m.p. = 49-51°C. NMR, MS.

Example 108

5-Ethyl-2-hydroxy-4-[6-methyl-6-(1H-tetrazol-5-yl)heptyloxy]acetophenone

A mixture of 317 mg of 7-(4-acetyl-2-ethyl-5-hydroxyphenoxy)-2,2-dimethylheptanenitrile and 996 mg of tributyltinazide was heated at 80°C for 9 days. After cooling, the reaction mixture was stirred with a few milliliters of methanol for 15 minutes. The mixture was concentrated in vacuo and the residue was purified by high pressure liquid chromatography over silica gel eluting with a 0-70% ethyl acetate in hexane gradient with 0-1% acetic acid added. The appropriate fractions were combined and concentrated in vacuo and the resulting residue was crystallized from methylene chloride/hexane to provide 16 mg of the desired title product, m.p. = 160-162°C. NMR, MS.

Example 109

2-Hydroxy-5-allyl-4-[4-(1H-tetrazol-5-yl)-butoxy]acetophenone

A mixture of 8.19 g of 5-(4-acetyl-5-hydroxy-2-allylphenoxy)pentanenitrile, 4.86 g of ammonium chloride, and 5.85 g of sodium azide in 50 ml of dimethylformamide was heated at 120°C overnight. An additional 4.86 g of ammonium chloride and 5.85 g of sodium azide were added and the reaction was stirred an additional 6 hours. The solvent was removed in vacuo and the residue was partitioned between ethyl acetate and dilute cold hydrochloric acid. The organic layer was extracted with a potassium carbonate solution. The aqueous layer was acidified with dilute hydrochloric acid and extracted with ethyl acetate. The organic layer was dried over sodium sulfate, filtered, and concentrated to dryness. Crystallization of the residue from diethyl ether/hexane provided 1.1 g of the desired title product, m.p. = 140-141°C.
Analysis for $C_{16}H_{20}N_4O_3$:
Calculated: C, 60.75; H, 6.37; N, 17.71;
Found: C, 60.55; H, 6.57; N, 17.61.

Example 110

5-Ethyl-2-hydroxy-4-[4-(1H-tetrazol-5-yl)butoxy]acetophenone

The title compound was prepared by the procedure of Example 109 from the corresponding nitrile in 29% yield, m.p. = 171-172°C.

Analysis for $C_{15}H_{20}N_4O_3$:
Calculated: C, 59.20; H, 6.62; N, 18.41;
Found: C, 58.95; H, 6.35; N, 18.44.

Example 111

5,5'-[(4-Acetyl-6-methoxymethyl-1,3-phenylene)bis(oxy)]bis[pentanenitrile]

A mixture of 688 mg of 5,5'-[(4-acetyl-6hydroxymethyl-1,3-phenylene)bis(oxy)]bis[pentanenitrile] in 15 ml of dimethylformamide was added to a suspension of 0.184 g of a 50% oil dispersion of sodium hydride and 0.62 ml of methyl iodide in 45 ml of dimethylformamide at 0°C. The reaction was allowed to come to room temperature and stirred overnight. The reaction mixture was partitioned between ethyl acetate and dilute hydrochloric acid to which sodium chloride had been added. The organic layer was separated, dried over sodium sulfate, and concentrated in vacuo. The resulting residue was purified by high pressure liquid chromatography over silica gel eluting with a 0-70% ethyl acetate in hexane gradient to provide 110 mg of the desired title product. NMR, MS.

Example 112

7-(4-Acetyl-5-methoxy-2-methoxymethylphenoxy)heptanenitrile

The title product was prepared in 25.1% yield from the corresponding hydroxymethyl analog according to the procedure of Example 111. NMR.

Example 113

5-(4-Acetyl-5-methoxy-2-allylphenoxy)pentanenitrile

A mixture of 1.365 g of 5-(4-acetyl-5-hydroxy-2-allylphenoxy)pentanenitrile, 0.966 g of potassium carbonate, and 1.42 g of methyl iodide in 50 ml of dimethylformamide was stirred together at room temperature for 5 hours. The mixture was partitioned between dilute hydrochloric acid and ethyl acetate. The organic layer was separated, dried over sodium sulfate, and concentrated in vacuo to provide 1.2 g of the desired title product, m.p. = 40-42°C. NMR, MS.

Example 114

3-[(4-Acetyl-2-ethyl-5-methoxyphenoxy)methyl]benzonitrile

The title compound was prepared in 30% yield from the compound of Example 55 and methyl iodide according to the procedure of Example 16, m.p. 124-126°C.
Analysis for $C_{19}H_{19}NO_3$:
Calculated: C, 73.77; H, 6.19; N, 4.53;
Found: C, 73.51; H, 6.22; N, 4.51.

Example 115

5-(4-Acetyl-5-methoxy-2-allylphenoxy)pentanenitrile

The title compound was prepared in 14% yield from the corresponding phenol according to the procedure of Example 111, m.p. = 41-42°C.
Analysis for $C_{17}H_{21}NO_3$:
Calculated: C, 71.06; H, 7.37; N, 4.87;
Found: C, 70.09; H, 7.17; N, 4.83.

Example 116

2-Hydroxy-5-propyl-4-[4-(1-tetrazol-5-yl)-butoxy]acetophenone

One gram of 2-hydroxy-5-allyl-4-[4-(1H-tetrazol-5-yl)butoxy]acetophenone was hydrogenated in the presence of 0.5 g of 5% palladium on carbon in 50 ml of ethanol for 1 hour. The reaction mixture was filtered and concentrated in vacuo. The residue was crystallized from diethyl ether to provide 0.7 g of the desired title product, m.p. = 135-136°C.
Analysis for $C_{16}H_{22}N_4O_3$:
Calculated: C, 60.36; H, 6.97; N, 17.60;
Found; C, 60.65; H, 7.12; N, 17.46.

Examples 117 and 118

The following compounds were prepared according to the procedure of Example 116 from the corresponding allyl derivatives.

117. 6-(4-Acetyl-5-hydroxy-2-propylphenoxy)hexanoic acid, 60% yield, m.p. = 90-92°C.
Analysis for $C_{17}H_{22}O_5$:
Calculated: C, 66.65; H, 7.24;
Found: C, 66.48; H, 7.29.
118. 5-(4-Acetyl-5-hydroxy-2-propylphenoxy)pentanenitrile, 77% yield, m.p. = 64-66°C.
Analysis for $C_{16}H_{21}NO_3$:
Calculated: C, 69.79; H, 7.69; N, 5.09;
Found: C, 70.00; H, 7.56; N, 5.27.

Examples 119-120

2-Hydroxy-4-[4-(1-methyl-1H-tetrazol-5-yl)-butoxy]-5-allylacetophenone and 2-hydroxy-4-[4-(2-methyl-2H-tetrazol-5-yl)butoxy]-5-allylacetophenone

A mixture of 632 mg of 2-hydroxy-4-[4-(1H-tetrazol-5-yl)butoxy]-5-allylacetophenone, 0.249 ml of methyl iodide, 0.29 g of potassium carbonate and 50 ml of dimethylformamide were allowed to react overnight. The mixture was then heated to 50°C under a nitrogen atmosphere for 4 hours, cooled, and partitoned between dilute hydrochloric acid and ethyl acetate. The organic layer was separated, dried over sodium sulfate, and concentrated in vacuo. The residue was purified by preparative thin layer chromatography over silica gel eluting with 100% ethyl acetate providing 170 mg of the title 1-methyl derivative and 100 mg of the desired 2-methyl compound.

119. 2-Hydroxy-4-[4-(1-methyl-1H-tetrazol-5-yl)butoxy]-5-allylacetophenone, m.p. = 38-40°C.
Analysis for $C_7H_{22}N_4O_3$:
Calculated: C, 61.80; H, 6.71; N, 16.74;
Found: C, 61.53; H, 6.99; N, 16.74.
120. 2-Hydroxy-4-[4-(2-methyl-2H-tetrazol-5-yl)butoxy]-5-allylacetophenone, m.p. = 80-81°C.
Analysis for $C_{17}H_{22}N_4O_3$:
Calculated: C, 61.80; H, 6.71; N, 16.96;
Found: C, 62.05; H, 6.97; N, 16.77.

Examples 121-125

The following compounds were prepared according to the procedure of Examples 119-120 from the corresponding tetrazole derivative.

121. 2-Hydroxy-4-{[3-(2-methyl-2H-tetrazol-5-yl)phenyl}methoxy]-5-allylacetophenone, 10% yield. NMR.
122. 2-Hydroxy-4-[4-(1-methyl-1H-tetrazol-5-yl)butoxy]-5-ethylacetophenone, 38% yield, m.p. = 90-91°C.
Analysis for $C_{16}H_{22}N_4O_3$:
Calculated: C, 60.36; H, 6.97; N, 17.60;
Found: C, 60.69; H, 7.25; N, 17.27.
123. 2-Hydroxy-4-[4-(2-methyl-2H-tetrazol-5-yl)butoxy]-5-ethylacetophenone, 27% yield, m.p. = 65-66°C.
Analysis for $C_{16}H_{22}N_4O_3$:
Calculated: C, 60.36; H, 6.97; N, 17.60;
Found: C, 60.53; H, 6.59; N, 17.43.

Example 124

5,5'-{[4-Acetyl-6-(hydroxymethyl)-1,3-phenylene]bis(oxy)}bis[pentanenitrile]

Two grams of 5,5'-{[4-acetyl-6-formyl-1,3-phenylene]bis(oxy)}bis[pentanenitrile] were hydrogenated in the presence of 1 g of 10% palladium on carbon in 400 ml of ethanol. The reaction mixture was filtered, concentrated in vacuo and triturated with diethyl ether to provide 1.6 g of the desired product, m.p. = 98-99°C.

Analysis for $C_{19}H_{24}N_2O_4$:
Calculated: C, 66.26; H, 7.02; N, 8.13;
Found: C, 65.96; H, 6.76; N, 8.01.

Example 125

5-[4-Acetyl-5-hydroxy-2-(2-hydroxypropyl)phenoxy]pentanenitrile

A. Preparation of 5-[4-acetyl-5-hydroxy-2-(2,3-epoxypropyl)phenoxy]pentanenitrile.

To a solution of 5.46 g of 5-[4-acetyl-5-hydroxy-2-allylphenoxy]pentanenitrile in 100 ml of methylene chloride were added 6.1 g of 85% meta-chloroperbenzoic acid. After stirring for 5 hours, the reaction mixture was concentrated in vacuo. The residue was partitioned between ethyl acetate and a cold sodium bicarbonate solution. The organic layer was separated, dried over sodium sulfate, and concentrated in vacuo to approximately 35 ml. The solution was placed in a freezer overnight providing 4.08 g of the crystalline title product which were recovered by filtration, m.p. 86-87°C.

B. Preparation of 5-[4-acetyl-5-hydroxy-2-(2-hydroxypropyl)phenoxy]pentanenitrile.

The epoxide intermediate from Example 125A above (0.9 g) was hydrogenated in the presence of 0.5 g of 5% palladium on carbon in 50 ml of ethanol. Filtration of the reaction mixture and crystallization from diethyl ether provided 190 mg of the desired title product, m.p. = 86-88°C.
Analysis for $C_{16}H_{21}NO_4$:
Calculated: C, 65.96; H, 7.27; N, 4.81;
Found: C, 65.79; H, 4.32; N, 4.56.

Example 126

5,5'-{[4-Acetyl-6-(chloromethyl)-1,3-phenylene]bis(oxy)}bis[pentanenitrile]

The compound of Example 124 (688 mg) was dissolved in 50 ml of dimethylformamide. To this solution were added 0.29 ml of collidine followed by 0.252 g of lithium chloride. The reaction was cooled by means of an external ice bath and 0.507 ml of methane sulfonyl chloride were added. After stirring for 2 hours, the reaction was allowed to warm to room temperature. The mixture was poured into cold dilute hydrochloric acid to which sodium chloride had been added and the solution was extracted with ethyl acetate. The organic layer was washed twice with cold hydrochloric acid/sodium chloride, dried over sodium sulfate, and concentrated in vacuo. Concentration of the solution provided a residue which was crystallized from methylene chloride/hexane to provide 180 mg of the desired title product, m.p. = 100-110°C (decomposition).
Analysis for $C_{19}H_{23}ClN_2O_3$:
Calculated: C, 67.02; H, 7.31; N, 7.82;
Found: C, 67.02; H, 7.04; N, 7.73.

Example 127

7-[4-Acetyl-5-hydroxy-2-(chloromethyl)phenoxy]heptanenitrile

The title product was prepared in 70% yield from the corresponding hydroxymethyl compound according to the procedure of Example 126. NMR.

Example 128

7-[4-Acetyl-5-hydroxy-2-(methylthiomethyl)phenoxy]heptanenitrile

A mixture of 600 mg of the compound from Example 127 was dissolved in 50 ml of methylene chloride and cooled to 0°C. Fifteen grams of methanethiol were added followed by the addition of 2 ml of triethylamine. The reaction was allowed to warm to room temperature and was stirred overnight. The reaction mixture was concentrated in vacuo and partitioned between ethyl acetate and an acidic sodium chloride solution. The organic layer was separated, dried over sodium sulfate, and concentrated in vacuo. The residue was purified by preparative thin layer chromatography over silica gel eluting with 40% ethyl acetate in hexane providing 200 mg of the desired title product, m.p. = 78°C.
Analysis for $C_{17}H_{23}NO_3S$:
Calculated: C, 63.52; H, 7.21; N, 4.36; S, 9.98;
Found: C, 63.64; H, 6.98; N, 4.60; S, 9.71.

Example 129

7-[4-Acetyl-5-hydroxy-2-(methoxymethyl)phenoxy]heptanenitrile

Approximately 3 millimoles of the chloro compound of Example 127 were treated with 0.624 g of silver perchlorate in 50 ml of methanol at 0°C. The reaction mixture was allowed to warm to room temperature over a 2 hour period. The mixture was then partitioned between ethyl acetate and an acidic sodium chloride solution. The organic layer was separated, dried over sodium sulfate, filtered, and concentrated in vacuo. The residue was purified by preparative thin layer chromatography over silica gel eluting with 1:1 ethyl acetate/hexane providing 50 mg of the title compound. NMR, MS.

Example 130

5-(4-Benzoyl-2-ethyl-5-hydroxyphenoxy)pentanenitrile

Following the procedure of Example 16, 0.726 g of 5-ethyl-2,4-dihydroxybenzophenone and 0.53 g of 5-bromopentanenitrile were allowed to react and provided 0.37 g of the desired title product, m.p. = 42-44°C.
Analysis for $C_{20}H_{21}NO_3$:
Calculated: C, 74.28; H, 6.55; N, 4.33;
Found: C, 74.14; H, 6.62; N, 4.51.

Example 131

5-(4-Acetyl-5-hydroxy-2-allylphenoxy)pentanoic acid

When 2 g of 5-(4-acetyl-5-hydroxy-2-allylphenoxy)pentanenitrile were hydrolyzed following the procedure of Example 84, 1.98 g of the desired title product were recovered, m.p. = 110-111°C.
Analysis for $C_{16}H_{20}O_5$:
Calculated: C, 65.97; H, 6.57;
Found: C, 65.86; H, 6.81.

Example 132

5-(4-Acetyl-5-hydroxy-2-propylphenoxy)pentanoic acid

The compound of Example 131 (1.46 g) was added to 150 ml of ethanol and hydrogenated in the presence of 1.5 g of 5% palladium on carbon. After the theoretical amount of hydrogen was taken up, the reaction was discontinued, the reaction mixture was filtered. The filtrate was concentrated in vacuo and provided 1.49 g of the desired title product,. m.p. = 106°C.
Analysis for $C_{16}H_{22}O_5$:
Calculated: C, 65.51; H, 7.22;
Found: C, 66.77; H, 8.12.

Example 133

5-(4-Acetyl-2-ethyl-5-hydroxyphenoxy)-2,2-dimethylpentanenitrile

Following the procedure of Example 68, 4.52 g of 4-(3-bromopropoxy)-5-ethyl-2-hydroxyacetophenone and 1.36 ml of isobutyronitrile were reacted to provide 1.72 g of the desired title compound, m.p. = 55-56°C.
Analysis for $C_{17}H_{23}NO_3$:
Calculated: C, 70.56; H, 8.01; N, 4.84;
Found: C, 69.19; H, 8.05; N, 4.33.

Examples 134-135

5-(4-Acetyl-2-ethyl-5-hydroxyphenoxy)-2,2-dimethylpentanoic acid and 5-(4-acetyl-2-ethyl-5-hydroxyphenoxy)-2,2-dimethylpentanamide

Following the procedure of Example 84, 114 mg of the nitrile of Example 133, 15 ml of 5N sodium hydroxide, and 50 ml of ethanol were heated at reflux for 24 hours. After workup, purification by preparative thin layer chromatography over silica gel provided 11 mg of the title amide and 13 mg of the title acid.

134. 5-(4-Acetyl-2-ethyl-5-hydroxyphenoxy)-2,2-dimethylpentanoic acid, m.p. = 128-131°C.

Analysis for $C_{17}H_{24}O_5$:
Calculated: . C. 66.21; H, 7.85; N, 0;
Found: C, 63.14; H, 7.55; N, 0.60.

135. 5-(4-Acetyl-2-ethyl-5-hydroxyphenoxy)-2,2-dimethylpentamide, m.p. = 104-105°C.
Analysis for $C_{17}H_{25}NO_4$:
Calculated: C, 66.43; H, 8.20; N, 4.56;
Found: C, 65.76; H, 8.57; N, 3.97.

Example 136

1-(5-Ethyl-2-hydroxy-4-{[4-methyl-4-(1H-tetrazol-5-yl)pentyl]oxy}phenyl)ethanone

The title tetrazole was prepared in 48% yield from the nitrile from Example 133 according to the procedure provided in Example 108, m.p. = 156-157°C.
Analysis for $C_{17}H_{24}N_4O_3$:
Calculated: C, 61.43; H, 7.28; N, 16.86;
Found: C, 61.60; H, 7.02; N, 16.80.

Example 137

1-{4-[(5-Aminopentyl)oxy]-5-ethyl-2-hydroxyphenyl}ethanone

A mixture of 2.0 g of 5-(4-acetyl-2-ethyl-5-hydroxyphenoxy)pentanenitrile, 50 ml of acetic acid, and 2.0 g of 10% palladium on carbon were subjected to hydrogenation for approximately 2 hours. The mixture was then filtered and concentrated in vacuo. The residue was triturated with diethyl ether to provide 2 g of the desired title product, m.p. = 75-76°C. NMR.

Example 138

N-[5-(4-Acetyl-2-ethyl-5-hydroxyphenoxy)pentyl]acetamide

In a manner analogous to that taught in Example 77, 265 mg of the amine from Example 137 was acylated with acetyl chloride and pyridine to provide 158 mg of the desired title product, m.p. = 115-116°C.
Analysis for $C_{17}H_{25}NO_4$:
Calculated: C, 66.43; H, 8.20; N, 4.56;
Found: C, 66.24; H, 8.29; N, 4.47.

Example 139

5-(4-Acetyl-5-hydroxy-2-allylphenoxy)pentanenitrile

The title product was prepared in 88% yield from 5-allyl-2,4-dihydroxyacetophenone and 5-bromopentanenitrile following the procedure of Example 16, m.p. = 54°C.
Analysis for $C_{16}H_{19}NO_3$:
Calculated: C, 70.31; H, 7.01; N, 5.12;
Found: C, 69.38; H, 7.54; N, 4.80.

Example 140

1-(5-Ethyl-2-hydroxy-4-{[6-(1H-tetrazol-5-yl)-hexyl]oxy}phenyl)ethanone

The title compound was prepared in 63% yield from the nitrile of Example 20 following the procedure of Example 108, m.p. = 126-127°C.
Analysis for $C_{17}H_{24}N_4O_3$:
Calculated: C, 61.43; H, 7.28; N, 16.86;
Found: C, 61.47; H, 7.11; N, 16.88.

Examples 141-142

5-[4-(4-Acetyl-2-ethyl-5-hydroxyphenoxy)butyl]-2H-tetrazole-2-acetic acid, ethyl ester and 5-[4-(4-acetyl-2-ethyl-5-hydroxyphenoxy)butyl]-1H-tetrazole-1-acetic acid, ethyl ester

The title products were prepared by the method provided in Examples 119-120 employing 2-hydroxy-4-[4-(1H-tetrazol-5-yl)butoxy]-5-ethylacetophenone and ethyl bromoacetate.

141. 5-[4-(4-Acetyl-2-ethyl.-5-hydroxyphenoxy)butyl]-2H-tetrazole-2-acetic acid, ethyl ester, 29% yield, m.p. = 118-121°C.
Analysis for $C_{19}H_{26}N_4O_5$:
Calculated: C, 58.44; H, 6.71; N, 14.35;
Found: C, 58.15; H, 6.56; N, 14.52.

142. 5-[4-(4-Acetyl-2-ethyl-5-hydroxyphenoxy)butyl]-1H-tetrazole-1-acetic acid, ethyl ester, 18% yield, m.p. = 86-87°C.
Analysis for $C_{19}H_{26}N_4O_5$:
Calculated: C, 58.44; H, 6.71;
Found: C, 58.66; H, 6.89.

Examples 143-144

The following acid derivatives were prepared from the corresponding esters of Examples 141-142 upon hydrolysis in aqueous ethanol with potassium hydroxide.

143. 5-[4-(4-Acetyl-2-ethyl-5-hydroxyphenoxy)butyl]-2H-tetrazole-2-acetic acid, 80% yield, m.p. >230°C. NMR, MS.

144. 5-[4-(4-Acetyl-2-ethyl-5-hydroxyphenoxy)butyl]-1H-tetrazole-1-acetic acid, 80% yield, m.p. = 161-162°C.
Analysis for $C_{17}H_{22}N_4O_5$:
Calculated: C, 56.35; H, 6.12; N, 15.46;
Found: C, 56.62; H, 6.30; N, 15.25.

Example 145

7-(4-Acetyl-2-ethyl-5-hydroxyphenoxy)-N-1H-tetrazol-5-ylheptanamide

The acid chloride of 616 mg of 7-(4-acetyl5-hydroxy-2-ethylphenoxy)heptanoic acid was prepared according to the procedure of Example 95. To the acid chloride were added 2.06 g of 5-aminotetrazole hydrate, 16.4 g of sodium bicarbonate, and 100 ml of acetone. The reaction was stirred for 2 days. The solvent was removed in vacuo and the residue was partitioned between ethyl acetate and water. The water layer was separated and acidified with hydrochloric acid and extracted with ethyl acetate. The organic layer was separated, dried over sodium sulfate, filtered, and concentrated invacuo. Crystallization of the residue from methanol/water provided 76 mg of the desired product, m.p. = 196-200°C.
Analysis for $C_{18}H_{25}N_5O_4$:
Calculated: C, 57.59; H, 6.71; N, 18.65;
Found: C, 57.42; H, 6.60; N, 18.38.

Example 146

1-(5-Ethyl-2-hydroxy-4-{[5-(1H-1,2,4-triazol-1-yl)pentyl]oxy}phenyl)ethanone

A mixture of 0.3 g of 5-(4-acetyl-2-ethyl-5-hydroxyphenoxy)pentyl bromide, 0.06 g of triazole, and 0.13 g of potassium carbonate in dimethylformamide was heated at 80-90°C for approximately 18 hours. The mixture was poured into acidic water and extracted with ethyl acetate and methylene chloride. The extracts were combined, washed with dilute sodium hydroxide solution, dried over sodium sulfate, and concentrated in vacuo. The residue was twice crystallized from ethyl acetate/hexane to provide the title product having a melting point of 87°C.
Analysis for $C_{17}H_{23}N_3O_3$:
Calculated: C, 64.33; H, 7.30; N, 13.24;
Found: C, 64.07; H, 7.23; N, 13.06.

Example 147

4-[(4-Acetyl-2-ethyl-5-hydroxyphenoxy)methyl]-2-hydroxybenzoic acid, ethyl ester

A mixture of 0.75 g of 2,4-dihydroxy-5-ethylacetophenone, 1.23 g of ethyl 2-acetoxy-4-bromomethylbenzoate, 0.57 g of potassium carbonate, and a catalytic amount of potassium iodide were heated at reflux in methyl ethyl ketone overnight. The hot mixture was filtered and the filtrate concentrated to dryness. Crystallization of the residue from ethyl acetate/hexane provided. 130 mg of the desired title product, m.p. 145-146°C.
Analysis for $C_{20}H_{22}O_6$:
Calculated: C, 67.02; H, 6.18;
Found: C, 68.16; H, 6.15.

Example 148

4-[(4-Acetyl-2-ethyl-5-hydroxyphenoxy)methyl]-2-hydroxybenzoic acid

The title product was prepared in 69% yield from the corresponding ethyl ester upon heating with potassium hydroxide in aqueous ethanol, m.p. 214-216°C.
Analysis for $C_{18}H_{18}O_6$:
Calculated: C, 65.45; H, 5.49;
Found: C, 65.67; H, 5.60.

The compounds of this invention have demonstrated activity in vivo in various test systems designed to detect effective antiinflammatory agents. These pharmacodynamic effects were demonstrated in the following test systems.

Carrageenin Assay

The compounds were evaluated for antiinflammatory activity in the test method described by C.A. Winter, Proc. Soc. Exp. Biol. Med., 111, 544 (1962). In this test, inflammation is created by injecting carrageenin into the hind paws of rats. Test compounds are administered prior to injection to determine percent inhibition of the subsequent inflammation in comparison with control animals. The results are reported in Table I.

Table I

Antiinflammatory Activity in the Carrageenin Assay

| Compound of Example No. | Percent Inhibition |
|---|---|
| 19 | 26% |
| 20 | 19% |
| 47 | 53–65% |
| 48 | 30% |
| 49 | 43% |
| 50 | 50–82% |
| 51 | 79% |
| 53 | 50–60% |
| 55 | 70% |
| 68 | 33% |
| 82 | 31% |
| 83 | 26% |
| 87 | 15% |
| 100 | 38% |
| 108 | 61% |
| 110 | 64% |
| 122 | 53% |
| 123 | 55% |
| 136 | 41% |
| 140 | 31% |

*Compounds administered at a dose of 50 mg/kg i.p.

Arachidonic Acid-Induced Ear Edema Assay

Female BALB/C mice (Charles River; 18-20 grams) were divided into groups of five. Arachidonic acid was dissolved in ethanol at a concentration of 200 mg/ml. Each mouse received 2 mg/ear of arachidonic acid on the inner surface of the right ear. This dose of phlogistic was applied by an automatic pipette in 10 mcl volumes. The left ear (control) received ethanol. Drugs were applied topically in an ethanol-based

gel to the entire outer surface of both ears one hour prior to arachidonic acid application. Edema was measured by the increase in ear weight. An hour after arachidonic acid solution or vehicle was applied, an ear sample of 7 mm in diameter was obtained from both ears. Ear edema was calculated by subtracting the weight of the right ear sample (treated ear) from left ear (control), dividing by the weight of the left/control ear, and averaging the results for each group of mice according to compound and concentration. The results are reported below in Table II.

Table II

Inhibition of Arachidonic Acid Induced Ear Edema

| Compound of Example No. | Dose* | Percent Inhibition of Ear Swelling |
|---|---|---|
| 20 | 1% | |
| 47 | 0.05% | 52 |
| 48 | 0.05% | 13.6 |
| 49 | 0.05% | 31 |
| 50 | 0.05% | 29–47 |
| 51 | 0.05% | 43 |
| | 0.1% | 31 |
| 53 | 0.05% | 24 |
| 55 | 0.05% | 43–59 |
| | 1% | 57 |
| 56 | 0.05% | 6 |
| 59 | 0.05% | 0 |
| 68 | 1% | 46 |
| 78 | 1% | 46 |
| 82 | 1% | 9 |
| 83 | 0.028% | 59 |
| 87 | 1% | 3 |
| 98 | 1% | 17 |
| 100 | 1% | 18 |
| 108 | 1% | 60 |
| 110 | 1% | 58 |
| 114 | 0.05% | 40 |
| | 0.01% | 44 |
| 121 | 0.05% | 0 |
| 122 | 1% | 10 |
| 123 | 1% | 58 |

*concentration of compound in ethanol gel

Anti IL-1 Activity (Thymocyte Assay)

Thymus from 3-4 week old C3H/HeJ male mice and were passed through a #100 mesh screen to make a single cell suspension. The cells were suspended in RPMI-1640 supplemented with 2 × $10^{-5}$M 2-mercaptoethanol, 3% fetal calf serum, and 1% Penicillin/Streptomycin. The cell suspension was placed in a tissue culture flask and incubated at 37°C in 5% carbon dioxide in air for 1-2 hours. The thymocytes were decanted, leaving adherent cells behind, and the liquid was centrifuged at 1400 rpm for 10 minutes. After decanting the supernatant, the cells were resuspended and counted with a hemocytometer. The density was adjusted to 2 × $10^7$ cells/ml. Fifty µl of the suspension were added to each well of a 96-well microtiter plate, followed by the addition of 25 µl of PHA and 25 µl IL-1 (1:25 dilution of a 10 mcg/ml stock solution) per well.

Each compound to be tested was dissolved in 30% DMSO/70% PBS to make a stock solution of 1 mg/ml. Fifteen µl of each compound were added to 300 µl of media in the serial dilution plate. Twenty-five µl of each drug were added to the test plate in quadruplicate wells accross the plate. The plate was incubated at 37°C in 5% carbon dioxide in air for 48 hours. The wells were pulsed with tritiated thymidine (5

μCi/well) for 4 hours before harvesting on a Skatron cell harvester. The filter discs were placed in mini-vials, scintillation fluid (Tol-Pop) was added, and the vials counted. The percent of counts for each vial compared with control samples wherein no compound was added (vehicles only) was determined for each compound and are reported as Table III below as the percent T-cell suppression.

Table III

Suppression of IL-1 induced T-cell Proliferation

| Compound of Example | Percent T-cell suppression | |
|---|---|---|
| | 10 mcg/ml | 1 mcg/ml |
| 15 | 91 | 33 |
| 20 | 99 | 39 |
| 30 | 91 | 32 |
| 36 | 75 | 15 |
| 38 | 28 | -8 |
| 68 | 99 | 13 |
| 76 | 79 | 39 |
| 82 | 88 | 21 |
| 83 | 43 | 29 |
| 89 | 39 | 9 |
| 90 | 47 | 3 |
| 92 | 38 | 5 |
| 98 | 85 | 14 |
| 107 | 26 | 47 |
| 109 | 56 | 7 |
| 115 | 96 | 29 |
| 122 | 87 | 37 |
| 123 | 90 | 36 |
| 125 | 85 | 30 |
| 130 | 99 | 41 |
| 132 | 59 | 15 |

Mouse Endotoxin Shock Assay

Compounds were given to 18-20 g C57BL/6 male mice using various regimens depending upon mode of administration. Control animals received corresponding vehicles only. The mice were then challenged with 0.2 ml of chromatographically purified 0111:B4 E. coli LPS and galactosamine hydrochloride (GALN) given subcutaneously (back of the neck). GALN was given at an approximate dose of 500 mg/kg whereas the LPS dose ranged between 0.3-1.2 μg/mouse (15-60 μg/kg). Control animals generally die of shock 7-15 hours after administration of LPS/GALN although a small number (0-5%) may die 24-48 hours after such administration. Calculation of the $ED_{50}$ for each compound was performed using the Reed Muench Method for results obtained 48 hours after challenge. Results are summarized in Table IV below.

Table IV

| Mouse Endotoxin Shock Assay | |
|---|---|
| Compound of Example No. | $ED_{50}$ (mg/kg) |
| 47 | 24 |
| 48 | 18.6 |
| 49 | >50 |
| 50 | 9.5 |
| 51 | 27 |
| 54 | 21 |
| 55 | 3.1 |
| 68 | >50 |
| 82 | >50 |
| 83 | >50 |
| 93 | 25 |
| 108 | >50 |
| 122 | 42 |

The compounds of Formula I should be useful in treating any condition, including clinical conditions, which is characterized by the excessive release of leukotriene $B_4$. These conditions include psoriasis, arthritis, chronic lung diseases, inflammatory bowel disease, and other inflammatory states characterized by the infiltration and aggregation of polymorphonuclear leukocytes.

The term "excessive release" of leukotriene $B_4$ refers to an amount of leukotriene sufficient to cause the particular condition associated with such amount. The amount of $LTB_4$ which is considered to be excessive will depend on a variety of factors, including the amount of leukotriene required to cause the particular condition, and the species of the mammal involved. As will be appreciated by those skilled in the art, the success of treating a mammal suffering from or susceptible to a condition characterized by an excessive release of leukotriene $B_4$ with a compound of formula I will be measured by the regression or prevention of the symptoms of the condition.

Leukotriene $B_4$ antagonism was demonstrated by the following test procedure:

Inhibition of Binding of $^3H$-$LTB_4$ to Peripheral Human Neutrophils

The effectiveness of compounds to inhibit the binding of leukotriene $B_4$ to a specific receptor on the membrane of human neutrophils was measured by using an adaptation of a radio-ligand binding assay developed by Goldman and Goetzl, J. Immunol., 129 1600 (1982). Other investigators have developed similar assays (see, e.g., Kreisle, et al., J. Exp. Med., 157, 628 (1983) and Lin, et al., Prostaglandins, 28 837 (1984)).

Cells used in the assay were isolated by standard techniques of centrifugation on Ficoll-Hypaque, dextran 70 sedimentation and hypotonic lysis. The following procedure was used. Freshly-prepared buffy coat layers from two individuals were obtained from a local blood donor center. The cells were mixed and diluted to 484 ml. with phosphate buffered saline containing heparin (10 units/ml) and heat-inactivated calf serum (5%). This was divided into 20 ml. aliquots and the aliquots layered on top of Ficoll-Paque (12 ml.). The material was then centrifuged at 500 g. for 40 minutes at room temperature. The resulting upper layer of platelets and mononuclear cells was discarded. The lower layer containing erythrocytes and neutrophils was retained. Buffer was added (1 ml. per 4 ml. of lower layer) and the suspension mixed. For each milliliter of this mixture, 0.33 ml. of 6% Macrodex was added. After stirring, the cells were allowed to sediment for 1 hour at 37°C. The resulting erythrocyte pellet was discarded and the neutrophil enriched supernatant fluid centrifuged at 500 g. for 10 minutes at 4°C. Erythrocytes still present in this cell pellet were lysed by incubating the cells with 5-8 ml. ice-cold distilled water for 30-45 seconds. Subsequently, the volume was made up to 50 ml. by addition of ice-cold buffer and the cells resuspended. The suspension was then centrifuged at 300 g. for 10 minutes at 4°C. The cells were finally resuspended at a cell density of $2 \times 10^7$ cells/ml in the assay buffer. This buffer consisted of Hanks' balanced salt solution and 0.1% ovalbumin (pH 7.3). This isolation procedure resulted in cell preparations of ≥90% neutrophils and ≥90% viability.

The radio-ligand binding assay was conducted by incubating neutrophils ($1 \times 10^7$ cells) with 0.1-0.2 nM $^3H$-$LTB_4$ (sp. act. 150-220 Curies/mmol) and test compound ($1 \times 10^{-5}$M and $1 \times 10^{-6}$M) for 10 minutes at 4°C. The amount of bound $^3H$-$LTB_4$ was then measured and compared with the amount bound in the absence of test compound. The assay was carried out in microcentrifuge tubes by adding first 10 μl test

compound dissolved in DMSO, followed by adding 20 μl $^3$H-LTB$_4$ diluted in assay buffer, and finally adding 500 μl of the cell sus pension. At the end of the 10 minutes incubation, 300 μl of a mixture of dibutyl and dinonyl phthalate (7:2) were added and the tubes centrifuged for 2 minutes in a microcentrifuge. The radioactivity bound to the cell pellet was measured by scintillation spectroscopy. Appropriate corrections for nonspecific bonding of $^3$H-LTB$_4$ were made. The results are reported in Table V.

Table V

LTB$_4$ Binding Inhibition

| Example No. | Drug Concentration* $10^{-5}$M | $10^{-6}$M | $10^{-7}$M |
|---|---|---|---|
| 15 | 35 | 13 | |
| 16 | 8 | -7 | |
| 18 | 5 | 3 | |
| 19 | 90 | 61 | |
| 20 | 102 | 72 | |
| 21 | 81 | 32 | |
| 23 | 85 | 50 | |
| 24 | 94 | 59 | |
| 25 | 19 | 2 | |
| 26 | 46 | 5 | |
| 28 | 77 | 32 | |
| 29 | 51 | 12 | |
| 30 | 37 | 7 | |
| 31 | 30 | 14 | |
| 36 | 40 | 7 | |
| 37 | 31 | 8 | |
| 38 | 16 | 8 | |
| 39 | 50 | 9 | |
| 40 | 31 | 8 | |
| 41 | 59 | 22 | |
| 42 | 6 | 12 | |
| 44 | 47 | 9 | |

Table V continued

| LTB$_4$ Binding Inhibition | | | |
|---|---|---|---|
| | Drug Concentration* | | |
| Example No. | $10^{-5}$M | $10^{-6}$M | $10^{-7}$M |
| 45 | 51 | 15 | |
| 46 | 72 | 26 | |
| 47 | 32 | 5 | |
| 48 | 22 | 28 | |
| 49 | 47 | 15 | |
| 50 | 67 | 66 | 25 |
| 51 | 24 | 32 | |
| 53 | 5 | 13 | |
| 54 | 42 | 32 | |
| 55 | 60 | 71 | 25 |
| 56 | 42 | 15 | |
| 57 | 73 | 29 | |
| 58 | 67 | 22 | |
| 59 | 58 | 23 | |
| 60 | 12 | 6 | |
| 68 | 84 | 53 | |
| 69 | 88 | 51 | |
| 70 | 50 | 14 | |
| 71 | 30 | 17 | |
| 74 | 0 | 0 | |
| 75 | 0 | 0 | |
| 76 | 0 | 0 | |

Table V continued

| LTB$_4$ Binding Inhibition | | | |
|---|---|---|---|
| | Drug Concentration* | | |
| Example No. | $10^{-5}$M | $10^{-6}$M | $10^{-7}$M |
| 77 | 71 | 23 | |
| 78 | 78 | 38 | |
| 79 | 96 | 51 | |
| 80 | 100 | 62 | |
| 81 | 71–94 | 14–59 | |
| 82 | 91 | 50 | |
| 83 | 98 | 61 | |
| 84 | 81 | 28 | |
| 85 | 86 | 37 | |
| 86 | 73 | 23 | |
| 87 | 104 | 73 | 20 |
| 88 | 19 | –1 | |
| 89 | 11 | 15 | |
| 90 | 1 | –2 | |
| 91 | 26 | 20 | |
| 92 | 40 | 20 | |
| 93 | 64 | 22 | |
| 94 | 106 | 64 | |
| 96 | 87 | 37 | |
| 97 | 91 | 53 | |
| 98 | 100 | 61 | |
| 99 | 97 | 54 | |

Table V continued

| LTB$_4$ Binding Inhibition | | | |
|---|---|---|---|
| | Drug Concentration* | | |
| Example No. | $10^{-5}$M | $10^{-6}$M | $10^{-7}$M |
| 100 | 84 | 28 | |
| 101 | 101 | 82 | 45 |
| 102 | 97 | 57 | |
| 103 | 97 | 59 | |
| 104 | 32 | 5 | |
| 105 | 76 | 35 | |
| 106 | 9 | 1 | |
| 107 | 10 | 6 | |
| 108 | 105 | 96 | 60 |
| 109 | 41 | 3 | |
| 110 | 49 | 19 | |
| 114 | 52 | 8 | |
| 115 | 12–16 | 7–8 | |
| 118 | 83 | 38 | |
| 119 | 57 | 13 | |
| 120 | 97 | 55 | |
| 121 | 27–38 | 34–38 | |
| 122 | 66 | 24 | |
| 123 | 98 | 60 | |
| 125 | 10 | 6 | |
| 128 | 97 | 64 | |
| 129 | 47 | 7 | |

Table V continued

| LTB4 Binding Inhibition | | | |
|---|---|---|---|
| | Drug Concentration* | | |
| Example No. | $10^{-5}$M | $10^{-6}$M | $10^{-7}$M |
| 130 | 48 | 23 | |
| 132 | 0 | 0 | |
| 133 | 89 | 48 | |
| 134 | 93 | 45 | |
| 135 | 95 | 55 | |
| 136 | 100 | 77 | |
| 137 | 43 | 22 | |
| 138 | 98 | 58 | |
| 139 | 92 | 53 | |
| 140 | 94 | 59 | |
| 141 | 90 | 57 | |
| 142 | 34 | 5 | |
| 143 | 69 | 27 | |
| 144 | 63 | 20 | |
| 145 | 101 | 85 | |
| 146 | 81 | 29 | |
| 147 | 2 | 3 | |
| 148 | 44 | 14 | |

The compounds or formulations of the present invention may be administered by the oral and rectal routes, topically, parenterally, e.g., by injection and by continuous or discontinuous intra-arterial infusion, in the form of, for example, tablets, lozenges, sublingual tablets, sachets, cachets, elixirs, gels, suspensions, aerosols, ointments, for example, containing from 1 to 10% by weight of the active compound in a suitable base, soft and hard gelatin capsules, suppositories, injectable solutions and suspensions in physiologically acceptable media, and sterile packaged powders adsorbed onto a support material for making injectable solutions. Advantageously for this purpose, compositions may be provided in dosage unit form, preferably each dosage unit containing from about 5 to about 500 mg. (from about 5 to 50 mg. in the case of parenteral or inhalation administration, and from about 25 to 500 mg. in the case of oral or rectal administration) of a compound of Formula I. Dosages of from about 0.5 to about 300 mg./kg. per day, preferably 0.5 to 20 mg./kg., of active ingredient may be administered although it will, of course, readily be understood that the amount of the compound or compounds of Formula I actually to be administered will be determined by a physician, in the light of all the relevant circumstances including the condition to be treated, the choice of compound to be administered and the choice of route of administration and therefore the above preferred dosage range is not intended to limit the scope of the present invention in any way.

The formulations of the present invention normally will consist of at least one compound of Formula I mixed with a carrier, or diluted by a carrier, or enclosed or encapsulated by an ingestible carrier in the form of a capsule, sachet, cachet, paper or other container or by a disposable container such as an ampoule. A carrier or diluent may be a solid, semi-solid or liquid material which serves as a vehicle, excipient or medium for the active therapeutic substance.

Some examples of the diluents or carrier which may be employed in the pharmaceutical compositions of the present invention are lactose, dextrose, sucrose, sorbitol, mannitol, propylene glycol, liquid paraffin, white soft paraffin, kaolin, fumed silicon dioxide, microcrystalline cellulose, calcium silicate, silica, polyvinylpyrrolidone, cetostearyl alcohol, starch, modified starches, gum acacia, calcium phosphate, cocoa butter, ethoxylated esters, oil of theobroma, arachis oil, alginates, tragacanth, gelatin, syrup, methyl cellulose, polyoxyethylene sorbitan monolaurate, ethyl lactate, methyl and propyl hydroxybenzoate, sorbitan trioleate, sorbitan sesquioleate and oleyl alcohol and propellants such as trichloromonofluoromethane, dichlorodifluoromethane and dichlorotetrafluoroethane. In the case of tablets, a lubricant may be incorporated to prevent sticking and binding of the powdered ingredients in the dies and on the punch of the tableting machine. For such purpose there may be employed for instance aluminum, magnesium or calcium stearates, talc or mineral oil.

Preferred pharmaceutical forms of the present invention are capsules, tablets, suppositories, injectable solutions, creams and ointments. Especially preferred are formulations for inhalation application, such as an aerosol, topical formulations, and those for oral ingestion.

The following formulation examples may employ as active compounds any of the compounds of this invention. The examples are illustrative only and are not intended to limit the scope of the invention in any way.

Example 149

Hard gelatin capsules are prepared using the following ingredients:

| | Quantity (mg/capsule) |
|---|---|
| 3-[(4-Acetyl-2-ethyl-5-hydroxy-phenoxy)methyl]benzonitrile | 250 |
| Starch | 200 |
| Magnesium stearate | 10 |

The above ingredients are mixed and filled into hard gelatin capsules in 460 mg. quantities.

Example 150

A tablet is prepared using the ingredients below:

| | Quantity (mg/tablet) |
|---|---|
| 5-Ethyl-2-hydroxy-4-[6-methyl-6-(1H-tetrazol-5-yl)heptyloxy] acetophenone potassium salt | 250 |
| Cellulose, microcrystalline | 400 |
| Silicon dioxide, fumed | 10 |
| Magnesium stearate | 5 |

The components are blended and compressed to form tablets each weighing 665 mg.

Example 151

An aerosol solution is prepared containing the following components:

| | Weight % |
|---|---|
| 2-Hydroxy-4-[4-(2-methyl-2H-tetrazol-5-yl)butoxy]-5-ethyl-acetophenone | 0.25 |
| Ethanol | 30.00 |
| Propellant 11 (trichlorofluoromethane) | 10.25 |
| Propellant 12 (Dichlorodifluoromethane) | 29.75 |
| Propellant 114 (Dichlorotetrafluoroethane) | 29.75 |

The active compound is dissolved in the ethanol and the solution is added to the propellant 11, cooled to -30°C. and transferred to a filling device. The required amount is then fed to a container and further filled with the pre-mixed propellants 12 and 114 by means of the cold-filled method or pressure-filled method. The valve units are then fitted to the container.

Example 152

Tablets each containing 60 mg. of active ingredient are made up as follows:

| | |
|---|---|
| 7-(4-Acetyl-2-ethyl-5-hydroxyphenoxy)-2,2-dimethylheptanoic acid | 60 mg. |
| Starch | 45 mg. |
| Microcrystalline cellulose | 35 mg. |
| Polyvinylpyrrolidone (as 10% solution in water) | 4 mg. |
| Sodium carboxymethyl starch | 4.5 mg. |
| Magnesium stearate | 0.5 mg. |
| Talc | 1 mg. |
| Total | 150 mg. |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C. and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Example 153

Capsules each containing 80 mg. of medicament are made as follows:

| | |
|---|---|
| 2-Hydroxy-4-[6-(methylsulfonyl)-hexyloxy]-5-ethylacetophenone | 80 mg. |
| Starch | 59 mg. |
| Microcrystalline cellulose | 59 mg. |
| Magnesium stearate | 2 mg. |
| Total | 200 mg. |

The active ingredient, cellulose, starch and magnesium stearate are blended, passed through a No. 45 mesh U.S. sieve, and filled into hard gelatin capsules in 200 mg. quantities.

Example 154

Suppositories each containing 225 mg of active ingredient are made as follows:

| | |
|---|---|
| 5-[4-(4-Acetyl-2-ethyl-5-hydroxyphenoxy)butyl]-2H-tetrazole-2-acetic acid, sodium salt | 225 mg. |
| Unsaturated or saturated fatty acid glycerides to | 2000 mg. |

The active ingredient is passed through a No. 60 mesh U.S. sieve and suspended in the fatty acid glycerides previously melted using the minimum heat necessary. The mixture is then poured into a suppository mold of nominal 2 g. capacity and allowed to cool.

Example 155

Suspensions each containing 50 mg. of medicament per 5 ml. dose are made as follows:

| | |
|---|---|
| 5-(4-Acetyl-5-hydroxy-2-allylphenoxy)pentanenitrile | 50 mg |
| Sodium carboxymethyl cellulose | 50 mg |
| Sugar | 1 g |
| Methyl paraben | 0.05 mg |
| Propyl paraben | 0.03 mg |
| Flavor | q. v. |
| Color | q. v. |
| Purified water to | 5 ml |

The medicament is passed through a No. 45 mesh U.S. sieve and mixed with the sodium carboxymethylcellulose, sugar, and a portion of the water to form a suspension. The parabens, flavor and color are dissolved and diluted with some of the water and added, with stirring. Sufficient water is then added to produce the required volume.

**Claims for the Contracting States: BE, CH, DE, AT, FR, GB, IT, LI, LU, NL, SE**

1. A compound of the Formula I

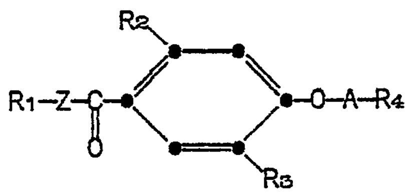

I

or a pharmaceutically acceptable salt thereof, wherein
$R_1$ is hydrogen or R'OOC–;
Z is $-(CH_2)_n-$ or phenylene;
n is 1–8;
$R_2$ is hydroxy, halo, or $-O-(CH_2)_m-Y$;
$R_3$ is $C_1$–$C_6$ alkyl, $C_1$–$C_6$ alkanoyl, $C_2$–$C_4$ alkenyl, $C_1$–$C_4$ alkoxy, hydroxy-substituted $C_1$–$C_3$ alkyl,or $-CH_2-D$;
A is a bond or straight or branched chain $C_1$–$C_{10}$ alkylidene;
$R_4$ is $C_1$–$C_6$ alkyl, $C_2$–$C_6$ alkenyl, $C_2$–$C_6$ alkynyl, hydroxy, –CN, halo, $-N_3$, $-NR_5R_6$, $-COR_7$, $-S(O)_p-(C_1-C_4$ alkyl), 1,2,4-triazol-l-yl, 5-tetrazoly l optionally substituted with a $C_1$–$C_4$ alkyl group or $-(CH_2)_qCOOR'$, or phenyl optionally substituted with one or two groups selected from halo, cyano, $C_1$–$C_3$ alkyl, trifluoromethyl, $-CH_2CN$, $-CH_2Br$, $C_1$–$C_4$ alkoxy, $-S(O)_p-(C_1-C_4$ alkyl), acetyl, –COOR', 5-tetrazolyl, or 5-tetrazolyl substituted with $C_1$–$C_4$ alkyl or $-(CH_2)_q-COOR'$;
where each R' is independently hydrogen or $C_1$-$C_4$ alkyl;
m is 1-4;
q is 1-4;
Y is hydrogen or -CN;
D is halo, $C_1$-$C_4$ alkoxy, or $-S-(C_1-C_4$ alkyl);
$R_5$ and $R_6$ are independently hydrogen, $C_1$-$C_3$ alkyl, or $C_2$-$C_4$ alkanoyl, or when taken together with the nitrogen atom to which they are attached form a morpholino ring;
$R_7$ is hydroxy, $C_1$-$C_4$ alkoxy, halo, $-NR_5R_6$, -NHOH,

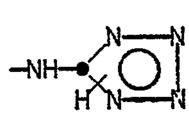 ,

or $C_1$-$C_3$ alkyl; and each p is 0, 1, or 2,
provided that when A is a bond, $R_4$ must be $C_1$-$C_6$ alkyl or an optionally substituted phenyl group, and further provided that when one of $R_5$ and $R_6$ is $C_2$-$C_4$ alkanoyl, the other of $R_5$ and $R_6$ is hydrogen.

2. A compound as claimed in claim 1 of the Formula Ia

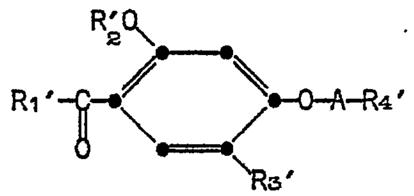

Ia

or a pharmaceutically acceptable salt thereof, wherein
$R_1'$ is methyl or ethyl;
$R_2'$ is hydrogen or methyl;
$R_3'$ is ethyl, propyl, $-CH_2-S-CH_3$, or allyl; and
$R_4'$ is hydroxy, cyano, -COOR', $-CONR_5R_6$, $-CO(C_1-C_3$ alkyl), $-S(O)_p-(C_1-C_4$ alkyl), 5-tetrazolyl optionally substituted with a $C_1-C_4$ alkyl group, or phenyl optionally substituted in the meta position with halo, cyano, $C_1-C_3$ alkyl, trifluoromethyl, $-CH_2CN$, $-CH_2Br$, $C_1-C_4$ alkoxy, $-S(O)_p-(C_1-C_4$ alkyl), acetyl, or -COOR'.

3. A compound of Formula Ia as claimed in claim 2, wherein the compound is 2-hydroxy-4-[4-(2-methyl-2H-tetrazol-5-yl)butoxy]-5-ethylacetophenone, 2-hydroxy-4-[6-(methylsulfonyl)hexyloxy]-5-ethylacetophenone, or 7-(4-acetyl-2-ethyl-5-hydroxyphenoxy)-2,2-dimethylheptanoic acid or a pharmaceutically acceptable salt thereof.

4. 3-[(4-Acetyl-2-ethyl-5-hydroxyphenoxy)methyl]benzonitrile.

5. 5-Ethyl-2-hydroxy-4-[6-methyl-6-(1H-tetrazol-5-yl)heptyloxy]acetophenone or a pharmaceutically acceptable salt thereof.

6. A pharmaceutical composition which comprises a compound as claimed in any one of the claims 1 to 5, or a pharmaceutically-acceptable salt thereof, associated with one or more pharmaceutically-acceptable carriers or excipients therefor.

7. A compound of Formula I, as claimed in any one of claims 1 to 5, or a pharmaceutically-acceptable salt thereof, for use in the treatment of inflammation.

8. A process for preparing a compound of Formula I as claimed in any one of claims 1 to 5 which comprises:

(a) acylating a compound of the formula

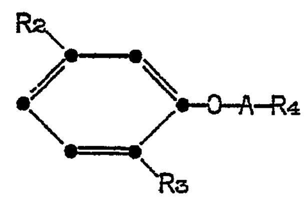

wherein $R_2$, $R_3$, $R_4$ and A are as defined for Formula I, under Friedel-Crafts conditions so as to produce a compound of the Formula I

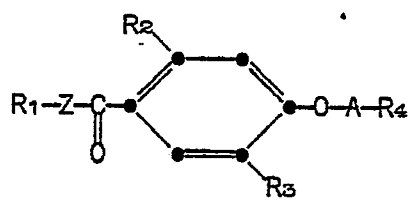

I

(b) alkylating a phenol of the formula

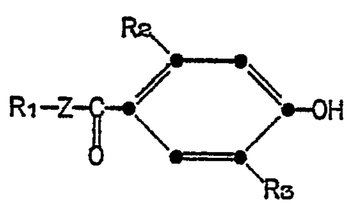

;

wherein $R_1$, $R_2$, $R_3$ and Z are as defined in claim 1, in the presence of a base strong enough to generate the anion of the phenol; or
(c) reacting a compound of claim 1 wherein $R_1$, Z, $R_2$, $R_3$, and A are as defined in claim 1 and $R_4$ is a group of the formula:

ii) 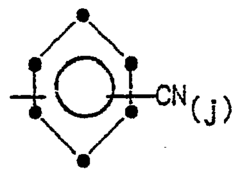 ;

with an azide reagent to give a compound of claim 1 wherein $R_4$ is a group of the formula

i) 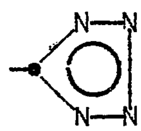

or

ii) 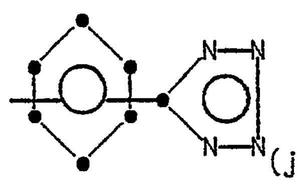 ;

d) hydrolyzing a compound of claim 1 wherein $R_4$ is a group of the formula:
i) -CN; or

ii) 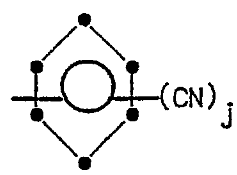 ;

or

iii) 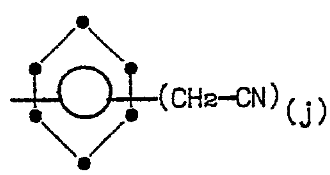 ;

to give a compound of claim 1 wherein $R_4$ is a group of the formula
i) -COOH; or

ii) 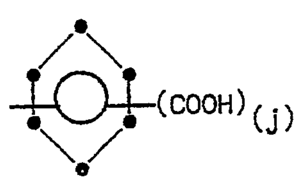

or

iii) 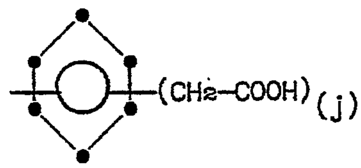

respectively;

e) hydrolyzing with aqueous base a compound of claim 1 wherein $R_1$, Z, $R_2$, $R_3$, and A are as defined in claim 1 and $R_4$ is a group of the formula:

i) -CN;

to give a compound of Formula I in claim 1 wherein $R_4$ is a primary amide group of the formula I in claim 1 wherein $R_4$ is a primary amide group of the formula

i) 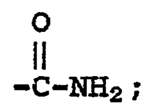

f) alkylating a compound of claim 1 wherein $R_1$, Z, $R_2$, $R_3$, and A are as defined for claim 1 and $R_4$ is a 5-tetrazolyl or a phenyl group substituted with one or two (5-tetrazolyl) groups; with an alkylating agent of the formula:

$X-(CH_2)_q-J$

wherein X is a good leaving group such as a halo group and J is a hydrogen atom or a group of the formula -COOR", wherein R" is a $C_1$ to $C_4$ alkyl group, in the presence of an acid scavenger, to produce a compound of claim 1 wherein $R_4$ is a group of the formula

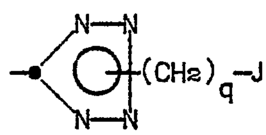 ;

or

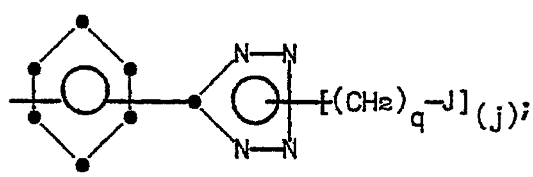

wherein q is the same as defined for claim 1, and j is one or two; org) oxidizing a compound of claim 1 wherein $R_1$, $R_2$, $R_3$, Z, and A are the same as claim 1 and $R_4$ is a group of the formula

i) $-S(O)_{p-1}-(C_1$ to $C_4$ alkyl); or

ii) 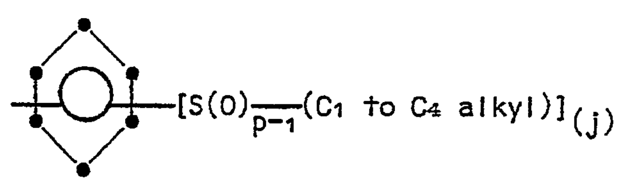 ;

to give a compound of claim 1 wherein $R_4$ is a group of the formula
i) $-S(O)_p-(C_1$ to $C_4$ alkyl); or

ii) 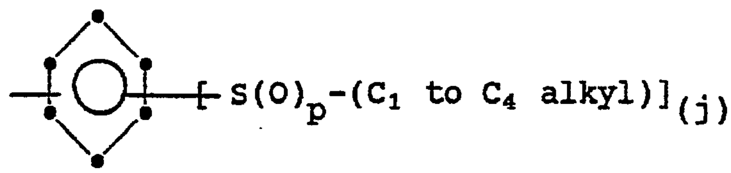

wherein j is one or two and p is one or two; or
h) reducing compounds of claim 1, wherein $R_1$, Z, $R_2$, $R_3$ and A are the same as claim 1 and $R_4$ is an azide group, to give the corresponding compounds of claim 1 wherein $R_4$ is a primary amino group;
i) acylating compounds of claim 1, wherein $R_1$, Z, $R_2$, $R_3$, and A are the same as for claim 1 and $R_4$ is a primary amino group or a primary amido group, with an acylating agent of the formula

$CH_3(CH_2)_nCO-LG$

wherein n is 1 to 3 and "LG" is chloro, bromo or iodo or a group forming an acid anhydride, in the presence of an acid scavenger, to give a compound of claim 1 wherein $R_4$ is a $C_2$ to $C_4$ alkanoylamino group or an N-($C_2$ to $C_4$ alkanoyl)amido group, respectively;
j) de-esterifying compound of claim 1 wherein $R_1$, Z, $R_2$, $R_3$, and A are the same as claim 1 and $R_4$ is a group of the formula

i) $-COR_7$, wherein $R_7$ is $C_1$ to $C_4$ alkoxy;

ii) 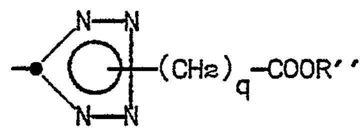

wherein q is the same as claim 1. and R" is $C_1$ to $C_4$ alkyl;

iii) 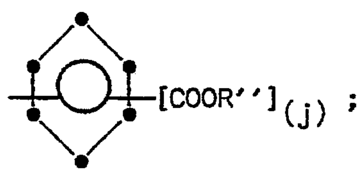

wherein R" is $C_1$ to $C_4$ alkyl and (j) is one or two; or

iv) 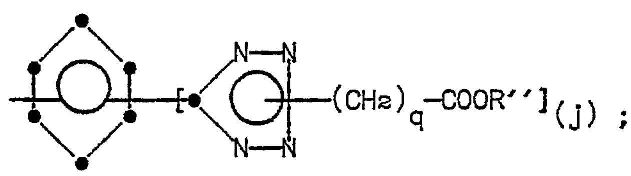

wherein (j) is one or two, q is as defined for claim 1 and R" is $C_1$ to $C_4$ alkyl;
to give compounds of claim 1 wherein $R_4$ is a group of the formula:

i) -COOH;

ii) 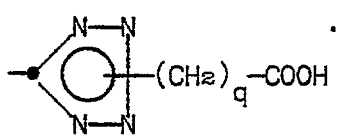

wherein q is from one to four;

iii) 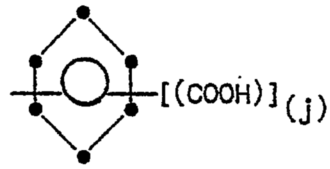 ;

wherein (j) is one or two; or

iv) 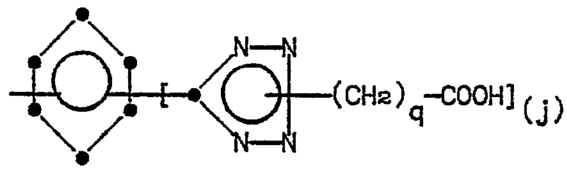

wherein q is one to four and (j) is one or two;

k) esterifying a compound of claim 1 wherein $R_1$, Z, $R_2$, $R_3$, and A are the same as claim 1 and $R_4$ is a group of the formula

i) -COOH;

ii) 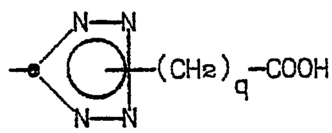 ;

wherein q is from one to four;

iii) 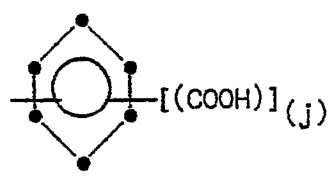 ;

wherein (j) is one or two; or

iv) 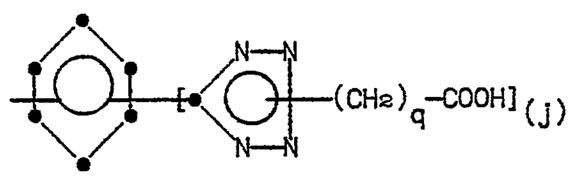

wherein q is one to four and (j) is one or two;

by reacting a compound of the formula $X-(CH_2)_d-CH_3$ wherein X is a good leaving group and d is one to three so as to form a compound of claim 1 wherein $R_4$ is a group of the formula:

i) $-COR_7$, wherein $R_7$ is $C_1$ to $C_4$ alkoxy;

ii) 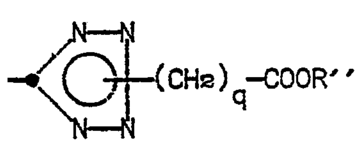

wherein q is the same as in claim 1 and R″ is $C_1$ to $C_4$ alkyl;

iii) 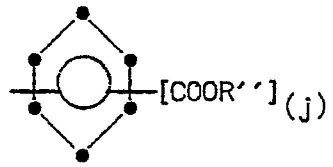 ;

wherein R″ is $C_1$ to $C_4$ alkyl and (j) is one or two; or

iv) 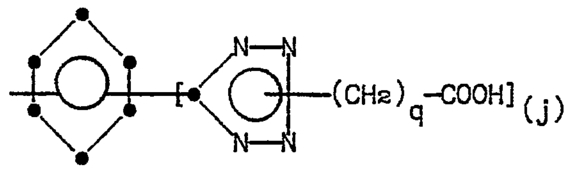

wherein (j) is one or two, q is as defined for claim 1 and R″ is $C_1$ to $C_4$ alkyl;

l) demethylating a compound of claim 1 wherein $R_1$, Z, $R_3$, A, and $R_4$ are as described for claim 1 and $R_2$ is a group of the formula

$-O-(CH_2)_m-Y$

wherein m is one and y is a hydrogen atom under standard demethylating conditions to give a compound of claim 1 wherein $R_2$ is a hydroxy group; or

m) reacting a compound of claim 1 wherein $R_1$, $R_2$, $R_3$, Z, and A are as defined for claim 1 and $R_4$ is a group of the formula $-COR_7$, wherein $R_7$ is a hydroxy group, with a halogenating reagent such as $POCl_3$, $POBr_3$, $PCl_5$, $PBr_5$, $SOCl_2$, $(\phi O)_3PCH_3I$, and the like to give a compound of claim 1 wherein $R_4$ is a group of the formula $-COR_7$ wherein $R_7$ is a halo group;

n) reacting a compound of claim 1, wherein $R_1$, Z, $R_2$, $R_3$, Z and A are as defined for claim 1 and wherein $R_4$ is halo, with:

i) an alkali metal cyanide to produce a compound of claim 1 wherein $R_4$ is cyano;

ii) a primary or secondary amine of the formula $HNR_5R_6$ to produce a compound of the claim 1 wherein $R_4$ is a group of the formula $-NR_5R_6$; or

iv) a thiolate anion or thiol of the formula

$T-S(O)_p-(C_1 \text{ to } C_4 \text{ alkyl})$

wherein T is a hydrogen atom or an alkali metal atom, to produce a compound of claim 1 wherein $R_4$ is a group of the formula

$-S(O)_p-(C_1 \text{ to } C_4 \text{ alkyl})$

wherein p is zero; or

o) reacting a compound of claim 1 wherein $R_1$, Z, $R_2$, $R_3$, and A, are the same as claim 1 and $R_4$ is a group of the formula $-COR_7$, wherein $R_7$ is a halo group, with:

i) an amine of the formula $HNR_5R_6$ wherein $R_5$ and $R_6$ are as defined for claim 1, to produce a compound wherein $R_7$ is $NR_5R_6$; or

ii) hydroxylamine to produce a compound of claim 1 wherein $R_7$ is a group of the formula -NHOH; or

iii) 5-aminotetrazole to produce a compound of claim 1 wherein $R_7$ is a 5-aminotetrazole bonded to the acyl group through the amino group nitrogen; or

iv) with a $C_1$ to $C_4$ alkanol or $C_1$ to $C_4$ alkoxide to produce a compound of claim 1 wherein $R_7$ is $C_1$ to $C_4$ alkoxy.

9. A process for producing the compound in claim 5, which comprises:

a) alkylating a diphenol of the formula:

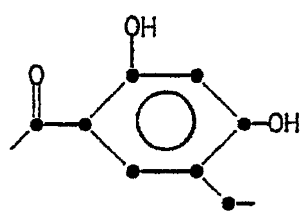 V

with 1,5-dibromopentane to produce a compound of the formula:

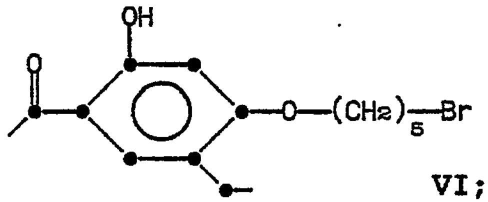

VI;

b) alkylating the compound of Formula VI with sodium 2-cyanopropan-2-ate to produce a cyano compound of the formula

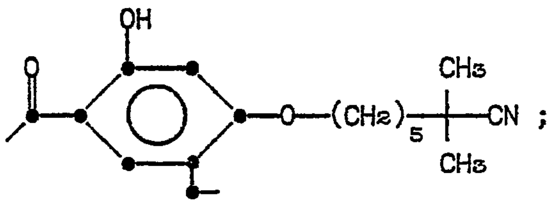

VII

c) reacting the cyano compound of Formula VII with an azide reagent such as tri(n-butyl)tin azide to give a tetrazole compound of the formula

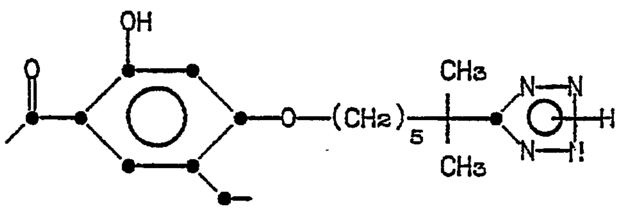

VIII

10. A compound of Formula I as claimed in claim 1 wherein $R_1$ is hydrogen, Z is a group of the formula $-(CH_2)_n-$ wherein n is 1, $R_2$ is hydroxy, A is a group of the formula:

$-(CH_2)_2-C(CH_3)_2-$

and $R_4$ is a cyano group.

**Claims for the Contracting States: GR, ES**

1. A process for preparing a compound of the Formula I

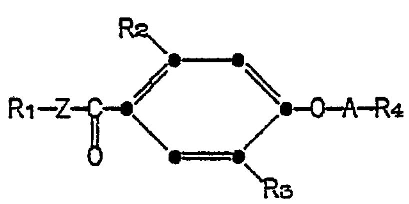

I

or a pharmaceutically acceptable salt thereof, wherein
$R_1$ is hydrogen or R'OOC–;
Z is $-(CH_2)_n-$ or phenylene;
n is 1–8;
$R_2$ is hydroxy, halo, or $-O-(CH_2)_m-Y$;
$R_3$ is $C_1$–$C_6$ alkyl, $C_1$–$C_6$ alkanoyl, $C_2$–$C_4$ alkenyl, $C_1$–$C_4$ alkoxy, hydroxy-substituted $C_1$–$C_3$ alkyl, or $-CH_2-D$;
A is a bond or straight or branched chain $C_1$–$C_{10}$ alkylidene;
$R_4$ is $C_1$–$C_6$ alkyl, $C_2$–$C_6$ alkenyl, $C_2$–$C_6$ alkynyl, hydroxy, –CN, halo, $-N_3$, $-NR_5R_6$, $-COR_7$, $-S(O)_p-$

($C_1$–$C_4$ alkyl), 1,2,4-triazol-l-yl, 5-tetrazolyl optionally substituted with a $C_1$–$C_4$ alkyl group or $-(CH_2)_qCOOR'$, or phenyl optionally substituted with one or two groups selected from halo, cyano, $C_1$–$C_3$ alkyl, trifluoromethyl, $-CH_2CN$, $-CH_2Br$, $C_1$–$C_4$ alkoxy, $-S(O)_p-$($C_1$–$C_4$ alkyl), acetyl, $-COOR'$, 5-tetrazolyl, or 5-tetrazolyl substituted with $C_1$–$C_4$ alkyl or $-(CH_2)_q-COOR'$;
where each R′ is independently hydrogen or $C_1$-$C_4$ alkyl;
m is 1-4;
q is 1-4;
Y is hydrogen or -CN;
D is halo, $C_1$-$C_4$ alkoxy, or -S-($C_1$-$C_4$ alkyl);
$R_5$ and $R_6$ are independently hydrogen, $C_1$-$C_3$ alkyl, or $C_2$-$C_4$ alkanoyl, or when taken together with the nitrogen atom to which they are attached form a morpholino ring;
$R_7$ is hydroxy, $C_1$-$C_4$ alkoxy, halo, $-NR_5R_6$, -NHOH,

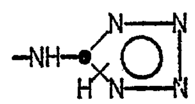 ,

or $C_1$-$C_3$ alkyl; and each p is 0, 1, or 2,
provided that when A is a bond, $R_4$ must be $C_1$-$C_6$ alkyl or an optionally substituted phenyl group, and further provided that when one of $R_5$ and $R_6$ is $C_2$-$C_4$ alkanoyl, the other of $R_5$ and $R_6$ is hydrogen.
which comprises:

(a) acylating a compound of the formula

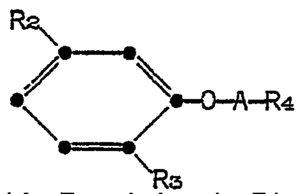

wherein $R_2$, $R_3$, $R_4$ and A are as defined for Formula I, under Friedel-Crafts conditions so as to produce a compound of the Formula I

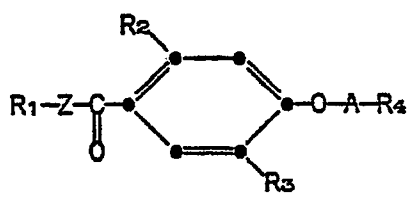

I

or;

(b) alkylating a phenol of the formula

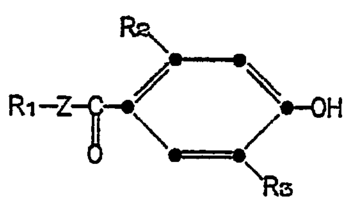

;

wherein $R_1$, $R_2$, $R_3$ and Z are as defined above, in the presence of a base strong enough to generate the anion of the phenol; or

(c) reacting a compound of Formula I wherein $R_1$, Z, $R_2$, $R_3$, and A are as defined above and $R_4$ is a group of the formula:

i) -CN; or

ii) 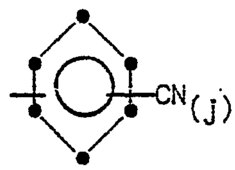 ;

with an azide reagent to give a compound of Formula I wherein $R_4$ is a group of the formula

i) 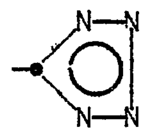

or

ii) 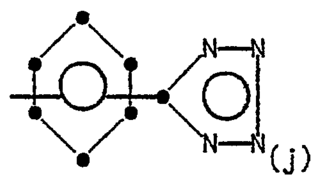 ;

d) hydrolyzing a compound of Formula I wherein $R_4$ is a group of the formula:

i) -CN; or

ii) 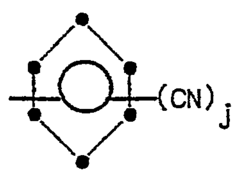 ;

or

iii) 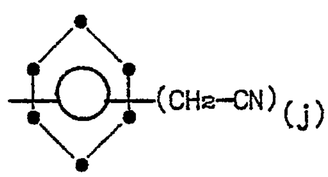 ;

to give a compound of Formula I wherein $R_4$ is a group of the formula

i) -COOH; or

ii) 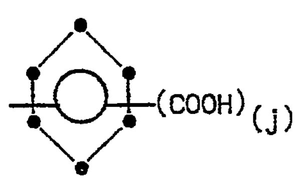

or

iii) 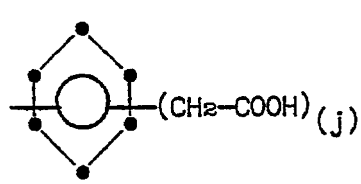

respectively;

e) hydrolyzing with aqueous base a compound of Formula I wherein $R_1$, Z, $R_2$, $R_3$, and A are as defined in Formula I and $R_4$ is a group of the formula:

i) -CN;

to give a compound of Formula I wherein $R_4$ is a primary amide group of the formula

i) 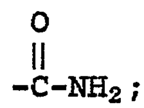

f) alkylating a compound of Formula I wherein $R_1$, Z, $R_2$, $R_3$, and A are as defined above and $R_4$ is a 5-tetrazolyl or a phenyl group substituted with one or two (5-tetrazolyl) groups; with an alkylating agent of the formula:

$X-(CH_2)_q-J$

wherein X is a good leaving group such as a halo group and J is a hydrogen atom or a group of the formula -COOR″, wherein R″ is a $C_1$ to $C_4$ alkyl group, in the presence of an acid scavenger, to produce a compound of Formula I wherein $R_4$ is a group of the formula

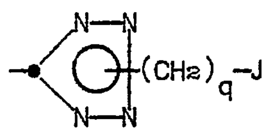 ;

or

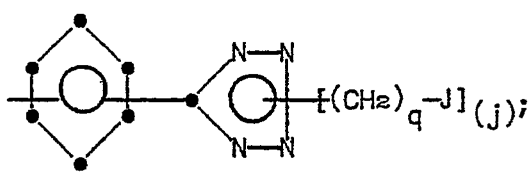

wherein q is the same as defined above, and j is one or two;
or
g) oxidizing a compound of Formula I wherein $R_1$, $R_2$, $R_3$, Z, and A are as defined above and $R_4$ is a group of the formula

i) $-S(O)_{p-1}-(C_1$ to $C_4$ alkyl); or

ii) 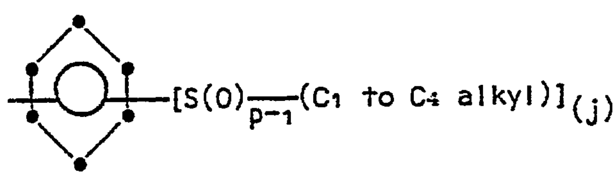 ;

to give a compound of Formula I wherein $R_4$ is a group of the formula

i) $-S(O)_p-(C_1$ to $C_4$ alkyl); or

ii) 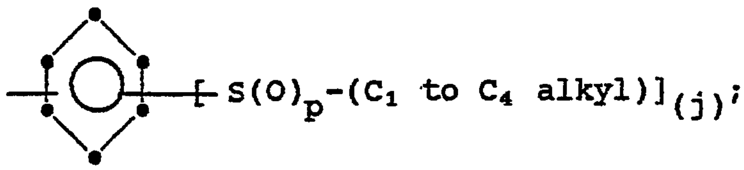

wherein j is one or two and p is one or two; or

h) reducing a compound of Formula I, wherein $R_1$, Z, $R_2$, $R_3$ and A are as defined above and $R_4$ is an azide group, to give the corresponding compounds wherein $R_4$ is a primary amino group;

i) acylating compounds of Formula I, wherein $R_1$, Z, $R_2$, $R_3$, and A are as defined above and $R_4$ is a primary amino group or a primary amido group, with an acylating agent of the formula

$CH_3(CH_2)_nCO\text{-}LG$

wherein n is 1 to 3 and "LG" is chloro, bromo or iodo or a group forming an acid anhydride, in the presence of an acid scavenger, to give a compound of Formula I wherein $R_4$ is a $C_2$ to $C_4$ alkanoylamino group or an N-($C_2$ to $C_4$ alkanoyl)amido group, respectively;

j) de-esterifying a compound of Formula I wherein $R_1$, Z, $R_2$, $R_3$, and A are as defined above and $R_4$ is a group of the formula

i) $-COR_7$, wherein $R_7$ is $C_1$ to $C_4$ alkoxy;

ii) 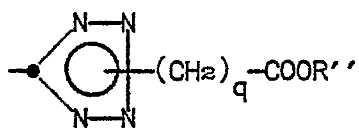

wherein q is as defined above and R″ is $C_1$ to $C_4$ alkyl;

iii) 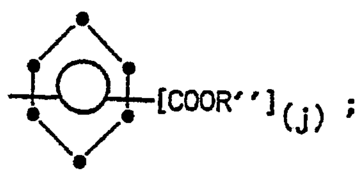

wherein R″ is $C_1$ to $C_4$ alkyl and (j) is one or two; or

iv) 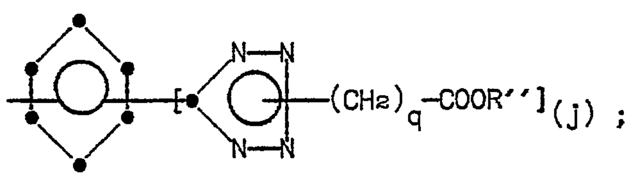

wherein (j) is one or two, q is as defined above and R″ is $C_1$ to $C_4$ alkyl;

to give compounds of Formula I wherein $R_4$ is a group of the formula:

i) -COOH;

ii) 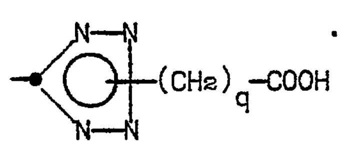

wherein q is from one to four;

iii) 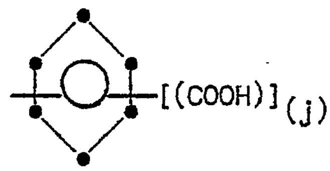 ;

wherein (j) is one or two; or

iv) 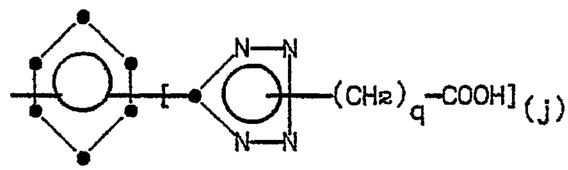

wherein q is one to four and (j) is one or two;
k) esterifying a compound of Formula I wherein $R_1$, Z, $R_2$, $R_3$, and A are as defined above and $R_4$ is a group of the formula

i) -COOH;

ii) 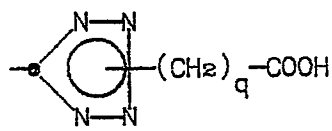 ;

wherein q is from one to four;

iii) 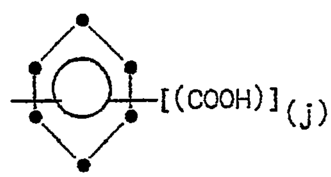 ;

wherein (j) is one or two; or

iv) 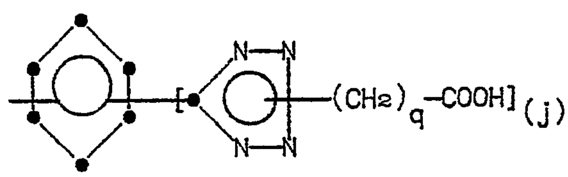

wherein q is one to four and (j) is one or two;
by reacting a compound of the formula $X-(CH_2)_d-CH_3$ wherein X is a good leaving group and d is one to three so as to form a compound of Formula I wherein $R_4$ is a group of the formula:
i) $-COR_7$, wherein $R_7$ is $C_1$ to $C_4$ alkoxy;

ii) 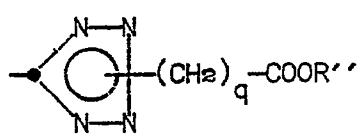

wherein q is as defined above and R″ is $C_1$ to $C_4$ alkyl;

iii) 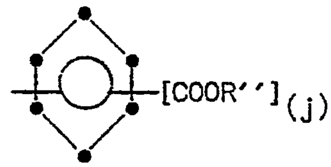 ;

wherein R″ is $C_1$ to $C_4$ alkyl and (j) is one or two; or

iv) 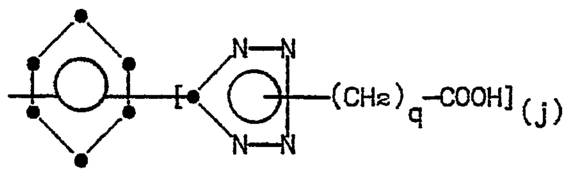

wherein (j) is one or two, q is as defined above and R″ is $C_1$ to $C_4$ alkyl;

l) demethylating a compound of Formula I wherein $R_1$, Z, $R_3$, A, and $R_4$ are as defined above and $R_2$ is a group of the formula

$-O-(CH_2)_m-Y$

wherein m is one and y is a hydrogen atom under standard demethylating conditions to give a compound of Formula I wherein $R_2$ is a hydroxy group; or

m) reacting a compound of Formula I wherein $R_1$, $R_2$, $R_3$, Z, and A are as defined above and $R_4$ is a group of the formula $-COR_7$, wherein $R_7$ is a hydroxy group, with a halogenating reagent such as $POCl_3$, $POBr_3$, $PCl_5$, $PBr_5$, $SOCl_2$, $(\phi O)_3PCH_3I$, and the like to give a compound of Formula I wherein $R_4$ is a group of the formula $-COR_7$ wherein $R_7$ is a halo group;

n) reacting a compound of Formula I, wherein $R_1$, Z, $R_2$, $R_3$, Z and A are as defined above and wherein $R_4$ is halo, with:

i) an alkali metal cyanide to produce a compound of Formula I wherein $R_4$ is cyano;

ii) a primary or secondary amine of the formula $HNR_5R_6$ to produce a compound of the Formula I wherein $R_4$ is a group of the formula $-NR_5R_6$; or

iv) a thiolate anion or thiol of the formula

$T-S(O)_p-(C_1$ to $C_4$ alkyl)

wherein T is a hydrogen atom or an alkali metal atom, to produce a compound of Formula I wherein $R_4$ is a group of the formula

$-S(O)_p-(C_1$ to $C_4$ alkyl)

wherein p is zero; or

o) reacting a compound of Formula I wherein $R_1$, Z, $R_2$, $R_3$, and A, are as defined above and $R_4$ is a group of the formula $-COR_7$, wherein $R_7$ is a halo group, with:

i) an amine of the formula $HNR_5R_6$ wherein $R_5$ and $R_6$ are as defined above, to produce a compound wherein $R_7$ is $NR_5R_6$; or

ii) hydroxylamine to produce a compound of Formula I wherein $R_7$ is a group of the formula -NHOH; or

iii) 5-aminotetrazole to produce a compound of Formula I wherein $R_7$ is a 5-aminotetrazole bonded to the acyl group through the amino group nitrogen; or

iv) with a $C_1$ to $C_4$ alkanol or $C_1$ to $C_4$ alkoxide to produce a compound of Formula I wherein $R_7$ is $C_1$ to $C_4$ alkoxy.

2. A process for preparing a compound as claimed in claim 1 of the Formula Ia

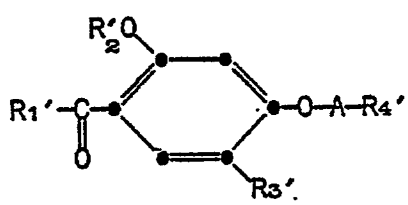

Ia

or a pharmaceutically acceptable salt thereof, wherein
$R_1'$ is methyl or ethyl;
$R_2'$ is hydrogen or methyl;
$R_3'$ is ethyl, propyl, $-CH_2-S-CH_3$, or allyl; and
$R_4'$ is hydroxy, cyano, -COOR', $-CONR_5R_6$, $-CO(C_1-C_3$ alkyl), $-S(O)_p-(C_1-C_4$ alkyl), 5-tetrazolyl optionally substituted with a $C_1-C_4$ alkyl group, or phenyl optionally substituted in the meta position with halo, cyano, $C_1-C_3$ alkyl, trifluoromethyl, $-CH_2CN$, $-CH_2Br$, $C_1-C_4$ alkoxy, $-S(O)_p-(C_1-C_4$ alkyl), acetyl, or -COOR'.

3. A process for preparing a compound of Formula Ia as claimed in claim 2, wherein the compound is 2-hydroxy-4-[4-(2-methyl-2H-tetrazol-5-yl)butoxy]-5-ethylacetophenone, 2-hydroxy-4-[6-(methylsulfonyl)hexyloxy]-5-ethylacetophenone, or 7-(4-acetyl-2-ethyl-5-hydroxyphenoxy)-2,2-dimethylheptanoic acid or a pharmaceutically acceptable salt thereof.

4. A process for preparing 3-[(4-acetyl-2-ethyl-5-hydroxyphenoxy)methyl]benzonitrile.

5. A process for preparing 5-ethyl-2-hydroxy-4-[6-methyl-6-(1H-tetrazol-5-yl)heptyloxy]acetophenone or a pharmaceutically acceptable salt thereof.

6. A process for producing the compound in claim 5, which comprises:

a) alkylating a diphenol of the formula:

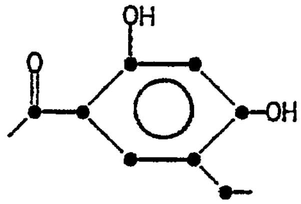

V

with 1,5-dibromopentane to produce a compound of the formula:

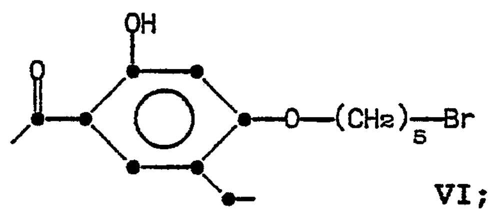

VI;

b) alkylating the compound of Formula VI with sodium 2-cyanopropan-2-ate to produce a cyano compound of the formula

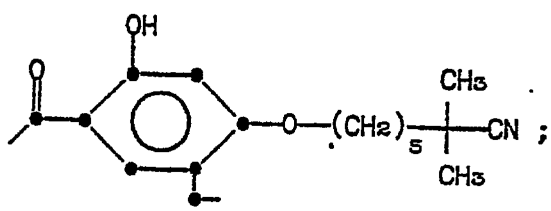

VII

c) reacting the cyano compound of Formula VII with an azide reagent such as tri(n-butyl)tin azide to give a tetrazole compound of the formula

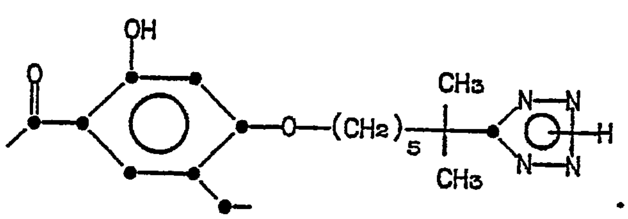

VIII

7. A process for preparing a pharmaceutical formulation which comprises admixing a compound of Formula I, or a pharmaceutically-acceptable salt thereof, as defined in any one of claims 1 to 5, with one or more pharmaceutically acceptable carriers or excipients therefor.

**Patentansprüche für die Vertragsstaaten: BE, CH, DE, AT, FR, GB, IT, LI, LU, NL, SE**

1. Verbindung der Formel (I)

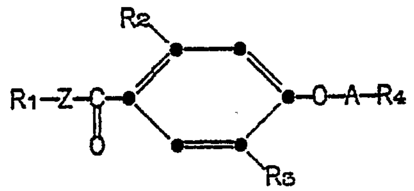

I

oder ein pharmazeutisch annehmbares Salz hiervon, worin
$R_1$ Wasserstoff oder R'OOC– ist,
Z für $-(CH_2)_n-$ oder Phenylen steht,
n eine Zahl von 1 bis 8 bedeutet,
$R_2$ Hydroxy, Halogen oder $-O-(CH_2)_m-Y$ darstellt,
$R_3$ für $C_1$–$C_6$-Alkyl, $C_1$–$C_6$-Alkanoyl, $C_2$–$C_4$-Alkenyl, $C_1$–$C_4$-Alkoxy, hydroxysubstituiertes $C_1$–$C_3$-Alkyl der $-CH_2-D$ steht,
A eine Bindung oder eine gerade oder verzweigte $C_1$–$C_{10}$-Alkylidenkette ist,
$R_4$ für $C_1$–$C_6$-Alkyl, $C_2$–$C_6$-Alkenyl, $C_2$–$C_6$-Alkinyl, 1,2,4-Triazol-1-yl, Hydroxy, –CN, Halogen, $-N_3$, $-NR_5R_6$, $-COR_7$, $-S(O)_p-(C_1-C_4\text{-Alkyl})$, 5-Tetrazolyl, das gegebenenfalls durch eine Gruppe $C_1$–$C_4$-Alkyl oder $-(CH_2)_q-COOR'$ substituiert ist, oder für Phenyl steht, das gegebenenfalls durch ein oder zwei Gruppen substituiert ist, die ausgewählt sind aus Halogen, Cyano, $C_1$–$C_3$-Alkyl, Trifluormethyl, $-CH_2CN$, $-CH_2Br$, $C_1$–$C_4$-Alkoxy, $-S(O)_p-(C_1-C_4\text{-Alkyl})$, Acetyl, –COOR', 5-Tetrazolyl oder 5-Tetrazolyl, welches durch $C_1$–$C_4$-Alkyl oder $-(CH_2)_q-COOR'$ substituiert ist,
wobei jeder der Reste R' unabhängig Wasserstoff oder $C_1$–$C_4$-Alkyl bedeutet,
m eine Zahl von 1 bis 4 ist,
q für 1 bis 4 steht,
Y Wasserstoff oder –CN darstellt,
D Halogen, $C_1$–$C_4$-Alkoxy oder $-S-(C_1-C_4\text{-Alkyl})$ ist,
$R_5$ und $R_6$ unabhängig Wasserstoff, $C_1$–$C_3$-Alkyl oder $C_2$–$C_4$-Alkanoyl sind oder zusammen mit dem Stickstoffatom, an das sie gebunden sind, einen Morpholinring bilden,
$R_7$ Hydroxy, $C_1$–$C_4$-Alkoxy, Halogen, $-NR_5R_6$, –NHOH,

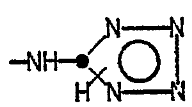 ,

oder $C_1$–$C_3$-Alkyl bedeutet und p jeweils für 0, 1 oder 2 steht,
mit der Maßgabe, daß falls A eine Bindung ist, der Substituent $R_4$ dann $C_1$–$C_6$-Alkyl oder gegebenenfalls substituiertes Phenyl sein muß, und mit der weiteren Maßgabe, daß falls einer der Substituenten $R_5$ und $R_6$ für $C_2$–$C_4$-Alkanoyl steht, der andere der Substituenten $R_5$ und $R_6$ dann Wasserstoff ist.

2. Verbindung nach Anspruch 1 der Formel (Ia)

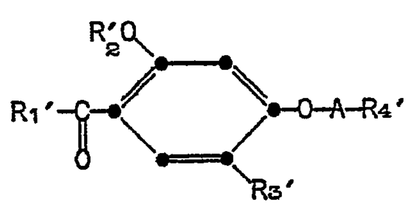

Ia

oder ein pharmazeutisch annehmbares Salz hiervon, worin
$R_1'$ Methyl oder Ethyl ist,
$R_2'$ Wasserstoff oder Methyl bedeutet,
$R_3'$ für Ethyl, Propyl, $-CH_2-S-CH_3$ oder Allyl steht und
$R_4'$ für Hydroxy, Cyano, –COOR', $-CONR_5R_6$, $-CO(C_1-C_3\text{-Alkyl})$, $-S(O)_p-(C_1-C_4\text{-Alkyl})$, 5-Tetrazolyl, das gegebenenfalls durch eine $C_1$–$C_4$-Alkylgruppe substituiert ist, oder für Phenyl steht, das gegebenenfalls in meta-Stellung durch Halogen, Cyano, $C_1$–$C_3$-Alkyl, Trifluormethyl, $-CH_2CN$, $-CH_2Br$, $C_1$–$C_4$-Alkoxy, $-S(O)_p-(C_1-C_4\text{-Alkyl})$, Acetyl oder –COOR' substituiert ist.

3. Verbindung der Formel (Ia) nach Anspruch 2, dadurch gekennzeichnet, daß die Verbindung 2-Hydroxy-4-[4-(2-methyl-2H-tetrazol-5-yl)butoxy]-5-ethylacetophenon, 2-Hydroxy-4-[6-(methylsulfonyl)hexyloxy]-5-ethylacetophenon oder 7-(4-Acetyl-2-ethyl-5-hydroxyphenoxy)-2,2-dimethylheptancarbonsäure oder ein pharmazeutisch annehmbares Salz hiervon ist.

4. 3-[(4-Acetyl-2-ethyl-5-hydroxyphenoxy)methyl]benzonitril.

5. 5-Ethyl-2-hydroxy-4-[6-methyl-6-(1H-tetrazol-5-yl)heptyloxy]acetophenon oder ein pharmazeutisch annehmbares Salz hiervon.

6. Pharmazeutische Zusammensetzung, dadurch gekennzeichnet, daß sie eine Verbindung nach einem der Ansprüche 1 bis 5 oder ein pharmazeutisch annehmbares Salz hiervon als Wirkstoff in Verbindung mit einem oder mehreren pharmazeutisch annehmbaren Trägern oder Hilfsstoffen hierfür enthält.

7. Verbindung der Formel (I) nach einem der Ansprüche 1 bis 5 oder ein pharmazeutisch annehmbares Salz hiervon zur Verwendung bei der Behandlung einer Inflammation.

8. Verfahren zur Herstellung einer Verbindung der Formel (I) nach einem der Ansprüche 1 bis 5, dadurch gekennzeichnet, daß

(a) eine Verbindung der Formel

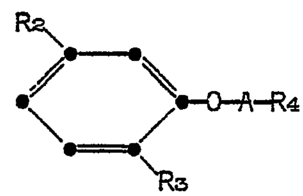

worin $R_2$, $R_3$, $R_4$ und A wie bei der Formel (I) definiert sind, unter Friedel-Crafts-Bedingungen zu einer Verbindung der Formel (I)

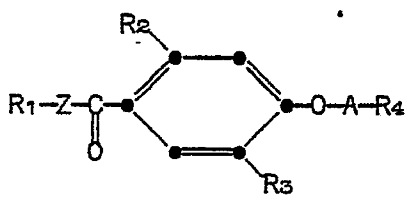

I

umgesetzt wird, oder

(b) ein Phenol der Formel

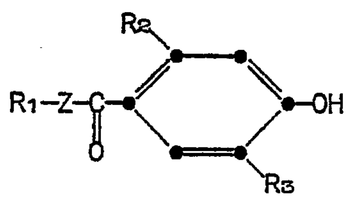

worin $R_1$, $R_2$, $R_3$ und Z wie im Anspruch 1 definiert sind, in Anwesenheit einer zur Bildung des Anions des Phenols ausreichend starken Base alkyliert wird, oder

(c) eine Verbindung nach Anspruch 1, worin $R_1$, Z, $R_2$, $R_3$ und A wie im Anspruch 1 definiert sind und $R_4$ eine Gruppe der Formeln

(i) –CN oder

ii) 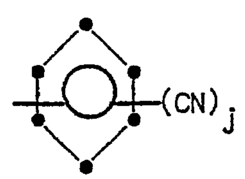

bedeutet, mit einem Azidreagens zu einer Verbindung nach Anspruch 1 umgesetzt wird, worin $R_4$ eine Gruppe der Formeln

i) 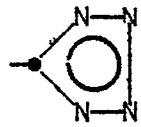

oder

ii) 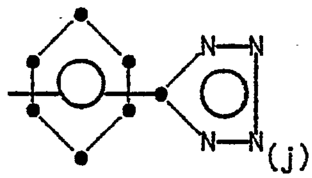

ist, oder

(d) eine Verbindung nach Anspruch 1, worin $R_4$ eine Gruppe der Formeln

(i) –CN oder

(ii) 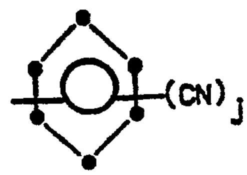

oder

iii) 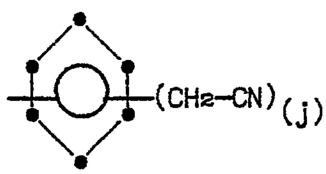

bedeutet, zu einer Verbindung nach Anspruch 1 hydrolysiert wird, worin $R_4$ eine Gruppe der Formeln

(i) –COOH oder

ii) 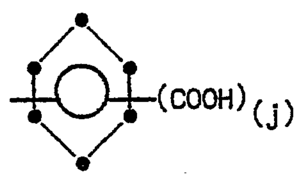

oder

iii) 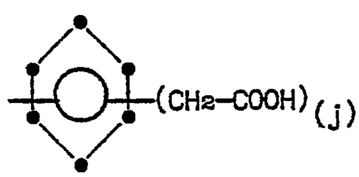

ist, oder

(e) eine Verbindung nach Anspruch 1, worin $R_1$, Z, $R_2$, $R_3$ und A wie im Anspruch 1 definiert sind und $R_4$ eine Gruppe der Formel

(i) –CN

bedeutet, mit einer wäßrigen Base zu einer Verbindung der Formel (I) nach Anspruch 1 hydrolysiert wird, worin $R_4$ eine primäre Amidgruppe der Formel

i) 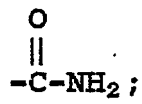

ist, oder

(f) eine Verbindung nach Anspruch 1, worin $R_1$, Z, $R_2$, $R_3$ und A wie im Anspruch 1 definiert sind und $R_4$ für eine 5-Tetrazolylgruppe oder eine Phenylgruppe steht, die mit ein oder zwei (5-Tetrazolyl)-Gruppen substituiert ist, durch Umsetzung mit einem Alkylierungsmittel der Formel

$X-(CH_2)_q-J$

worin X eine gute Abgangsgruppe ist, wie eine Halogengruppe, und J ein Wasserstoffatom oder eine Gruppe der Formel $-COOR''$ bedeutet, worin R″ für eine $C_1-C_4$-Alkylgruppe steht, in Anwesenheit eines Säurebindemittels in eine Verbindung nach Anspruch 1 überführen wird, worin $R_4$ eine Gruppe der Formeln

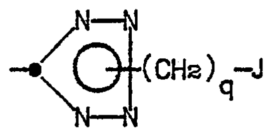

oder

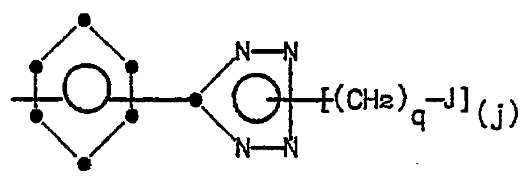

bedeutet, worin q wie im Anspruch 1 definiert ist und j für 1 oder 2 steht, oder

(g) eine Verbindung nach Anspruch 1, worin $R_1$, $R_2$, $R_3$, Z und A wie im Anspruch 1 definiert sind und $R_4$ eine Gruppe der Formeln

(i) $-S(O)_{p-1}(C_1-C_4\text{-Alkyl})$ oder

(ii) 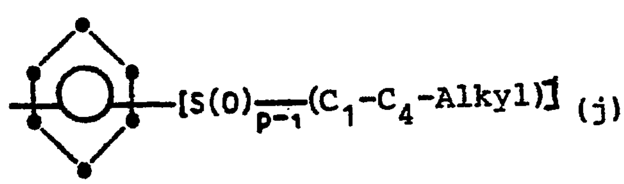

ist, zu einer Verbindung nach Anspruch 1 oxidiert wird, worin $R_4$ eine Gruppe der Formeln

(i) $-S(O)_p-(C_1-C_4\text{-Alkyl})$ oder

ii) 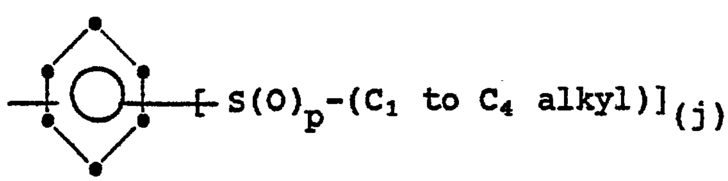

worin j für 1 oder 2 steht und p für 1 oder 2 steht, bedeutet, oder

(h) Verbindungen nach Anspruch 1, worin $R_1$, Z, $R_2$, $R_3$ und A wie im Anspruch 1 definiert sind und $R_4$ eine Azidgruppe ist, zu den entsprechenden Verbindungen nach Anspruch 1 reduziert werden, worin $R_4$ eine primäre Aminogruppe bedeutet, oder

(i) Verbindungen nach Anspruch 1, worin $R_1$, Z, $R_2$, $R_3$ und A wie im Anspruch 1 definiert sind und $R_4$ ei-

ne primäre Aminogruppe oder eine primäre Amidogruppe ist, durch Umsetzung mit einem Acylierungsmittel der Formel

$CH_3(CH_2)_nCO{-}LG$

worin n für 1 bis 3 steht und LG Chlor, Brom oder Iod oder eine ein Säureanhydrid bildende Gruppe bedeutet, in Gegenwart eines Säurebindemittels in eine Verbindung nach Anspruch 1 überführt werden, worin $R_4$ für eine $C_2$–$C_4$-Alkanoylaminogruppe oder eine N–($C_2$–$C_4$-Alkanoyl)amidogruppe steht, oder

(j) eine Verbindung nach Anspruch 1, worin $R_1$, Z, $R_2$, $R_3$ und A wie im Anspruch 1 definiert sind und $R_4$ eine Gruppe der folgenden Formeln bedeutet

(i) $-COR_7$,

worin $R_7$ für $C_1$–$C_4$-Alkoxy steht,

ii) 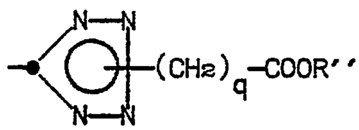

worin q wie im Anspruch 1 definiert ist und R″ für $C_1$–$C_4$-Alkyl steht,

iii) 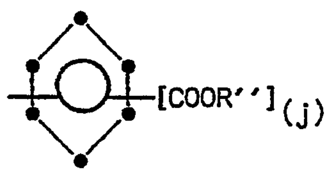 ;

worin R″ für $C_1$–$C_4$-Alkyl steht und (j) für 1 oder 2 steht, oder

iv) 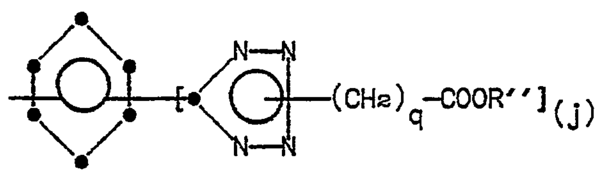

worin (j) für 1 oder 2 steht, q wie im Anspruch 1 definiert ist und R″ für $C_1$–$C_4$-Alkyl steht,
durch Entesterung in Verbindung nach Anspruch 1 überführt wird, worin $R_4$ eine Gruppe der folgenden Formeln bedeutet

(i) $-COOH$

ii) 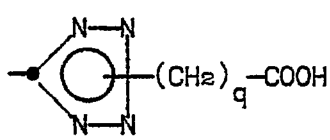

worin q für 1 bis 4 steht,

iii) 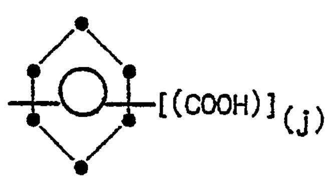

worin (j) für 1 oder 2 steht, oder

iv) 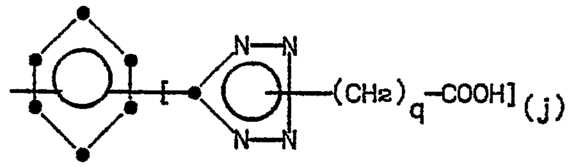

worin q für 1 bis 4 steht und (j) für 1 oder 2 steht, oder
(k) eine Verbindung nach Anspruch 1, worin $R_1$, Z, $R_2$, $R_3$ und A wie im Anspruch 1 definiert sind und $R_4$ eine Gruppe der folgenden Formeln bedeutet

(i) –COOH

ii) 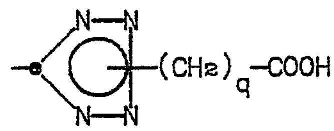 ;

worin q für 1 bis 4 steht,

iii) 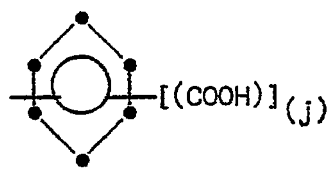 ;

worin (j) für 1 oder 2 steht, oder

iv) 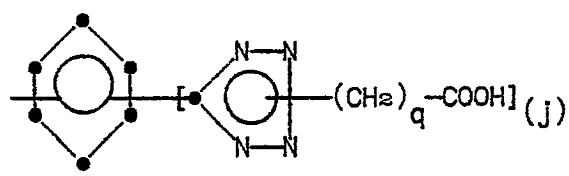

worin q für 1 bis 4 steht und (j) für 1 oder 2 steht,
durch Umsetzung mit einer Verbindung der Formel $X-(CH_2)_d-CH_3$, worin X eine gute Abgangsgruppe ist und d für 1 bis 3 steht, unter Entesterung in eine Verbindung nach Anspruch 1 überführt wird, worin $R_4$ eine Gruppe der folgenden Formeln bedeutet

(i) $-COR_7$, worin $R_7$ für $C_1$–$C_4$-Alkoxy steht,

ii) 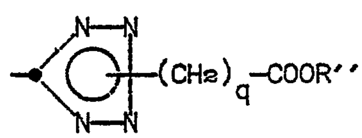

worin q wie im Anspruch 1 definiert ist und R″ für $C_1$–$C_4$-Alkyl steht,

iii) 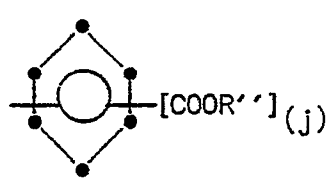 ;

worin R″ für $C_1$–$C_4$-Alkyl steht und (j) für 1 oder 2 steht, oder

iv) 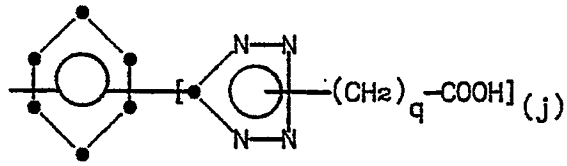

worin (j) für 1 oder 2 steht, q wie im Anspruch 1 definiert ist und R″ für $C_1$–$C_4$-Alkyl steht,

(l) eine Verbindung nach Anspruch 1, worin $R_1$, Z, $R_3$, A und $R_4$ wie im Anspruch 1 definiert sind und $R_2$ eine Gruppe der Formel

$-O-(CH_2)_m-Y$

bedeutet, worin m für 1 steht und Y ein Wasserstoffatom ist, unter üblichen Demethylierungsbedingungen in eine Verbindung nach Anspruch 1 überführt wird, worin $R_2$ eine Hydroxygruppe ist, oder

(m) eine Verbindung nach Anspruch 1, worin $R_1$, $R_2$, $R_3$, Z und A wie im Anspruch 1 definiert sind und $R_4$ eine Gruppe der Formel $-COR_7$ ist, worin $R_7$ eine Hydroxygruppe bedeutet, durch Umsetzung mit einem Halogenierungsmittel, wie $POCl_3$, $POBr_3$, $PCl_5$, $PBr_5$, $SOCl_2$, $(ØO)_3PCH_3I$ und dergleichen, in eine Verbindung nach Anspruch 1 überführt wird, worin $R_4$ eine Gruppe der Formel $-COR_7$ ist, worin $R_7$ eine Halogengruppe bedeutet, oder

(n) eine Verbindung nach Anspruch 1, worin $R_1$, $R_2$, $R_3$, Z und A wie im Anspruch 1 definiert sind und $R_4$ Halogen ist, umgesetzt wird mit

(i) einem Alkalimetallcyanid unter Bildung einer Verbindung nach Anspruch 1, worin $R_4$ Cyano ist, oder

(ii) einem primäre oder sekundären Amin der Formel $HNR_5R_6$ unter Bildung einer Verbindung nach Anspruch 1, worin $R_4$ eine Gruppe der Formel $-NR_5R_6$ bedeutet, oder

(iv) einem Thiolatanion oder einem Thiol der Formel

$T-S(O)_p-(C_1-C_4\text{-Alkyl})$

worin T ein Wasserstoffatom oder ein Alkalimetallatom darstellt, unter Bildung einer Verbindung nach Anspruch 1, worin $R_4$ eine Gruppe der Formel

$-S(O)_p-(C_1-C_4\text{-Alkyl})$

bedeutet, worin p für 0 steht, oder

(o) eine Verbindung nach Anspruch 1, worin $R_1$, Z, $R_2$, $R_3$ und A wie im Anspruch 1 definiert sind und $R_4$ eine Gruppe der Formel $-COR_7$ darstellt, worin $R_7$ eine Halogengruppe ist, umgesetzt wird mit

(i) einem Amin der Formel $HNR_5R_6$, worin $R_5$ und $R_6$ wie im Anspruch 1 definiert sind, unter Bildung einer Verbindung, worin $R_7$ für $NR_5R_6$, worin $R_5$ und $R_6$ wie im Anspruch 1 definiert sind, unter Bildung einer Verbindung, worin $R_7$ für $NR_5R_6$ steht, oder

(ii) einem Hydroxylamin unter Bildung einer Verbindung nach Anspruch 1, worin $R_7$ eine Gruppe der Formel $-NHOH$ ist, oder

(iii) ein 5-Aminotetrazol unter Bildung einer Verbindung nach Anspruch 1, worin $R_7$ für ein 5-Aminotetrazol steht, das über das Stickstoffatom der Aminogruppe an die Acylgruppe gebunden ist, oder

(iv) einem $C_1$–$C_4$-Alkanol oder einem $C_1$–$C_4$-Alkoxid unter Bildung einer Verbindung nach Anspruch 1, worin $R_7$ für ein $C_1$–$C_4$-Alkoxy steht.

9. Verfahren zur Herstellung einer Verbindung nach Anspruch 5, dadurch gekennzeichnet, daß

(a) ein Diphenol der Formel (V)

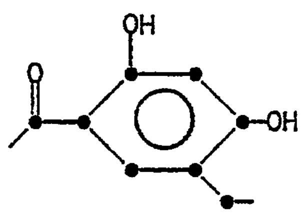

V

mit 1,5-Dibrompentan alkyliert und so eine Verbindung der Formel (VI)

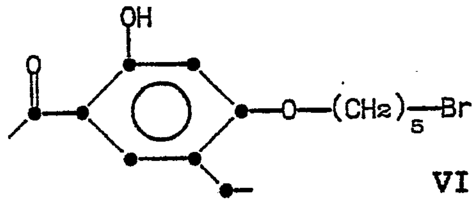

VI.

gebildet wird, oder
(b) die Verbindung der Formel (VI) mit Natrium-2-cyanopropan-2-at alkyliert und so eine Cyanoverbindung der Formel (VII)

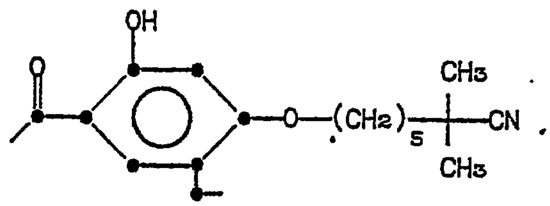

VII

gebildet wird, oder
(c) die Cyanoverbindung der Formel (VII) mit einem Azidreagens, wie Tri(n-butyl)zinnazid, umgesetzt und so eine Tetrazolverbindung der Formel (VIII)

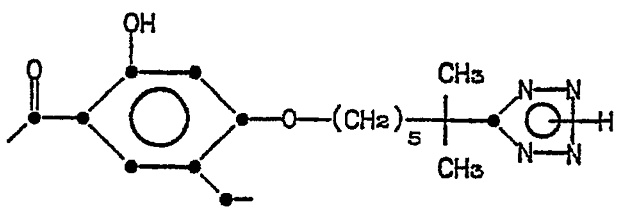

VIII

gebildet wird.

10. Verbindung der Formel (I) nach Anspruch 1, worin $R_1$ Wasserstoff ist, Z eine Gruppe der Formel $-(CH_2)_n-$ bedeutet, worin n für 1 steht, $R_2$ Hyroxy ist, A eine Gruppe der Formel

$-(CH_2)_2-C(CH_3)_2-$

darstellt und $R_4$ eine Cyanogruppe ist.

**Patentansprüche für die Vertragsstaaten: GR, ES**

1. Verfahren zur Herstellung einer Verbindung der Formel (I)

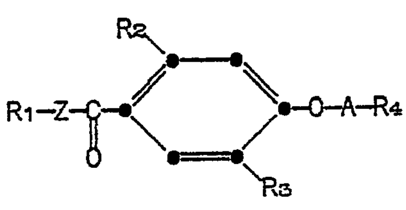

I

oder eines pharmazeutisch annehmbares Salz hiervon, worin
$R_1$ Wasserstoff oder R'OOC– ist,
Z für $-(CH_2)_n-$ oder Phenylen steht,
n eine Zahl von 1 bis 8 bedeutet,
$R_2$ Hydroxy, Halogen oder $-O-(CH_2)_m-Y$ darstellt,
$R_3$ für $C_1$–$C_6$-Alkyl, $C_1$–$C_6$-Alkanoyl, $C_2$–$C_4$-Alkenyl, $C_1$–$C_4$-Alkoxy, hydroxysubstituiertes $C_1$–$C_3$-Alkyl der $-CH_2-D$ steht,
A eine Bindung oder eine gerade oder verzweigte $C_1$–$C_{10}$-Alkylidenkette ist,

$R_4$ für $C_1$–$C_6$-Alkyl, $C_2$–$C_6$-Alkenyl, $C_2$–$C_6$-Alkinyl, 1,2,4-Triazol-1-yl, Hydroxy, –CN, Halogen, $-N_3$, $-NR_5$, $R_6$, $-COR_7$, $-S(O)_p-(C_1-C_4$-Alkyl), 5-Tetrazolyl, das gegebenenfalls durch eine Gruppe $C_1$–$C_4$-Alkyl oder $-(CH_2)_q-COOR'$ substituiert ist, oder für Phenyl steht, das gegebenenfalls durch ein oder zwei Gruppen substituiert ist, die ausgewählt sind aus Halogen, Cyano, $C_1$–$C_3$-Alkyl, Trifluormethyl, $-CH_2CN$, $-CH_2Br$, $C_1$–$C_4$-Alkoxy, $-S(O)_p-(C_1-C_4$-Alkyl), Acetyl, $-COOR'$, 5-Tetrazolyl oder 5-Tetrazolyl, welches durch $C_1$–$C_4$-Alkyl oder $-(CH_2)_q-COOR'$ substituiert ist,
wobei jeder der Reste R′ unabhängig Wasserstoff oder $C_1$–$C_4$-Alkyl bedeutet,
m eine Zahl von 1 bis 4 ist,
q für 1 bis 4 steht,
Y Wasserstoff oder –CN darstellt,
D Halogen, $C_1$–$C_4$-Alkoxy oder $-S-(C_1-C_4$-Alkyl) ist,
$R_5$ und $R_6$ unabhängig Wasserstoff, $C_1$–$C_3$-Alkyl oder $C_2$–$C_4$-Alkanoyl sind oder zusammen mit dem Stickstoffatom, an das sie gebunden sind, einen Morpholinring bilden,
$R_7$ Hydroxy, $C_1$–$C_4$-Alkoxy, Halogen, $-NR_5R_6$, –NHOH,

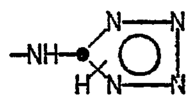

oder $C_1$–$C_3$-Alkyl bedeutet und p jeweils für 0, 1 oder 2 steht,
mit der Maßgabe, daß falls A eine Bindung ist, der Substituent $R_4$ dann $C_1$–$C_6$-Alkyl oder gegebenenfalls substituiertes Phenyl sein muß, und mit der weiteren Maßgabe, daß falls einer der Substituenten $R_5$ und $R_6$ für $C_2$–$C_4$-Alkanoyl steht, der andere der Substituenten $R_5$ und $R_6$ dann Wasserstoff ist,
dadurch gekennzeichnet, daß

(a) eine Verbindung der Formel

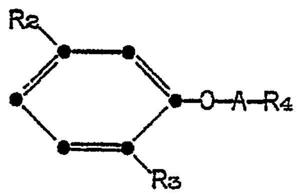

worin $R_2$, $R_3$, $R_4$ und A wie bei der Formel (I) definiert sind, unter Friedel-Crafts-Bedingungen zu einer Verbindung der Formel (I)

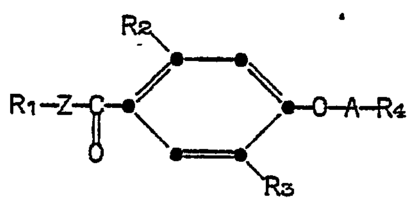 I

umgesetzt wird, oder

(b) ein Phenol der Formel

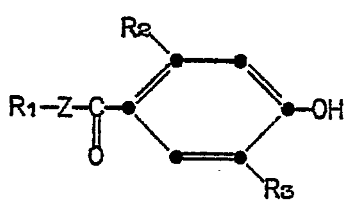 ;

worin $R_1$, $R_2$, $R_3$ und Z wie im Anspruch 1 definiert sind, in Anwesenheit einer zur Bildung des Anions des Phenols ausreichend starken Base alkyliert wird, oder

(c) eine Verbindung nach Anspruch 1, worin $R_1$, Z, $R_2$, $R_3$ und A wie im Anspruch 1 definiert sind und $R_4$ eine Gruppe der Formeln

(i) –CN oder

ii) 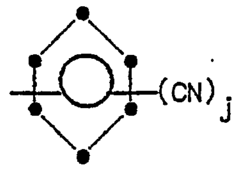 ;

bedeutet, mit einem Azidreagens zu einer Verbindung nach Anspruch 1 umgesetzt wird, worin $R_4$ eine Gruppe der Formeln

i) 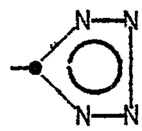

oder

ii) 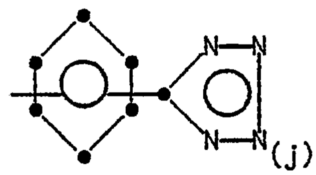 ;

ist, oder
(d) eine Verbindung nach Anspruch 1, worin $R_4$ eine Gruppe der Formeln

(i) –CN oder

(ii) 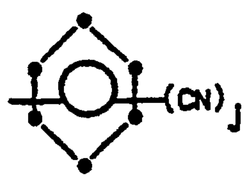

oder

iii) 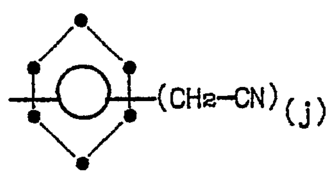 ;

bedeutet, zu einer Verbindung nach Anspruch 1 hydrolysiert wird, worin $R_4$ eine Gruppe der Formeln

(i) –COOH oder

ii) 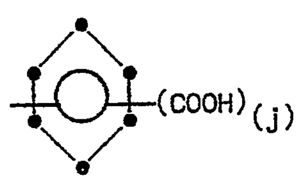

oder

iii) 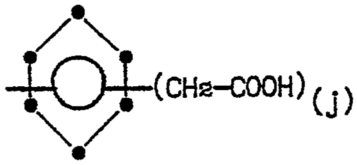

ist, oder

(e) eine Verbindung nach Anspruch 1, worin $R_1$, Z, $R_2$, $R_3$ und A wie im Anspruch 1 definiert sind und $R_4$ eine Gruppe der Formel

(i) –CN

bedeutet, mit einer wäßrigen Base zu einer Verbindung der Formel (I) hydrolysiert wird, worin $R_4$ eine primäre Amidgruppe der Formel

i) 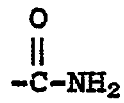 ;

ist, oder

(f) eine Verbindung nach Anspruch 1, worin $R_1$, Z, $R_2$, $R_3$ und A wie im Anspruch 1 definiert sind und $R_4$ für eine 5-Tetrazolylgruppe oder eine Phenylgruppe steht, die mit ein oder zwei (5-Tetrazolyl)-Gruppen substituiert ist, durch Umsetzung mit einem Alkylierungsmittel der Formel

$X-(CH_2)_q-J$

worin X eine gute Abgangsgruppe ist, wie eine Halogengruppe, und J ein Wasserstoffatom oder eine Gruppe der Formel –COOR″ bedeutet, worin R″ für eine $C_1$–$C_4$-Alkylgruppe steht, in Anwesenheit eines Säurebindemittels in eine Verbindung nach der Formel (I) überführt wird, worin $R_4$ eine Gruppe der Formeln

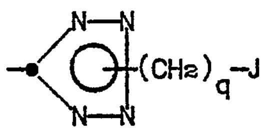

;

oder

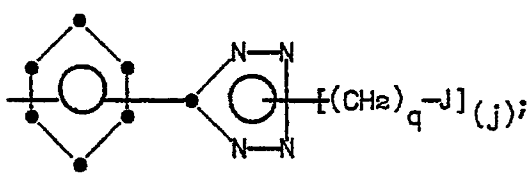

bedeutet, worin q wie oben definiert ist und j für 1 oder 2 steht, oder

(g) eine Verbindung nach Anspruch 1, worin $R_1$, $R_2$, $R_3$, Z und A wie im Anspruch 1 definiert sind und $R_4$ eine Gruppe der Formeln

(i) $-S(O)_{p-1}(C_1-C_4\text{-Alkyl})$ oder

(ii) 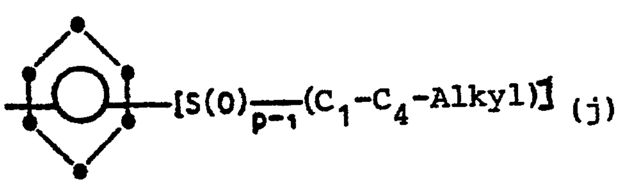

ist, zu einer Verbindung der Formel (I) oxidiert wird, worin $R_4$ eine Gruppe der Formeln

(i) $-S(O)_p-(C_1-C_4$-Alkyl) oder

ii) 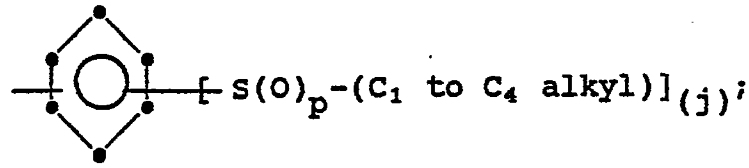

worin j für 1 oder 2 steht und p für 1 oder 2 steht, bedeutet, oder
(h) eine Verbindung der Formel (I), worin $R_1$, Z, $R_2$, $R_3$ und A wie oben definiert sind und $R_4$ eine Azidgruppe ist, zu den entsprechenden Verbindungen nach Anspruch 1 reduziert wird, worin $R_4$ eine primäre Aminogruppe bedeutet, oder
(i) Verbindungen eine Verbindung der Formel (I), worin $R_1$, Z, $R_2$, $R_3$ und A wie oben definiert sind und $R_4$ eine prmäre Aminogruppe oder eine primäre Amidogruppe ist, durch Umsetzung mit einem Acylierungsmittel der Formel

$CH_3(CH_2)_nCO-LG$

worin n für 1 bis 3 steht und LG Chlor, Brom oder Iod oder eine ein Säureanhydrid bildende Gruppe bedeutet, in Gegenwart eines Säurebindemittels in eine Verbindung nach Anspruch 1 überführt werden, worin $R_4$ für eine $C_2-C_4$-Alkanoylaminogruppe oder eine N-($C_2-C_4$-Alkanoyl)amidogruppe steht, oder
(j) eine Verbindung nach Anspruch 1, worin $R_1$, Z, $R_2$, $R_3$ und A wie im Anspruch 1 definiert sind und $R_4$ eine Gruppe der folgenden Formeln bedeutet

(i) $-COR_7$,

worin $R_7$ für $C_1-C_4$-Alkoxy steht,

ii) 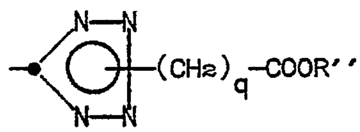

worin q wie oben definiert ist und R″ für $C_1-C_4$-Alkyl steht,

iii) 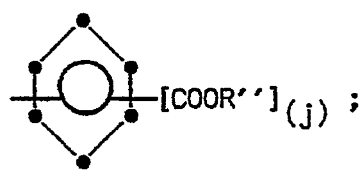

worin R″ für $C_1-C_4$-Alkyl steht und (j) für 1 oder 2 steht, oder

iv) 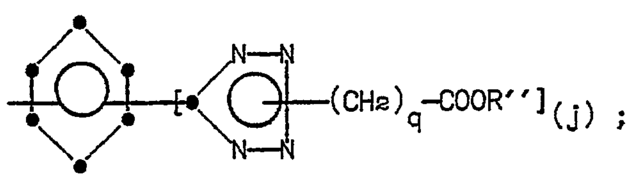

worin (j) für 1 oder 2 steht, q wie im Anspruch 1 definiert ist und R″ für $C_1-C_4$-Alkyl steht, durch Entesterung in Verbindungen der Formel (I) überführt wird, worin $R_4$ eine Gruppe der folgenden Formeln bedeutet

(i) $-COOH$

ii) 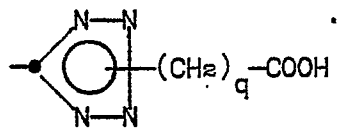

worin q für 1 bis 4 steht,

iii) 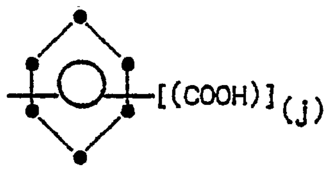 ;

worin (j) für 1 oder 2 steht, oder

iv) 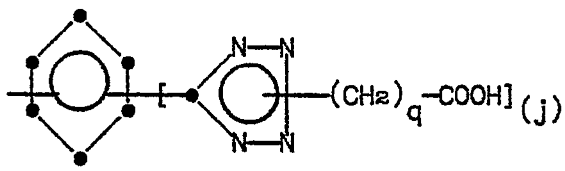

worin q für 1 bis 4 steht und (j) für 1 oder 2 steht,
(k) eine Verbindung der Formel (I), worin $R_1$, Z, $R_2$, $R_3$ und A wie oben definiert sind und $R_4$ eine Gruppe der folgenden Formeln bedeutet

(i) $-COOH$

ii) 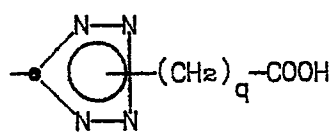 ;

worin q für 1 bis 4 steht,

iii) 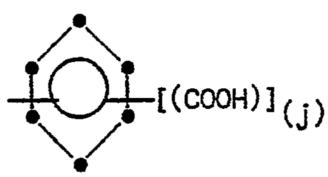 ;

worin (j) für 1 oder 2 steht, oder

iv) 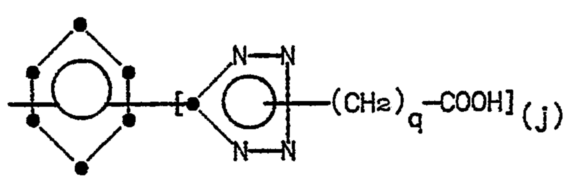

worin q für 1 bis 4 steht und (j) für 1 oder 2 steht,
durch Umsetzung mit einer Verbindung der Formel

$X-(CH_2)_d-CH_3$,

worin X eine gute Abgangsgruppe ist und d für 1 bis 3 steht, unter Entesterung in eine Verbindung der Formel (I) überführt wird, worin $R_4$ eine Gruppe der folgenden Formeln bedeutet

(i) $-COR_7$, worin $R_7$ für $C_1$–$C_4$-Alkoxy steht,

ii) 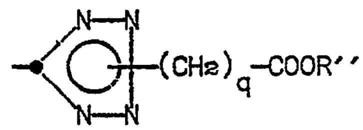

worin q wie im Anspruch 1 definiert ist und R″ für $C_1$–$C_4$-Alkyl steht,

iii) 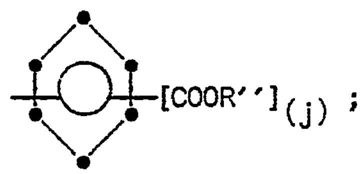 ;

worin R″ für $C_1$–$C_4$-Alkyl steht und (j) für 1 oder 2 steht, oder

iv) 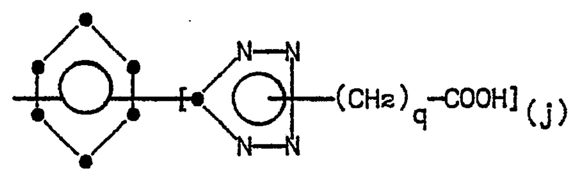 .

worin (j) für 1 oder 2 steht, q wie im Anspruch 1 definiert ist und R″ für $C_1$–$C_4$-Alkyl steht,
(l) eine Verbindung der Formel (I), worin $R_1$, Z, $R_3$, A und $R_4$ wie im Anspruch 1 definiert sind und $R_2$ eine Gruppe der Formel

$-O-(CH_2)_m-Y$

bedeutet, worin m für 1 steht und Y ein Wasserstoffatom ist, nach üblichen Demethylierungsbedingungen in eine Verbindung nach Anspruch 1 überführt wird, worin $R_2$ eine Hydroxygruppe ist, oder
(m) eine Verbindung nach Anspruch 1, worin $R_1$, $R_2$, $R_3$, Z und A wie im Anspruch 1 definiert sind und $R_4$ eine Gruppe der Formel $-COR_7$ ist, worin $R_7$ eine Hydroxygruppe bedeutet, durch Umsetzung mit einem Halogenierungsmittel, wie $POCl_3$, $POBr_3$, $PCl_5$, $PBr_5$, $SOCl_2$, $(ØO)_3PCH_3I$ und dergleichen, in eine Verbindung der Formel (I) überführt wird, worin $R_4$ eine Gruppe der Formel $-COR_7$ ist, worin $R_7$ eine Halogengruppe bedeutet, oder
(n) eine Verbindung nach Anspruch 1, worin $R_1$, $R_2$, $R_3$, Z und A wie oben definiert sind und $R_4$ Halogen ist, umgesetzt wird mit
(i) einem Alkalimetallcyanid unter Bildung einer Verbindung der Formel (I), worin $R_4$ Cyano ist, oder
(ii) einem primäre oder sekundären Amin der Formel $HNR_5R_6$ unter Bildung einer Verbindung der Formel (I), worin $R_4$ eine Gruppe der Formel $-NR_5R_6$ bedeutet, oder
(iv) einem Thiolatanion oder einem Thiol der Formel

$T-S(O)_p-(C_1-C_4\text{-Alkyl})$

worin T ein Wasserstoffatom oder ein Alkalimetallatom darstellt, unter Bildung einer Verbindung nach Anspruch 1, worin $R_4$ eine Gruppe der Formel

$-S(O)_p-(C_1-C_4\text{-Alkyl})$

bedeutet, worin p für 0 steht, oder
(o) eine Verbindung nach Anspruch 1, worin $R_1$, Z, $R_2$, $R_3$ und A wie oben definiert sind und $R_4$ eine Gruppe der Formel $-COR_7$ darstellt, worin $R_7$ eine Halogengruppe ist, umgesetzt wird mit
(i) einem Amin der Formel $HNR_5R_6$, worin $R_5$ und $R_6$ wie oben definiert sind, unter Bildung einer Verbindung, worin $R_7$ für $NR_5R_6$, worin für $NR_5R_6$ steht, oder
(ii) einem Hydroxylamin unter Bildung einer Verbindung der Formel (I), worin $R_7$ eine Gruppe der Formel $-NHOH$ ist, oder
(iii) ein 5-Aminotetrazol unter Bildung einer Verbindung der Formel (I), worin $R_7$ für ein 5-Aminotetrazol steht, das über das Stickstoffatom der Aminogruppe an die Acylgruppe gebunden ist, oder
(iv) einem $C_1$–$C_4$-Alkanol oder einem $C_1$–$C_4$-Alkoxid unter Bildung einer Verbindung nach Anspruch 1, worin $R_7$ für ein $C_1$–$C_4$-Alkoxy steht.

2. Verfahren zur Herstellung einer Verbindung nach Anspruch 1 der Formel (Ia)

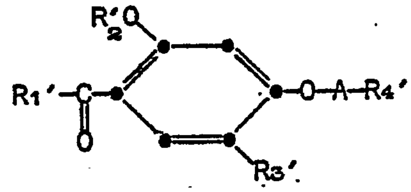

Ia

oder eines pharmazeutisch annehmbaren Salzes hiervon, worin
$R_1'$ Methyl oder Ethyl ist,
$R_2'$ Wasserstoff oder Methyl bedeutet,
$R_3'$ für Ethyl, Propyl, $-CH_2-S-CH_3$ oder Allyl steht und
$R_4'$ für Hydroxy, Cyano, $-COOR'$, $-CONR_5R_6$, $-CO(C_1-C_3\text{-Alkyl})$, $-S(O)_p-(C_1-C_4\text{-Alkyl})$, 5-Tetrazolyl, das gegebenenfalls durch eine $C_1-C_4$-Alkylgruppe substituiert ist, oder für Phenyl steht, das gegebenenfalls in meta-Stellung durch Halogen, Cyano, $C_1-C_3$-Alkyl, Trifluormethyl, $-CH_2CN$, $-CH_2Br$, $C_1-C_4$-Alkoxy, $-S(O)_p-(C_1-C_4\text{-Alkyl})$, Acetyl oder $-COOR'$ substituiert ist.

3. Verfahren zur Herstellung einer Verbindung der Formel (Ia) nach Anspruch 2, dadurch gekennzeichnet, daß die Verbindung 2-Hydroxy-4-[4-(2-methyl-2H-tetrazol-5-yl)butoxy]-5-ethylacetophenon, 2-Hydroxy-4-[6-(methylsulfonyl)hexyloxy]-5-ethylacetophenon oder 7-(4-Acetyl-2-ethyl-5-hydroxyphenoxy)-2,2-dimethylheptancarbonsäure oder ein pharmazeutisch annehmbares Salz hiervon ist.

4. Verfahren zur Herstellung von 3-[(4-Acetyl-2-ethyl-5-hydroxyphenoxy)methyl]benzonitril.

5. Verfahren zur Herstellung von 5-Ethyl-2-hydroxy-4-[6-methyl-6-(1H-tetrazol-5-yl)heptyloxy]acetophenon oder einem pharmazeutisch annehmbaren Salz hiervon.

6. Verfahren zur Herstellung der Verbindung von Anspruch 5, dadurch gekennzeichnet, daß
(a) ein Diphenol der Formel (V)

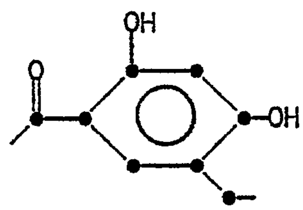

V

mit 1,5-Dibrompentan alkyliert und so eine Verbindung der Formel (VI)

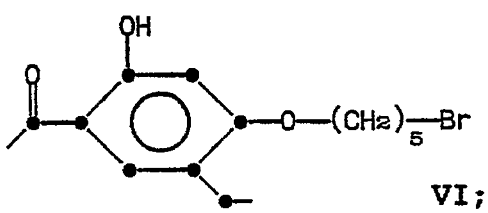

VI;

gebildet wird, oder
(b) die Verbindung der Formel (VI) mit Natrium-2-cyanopropan-2-at alkyliert und so eine Cyanoverbindung der Formel (VII)

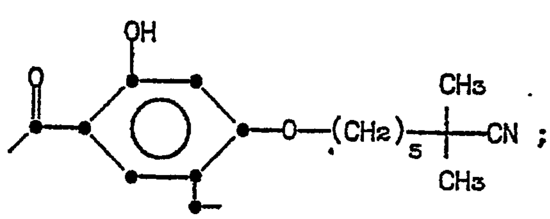

VII

gebildet wird, oder
(c) die Cyanoverbindung der Formel (VII) mit einem Azidreagens, wie Tri(n-butyl)zinnazid, umgesetzt und so eine Tetrazolverbindung der Formel (VIII)

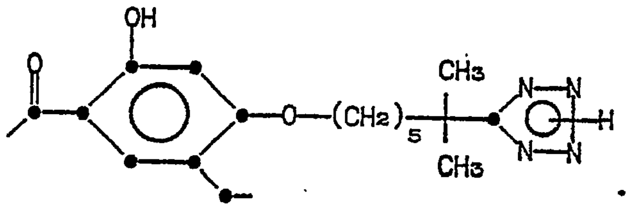

VIII

gebildet wird.

7. Verfahren zur Herstellung einer pharmazeutischen Formulierung, dadurch gekennzeichnet, daß eine Verbindung der Formel (I) oder ein pharmazeutisch annehmbares Salz hiervon nach einem der Ansprüche 1 bis 5 mit einem oder mehreren pharmazeutisch annehmbaren Trägern oder Hilfsstoffen hierfür vermischt wird.

**Revendications pour les Etats Contractants: BE, CH, DE, AT, FR, GB, IT, LI, LU, NL, SE**

1. Composé de formule I

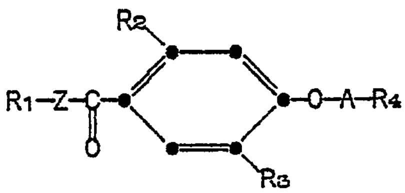

I

ou un de ses sels pharmaceutiquement acceptables, dans lesquels
$R_1$ représente un atome d'hydrogène ou R'–OOC–;
Z représente $-(CH_2)_n-$ ou un groupe phénylène;
n est égal à 1–8;
$R_2$ représente un groupe hydroxyle, un atome d'halogène, ou $-O-(CH_2)_m-Y$;
$R_3$ représente un groupe alkyle en $C_1$–$C_6$, un groupe alcanoyle en $C_1$–$C_6$, un groupe alcényle en $C_2$–$C_4$, un groupe alcoxy en $C_1$–$C_4$, un groupe alkyle en $C_1$–$C_3$ substitué par un groupe hydroxyle, ou $-CH_2-D$;
A est une liaison ou représente un groupe alkylidène en $C_1$–$C_{10}$ à chaîne droite ou ramifiée;
$R_4$ représente un groupe alkyle en $C_1$–$C_6$, un groupe alcényle en $C_2$–$C_6$, un groupe alcynyle en $C_2$–$C_6$, un groupe 1,2,4-triazol-1-yle, un groupe hydroxyle, –CN, un atome d'halogène, $-N_3$, $-NR_5R_6$, $-COR_7$, un groupe $-S(O)_p-$(alkyle en $C_1$–$C_4$), un groupe 5-tétrazolyle éventuellement substitué par un groupe alkyle en $C_1$–$C_4$ ou par $-(CH_2)_q-COOR'$, ou un groupe phényle éventuellement substitué par un ou deux groupes choisis parmi un atome d'halogène, un groupe cyano, un groupe alkyle en $C_1$–$C_3$, un groupe trifluorométhyle, $-CH_2CN$, $-CH_2Br$, un groupe alcoxy en $C_1$–$C_4$, un groupe $-S(O)_p-$(alkyle en $C_1$–$C_4$), un groupe acétyle, –COOR', un groupe 5-tétrazolyle, ou un groupe 5-tétrazolyle substitué par un groupe alkyle en $C_1$–$C_4$ ou par $-CH_2)_q-COOR'$;
où chaque radical R' représente indépendamment l'un de l'autre un atome d'hydrogène ou un groupe alkyle en $C_1$–$C_4$;
m est égal à 1–4;
q est égal à 1–4;
Y représente un atome d'hydrogène ou –CN;
D représente un atome d'halogène, un groupe alcoxy en $C_1$–$C_4$, ou un groupe –S–(alkyl en $C_1$–$C_4$);
$R_5$ et $R_6$ représentent chacun, indépendamment l'un de l'autre, un atome d'hydrogène, un groupe alkyle en $C_1$–$C_3$ ou un groupe alcynoyle en $C_2$–$C_4$, ou lorsqu'ils sont pris ensemble avec l'atome d'azote auquel ils sont fixés, forment un noyau morpholino;
$R_7$ représente un groupe hydroxyle, un groupe alcoxy en $C_1$–$C_4$, un atome d'halogène, $-NR_5R_6$, –NHOH,

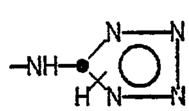 ,

où un groupe alkyle en $C_1$–$C_3$; et
chaque p est égal à 0, 1 ou 2;
avec cette réserve que, lorsque A représente une liaison, $R_4$ doit être un groupe alkyle en $C_1$–$C_6$ ou un

groupe phényle éventuellement substitué, et avec cette réserve supplémentaire que, lorsque'un des radicaux $R_5$ et $R_6$ représente un groupe alcanoyle en $C_2$–$C_4$, l'autre des radicaux $R_5$ et $R_6$ représente un atome d'hydrogène.

2. Composé selon la revendication 1, de formule Ia

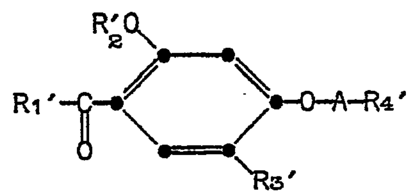

Ia

ou un de ses sels pharmaceutiquement acceptables, formule dans laquelle:
$R_1'$ représente un groupe méthyle ou un groupe éthyle;
$R_2'$ représente un atome d'hydrogène ou un groupe méthyle;
$R_3'$ représente un groupe éthyle, un groupe propyle, $-CH_2-S-CH_3$, ou un groupe allyle; et
$R_4'$ représente un groupe hydroxyle, un groupe cyano, –COOR', $-CONR_5R_6$, un groupe –CO(alkyl en $C_1$–$C_3$), un groupe $-S(O)_p$–(alkyl en $C_1$–$C_4$), un groupe 5-tétrazolyle éventuellement substitué par un groupe alkyle en $C_1$–$C_4$, ou un groupe phényle éventuellement substitué en position méta par un atome d'halogène, un groupe cyano, un groupe alkyle en $C_1$–$C_3$, un groupe trifluorométhyle, $-CH_2CN$, $-CH_2Br$, un groupe alcoxy en $C_1$–$C_4$, un groupe $-S(O)_p$–alkyle en $C_1$–$C_4$), un groupe acétyle, ou –COOR'.

3. Composé de formule Ia selon la revendication 2, ce composé étant la 2-hydroxy-4-[4-(2-méthyl-2H-tétrazol-5-yl)-butoxy]-5-éthyl-acétophénone, la 2-hydroxy-4-[6-(méthylsulfonyl)-hexyloxy]-5-éthylacétophénone ou l'acide 7-(4-acétyl-2-éthyl-5-hydroxyphénoxy)-2,2-diméthylheptanoïque, ou un de leurs sels pharmaceutiquement acceptables.

4. 3-[(4-acétyl-2-éthyl-5-hydroxyphénoxy)méthyl]benzonitrile.

5. 5-éthyl-2-hydroxy-4-[6-méthyl-6-(1H-tétrazol-5-yl)heptyloxy]acétophénone ou un de ses sels pharmaceutiquement acceptables.

6. Composition pharmaceutique comprenant un composé selon l'une quelconque des revendications 1 à 5, ou un sel pharmaceutiquement acceptable de cette composition, en association avec un ou plusieurs supports ou excipients pharmaceutiquement acceptables pour ce composé.

7. Composé de formule I selon l'une quelconque des revendications 1 à 5, ou un de ses sels pharmaceutiquement acceptables, destiné au traitement d'inflammations.

8. Procédé de préparation d'un composé de formule I selon l'une quelconque des revendications 1 à 5, ce procédé comprenant les étapes consistant à:

(a) acyler un composé de formule

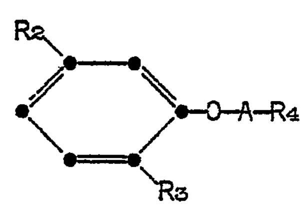

où $R_2$, $R_3$, $R_4$ et A ont les significations définies dans la formule I, dans des conditions de Friedel-Crafts, en vue de fournir un composé de formule I

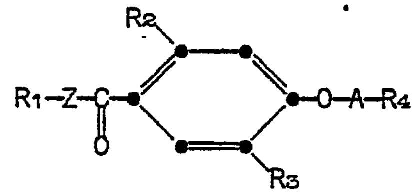

I

ou

(b) alkyler un phénol de formule

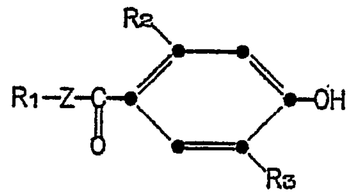

;

où $R_1$, $R_2$, $R_3$ et Z ont les significations définies dans la revendication 1, en présence d'une base forte suffisante pour générer l'anion du phénol; ou

(c) faire réagir un composé de formule selon la revendication I, où $R_1$, Z, $R_2$, $R_3$ et A ont les significations définies dans la revendication 1, et $R_4$ représente un groupe de formule:

i) –CN; ou

ii) 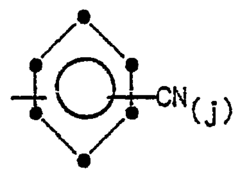 ;

avec un réactif azide pour obtenir un composé selon la revendication 1, où $R_4$ représente un groupe de formule

i) 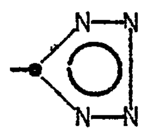

ou

ii) 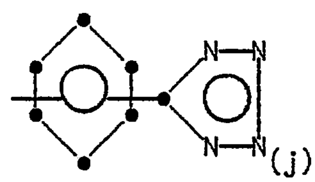 ;

(d) hydrolyser un composé selon la revendication 1, où $R_4$ représente un groupe de formule

i) –CN; ou

ii) 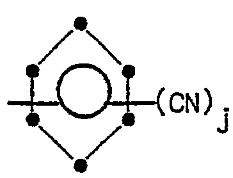 ;

ou

iii) 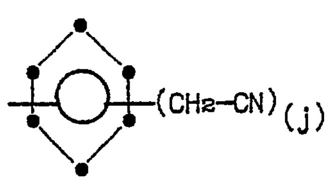 ;

pour obtenir un composé selon la revendication 1, où $R_4$ représente un groupe de formule

i) –COOH; ou

ii) 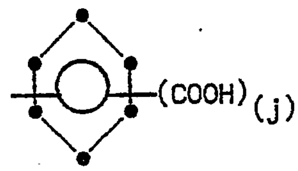

ou

iii) 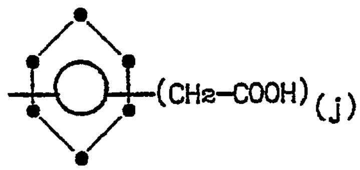

respectivement;
(e) hydrolyser, avec une base aqueuse, un composé selon la revendication 1, où $R_1$, Z, $R_2$, $R_3$, et A ont les significations définies dans la revendications 1 et $R_4$ représente un groupe de formule

i) –CN;

pour obtenir un composé de formule I selon la revendication 1, où $R_4$ représente un groupe amide primaire de formule

i) 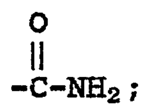

(f) alkyler un composé selon la revendication 1, où $R_1$, Z, $R_2$, $R_3$, et A ont les significations définies selon la revendication 1, et $R_4$ représente un groupe 5-tétrazolyle ou un groupe phényle substitué par un ou deux groupes (5-tétrazolyle); avec un agent d'alkylation de formule

$X–(CH_2)_q–J$

où X représente un bon groupe qui s'éloigne tel qu'un groupe halo et J représente un atome d'hydrogène ou un groupe de formule –COOR″, où R″ représente un groupe alkyle en $C_1$–$C_4$, en présence d'un agent fixateur d'acides, pour obtenir un composé selon la revendication 1, où $R_4$ représente un groupe de formule

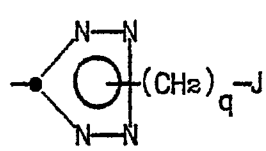

ou

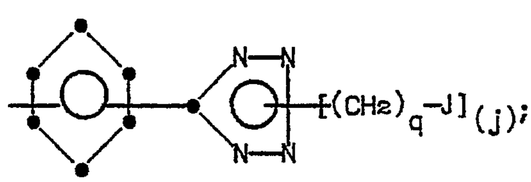

où q a la même signification telle que définie dans la revendication 1, et J représente un ou deux; ou
(g) oxyder un composé selon la revendication 1, où $R_1$, $R_2$, $R_3$, Z et A ont les significations telles que définies dans la revendication 1, et $R_4$ représente un groupe de formule

i) $-S(O)_{p-1}$(alkyle en $C_1$-$C_4$) ; ou

ii) 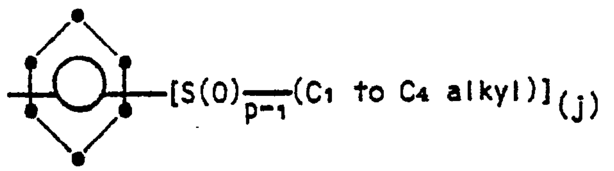

pour obtenir un composé selon la revendication 1, où $R_4$ représente un groupe de formule

i) $-S(O)_p-$(alkyle en $C_1$–$C_4$); ou

ii) 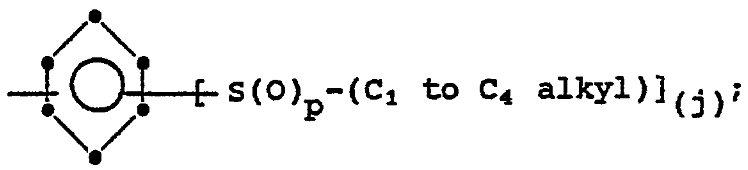

où j représente un ou deux et p représente un ou deux; ou
(h) réduire des composés selon la revendication 1, où $R_1$, Z, $R_2$, $R_3$ et A ont les significations telles que définies par la revendication 1, et $R_4$ représente un groupe azide, pour obtenir les composés correspondants selon la revendication 1, où $R_4$ représente un groupe amino primaire;
i) acyler les composés selon la revendication 1, où $R_1$, Z, $R_2$, $R_3$ et A ont les significations telles que définies dans la revendication 1, et $R_4$ représente un groupe amino primaire ou un groupe amino primaire, avec un agent d'acylation de formule

$CH_3(CH_2)_nCO–LG$

où n représente une valeur de 1 à 3 et "LG" représente un atom de chlore, un atome de brome ou un atome d'iode, ou un groupe formant un anhydride d'acide, en présence d'un agent fixateur d'acides, pour obtenir un composé selon la revendication 1, où $R_4$ représente un groupe (alcanoyl en $C_2$–$C_4$) amino ou un groupe N-(alcanoyl en $C_2$–$C_4$)amido, respectivement;
(j) désestérifier un composé selon la revendication 1, où $R_1$, Z, $R_2$, $R_3$ et A ont les significations telles que définies dans la revendication 1, et $R_4$ représente un groupe de formule
(i) $-COR_7$, où $R_7$ représente un groupe alcoxy en $C_1$–$C_4$:

ii) 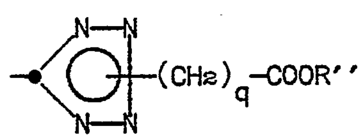

où q a la signification telle que définie dans la revendication 1 et R" représente un groupe alkyle en $C_1$–$C_4$;

iii) 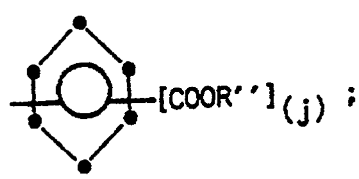

où R″ représente un groupe alkyle en $C_1$–$C_4$ et (j) représente un ou deux; ou

iv) 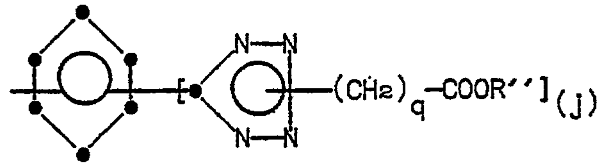 ;

où (j) représente un ou deux, q a la signification telle que définie dans la revendication 1 et R″ représente un groupe alkyle en $C_1$–$C_4$;
pour obtenir des composés selon la revendication 1, où $R_4$ représente un groupe de formule

i) –COOH;

ii) 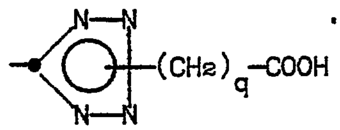

où q représente une valeur de un à quatre;

iii) 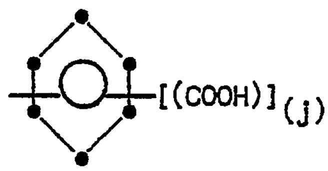 ;

où (j) représente un ou deux; ou

iv) 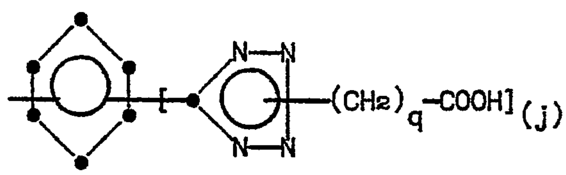

où q représente une valeur de un à quatre (j) représente un ou deux;
k) estérifier un composé selon la revendication 1, où $R_1$, Z, $R_2$, $R_3$, et A ont les significations telles que définies dans la revendication 1, et $R_4$ représente un groupe de formule

i) –COOH;

ii) 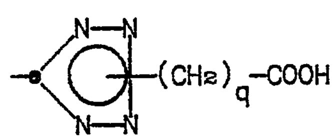 ;

où q représente une valeur de un à quatre

iii) 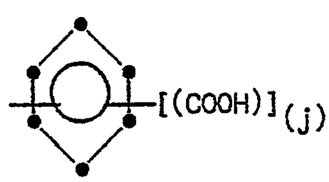 ;

où (j) représente un ou deux: ou

iv) 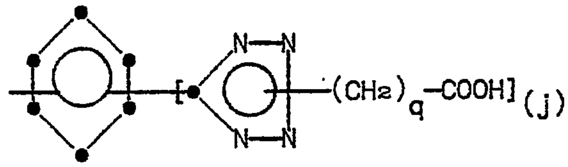

où q représente une valeur de un à quatre et (j) représente un ou deux;
en faisant réagir un composé de formule $X-(CH_2)_d-CH_3$ où X représente un bon groupe qui s'éloigne et d représente une valeur de un à trois, de façon à obtenir un composé selon la revendication 1, où $R_4$ représente un groupe de formule:

i) $-COR_7$, où $R_7$ représente un groupe alcoxy en $C_1-C_4$;

ii) 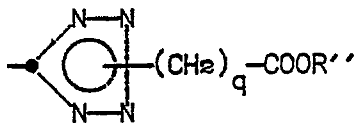

où q a la signification telle que définie par la revendication 1 et R″ représente un groupe alkyle en $C_1-C_4$;

iii) 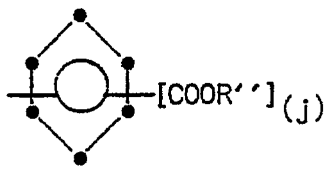 ;

où R″ représente un groupe alkyle en $C_1-C_4$ et (j) représente un ou deux; ou

iv) 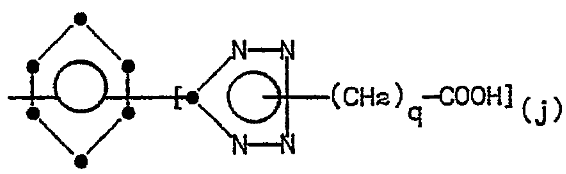

où (j) représente un ou deux, q a la signification telle que définie dans la revendication 1, et R″ représente un groupe alkyle en $C_1-C_4$;
(l) déméthyler un composé selon la revendication 1 où $R_1$, Z, $R_3$, A et $R_4$ ont les significations définies dans la revendication 1 et $R_2$ représente un groupe de formule

$-O-(CH_2)_m-Y$

où m représente un et y représente un atome d'hydrogène, dans des conditions classiques de déméthylation, pour obtenir un composé selon la revendication 1, où $R_2$ représente un groupe hydroxyle; ou
(m) faire réagir un composé selon la revendication 1, où $R_1$, $R_2$, $R_3$, Z, et A ont les significations définies dans la revendication 1 et $R_4$ représente un groupe de formule $-COR_7$, où $R_7$ représente un groupe de formule $-COR_7$, où $R_7$ représente un groupe hydroxyle, avec un réactif d'halogénation tel que $POCL_3$, $POBr_3$, $PCL_5$, $PBr_5$, $SOCl_2$, $(O)_3PCH_3I$, et analogues pour obtenir un composé selon la revendication 1, où $R_4$ représente un groupe de formule $-COR_7$, où $R_7$ représente un groupe de formule $-COR_7$, où $R_7$ représente un groupe halo;
(n) faire réagir un composé selon la revendication 1, où $R_1$, Z, $R_2$ et A ont les significations définies dans la revendication 1 et où $R_4$ représente un atome d'halogène, avec:

i) un cyanure de métal alcalin pour obtenir un composé selon la revendication 1, où $R_4$ représente un groupe cyano;
ii) une amine primaire ou secondaire de formule $HNR_5R_6$, pour obtenir un composé selon la revendication 1, où $R_4$ représente un groupe de formule $-NR_5R_6$; ou
iv) un anion thiolate ou un thiol de formule

$T-S(O)_p$-(alkyle en $C_1-C_4$)

où T représente un atome d'hydrogène ou un atome de métal alcalin, pour obtenir un composé selon la revendication 1, où $R_4$ représente un groupe de formule

$-S(O)_p-$(alkyle en $C_1-C_4$)

où p représente zéro; ou

(o) faire réagir un composé selon la revendication 1 où $R_1$, Z, $R_2$, et A ont les significations définies dans la revendication 1, et $R_4$ représente un groupe de formule $-COR_7$, où $R_7$ représente un groupe halo, avec:

i) une amine de formule $HNR_5R_6$ où $R_5$ et $R_6$ ont les significations définies dans la revendication 1, pour obtenir un composé dans lequel $R_7$ représente $NR_5R_6$; ou

ii) une hydroxylamine, pour obtenir un composé selon la revendication 1, où $R_7$ représente un groupe de formule $-NHOH$; ou

iii) un groupe 5-aminotétrazole, pour obtenir un compresé selon la revendication 1, ou R¨représente un groupe 5-aminotétrazole lié au groupe acyle via l'atome d'azote du groupe amino; ou

iv) un alcanol en $C_1-C_4$ ou un alcoxyde en $C_1-C_4$, pour obtenir un composé selon la revendication 1, où $R_7$ représente un groupe alcoxy en $C_1-C_4$.

9. Procédé pour la fabrication du composé selon la revendication 5, ce procédé consistant à:

(a) alkyler un diphénol de formule:

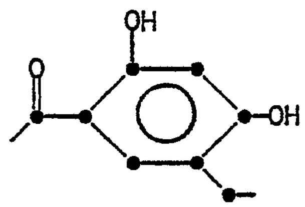

V

avec du 1,5-dibromopentane, pour obtenir un composé de formule

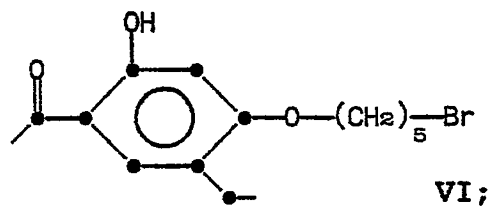

VI;

(b) alkyler le composé de formule VI avec du 2-cyanopropan-2-ate de sodium, pour obtenir un composé cyano de formule:

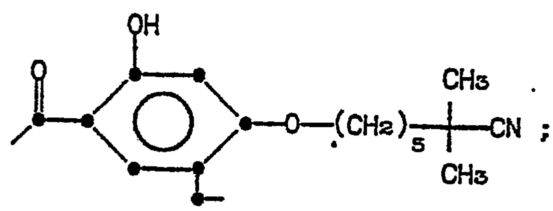

VII

(c) faire réagir le composé cyano de formule VII avec un réactif azide tel que l'azide de tri(n-butyl)étain, pour obtenir un composé tétrazole de formule:

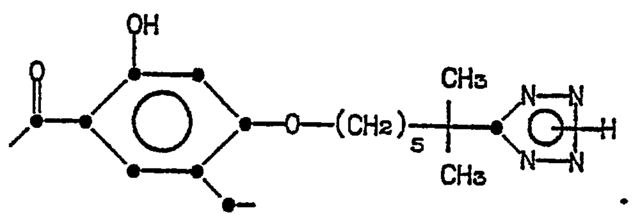

VIII

10. Composé de formule I selon la revendication 1, dans lequel $R_1$ représente un atome d'hydrogène, Z représente un groupe de formule $-(CH_2)n-$, où n représente 1, $R_2$ représente un groupe hydroxyle, A représente un groupe de formule:

$-(CH_2)_2-C(CH_3)_2-$

et $R_4$ représente un groupe cyano.

**Revendications pour les Etats Contractants: GR, ES**

1. Procédé de préparation d'un composé de formule I

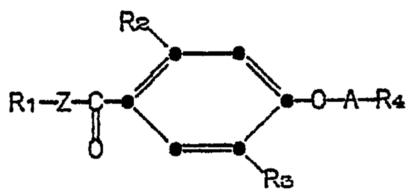

I

ou un de ses sels pharmaceutiquement acceptables, dans lesquels
$R_1$ représente un atome d'hydrogène ou R'–OOC–;
Z représente $-(CH_2)_n-$ ou un groupe phénylène;
n est égal à 1–8;
$R_2$ représente un groupe hydroxyle, un atome d'halogène, ou $-O-(CH_2)_m-Y$;
$R_3$ représente un groupe alkyle en $C_1$–$C_6$, un groupe alcanoyle en $C_1$–$C_6$, un groupe alcényle en $C_2$–$C_4$, un groupe alcoxy en $C_1$–$C_4$, un groupe alkyle en $C_1$–$C_3$ substitué par un groupe hydroxyle, ou $-CH_2-D$;
A est une liaison ou représente un groupe alkylidène en $C_1$–$C_{10}$ à chaîne droite ou ramifiée;
$R_4$ représente un groupe alkyle en $C_1$–$C_6$, un groupe alcényle en $C_2$–$C_6$, un groupe alcynyle en $C_2$–$C_6$, un groupe 1,2,4-triazol-1-yle, un groupe hydroxyle, –CN, un atome d'halogène, $-N_3$, $-NR_5R_6$, $-COR_7$, un groupe $-S(O)_p-$(alkyle en $C_1$–$C_4$), un groupe 5-tétrazolyle éventuellement substitué par un groupe alkyle en $C_1$–$C_4$ ou par $-(CH_2)_q-COOR'$, ou un groupe phényle éventuellement substitué par un ou deux groupes choisis parmi un atome d'halogène, un groupe cyano, un groupe alkyle en $C_1$–$C_3$, un groupe trifluorométhyle, $-CH_2CN$, $-CH_2Br$, un groupe alcoxy en $C_1$–$C_4$, un groupe $-S(O)_p-$(alkyle en $C_1$–$C_4$), un groupe acétyle, –COOR', un groupe 5-tétrazolyle, ou un groupe 5-tétrazolyle substitué par un groupe alkyle en $C_1$–$C_4$ ou par $-CH_2)_q-COOR'$;
où chaque radical R' représente indépendamment l'un de l'autre un atome d'hydrogène ou un groupe alkyle en $C_1$–$C_4$;
m est égal à 1–4;
q est égal à 1–4;
Y représente un atome d'hydrogène ou –CN;
D représente un atome d'halogène, un groupe alcoxy en $C_1$–$C_4$, ou un groupe –S–(alkyl en $C_1$–$C_4$);
$R_5$ et $R_6$ représentent chacun, indépendamment l'un de l'autre, un atome d'hydrogène, un groupe alkyle en $C_1$–$C_3$ ou un groupe alcynoyle en $C_2$–$C_4$, ou lorsqu'ils sont pris ensemble avec l'atome d'azote auquel ils sont fixés, forment un noyau morpholino;
$R_7$ représente un groupe hydroxyle, un groupe alcoxy en $C_1$–$C_4$, un atome d'halogène, $-NR_5R_6$, –NHOH,

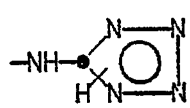,

ou un groupe alkyle en $C_1$–$C_3$; et
chaque p est égal à 0, 1 ou 2;
avec cette réserve que, lorsque A représente une liaison, $R_4$ doit être un groupe alkyle en $C_1$–$C_6$ ou un groupe phényle éventuellement substitué, et avec cette réserve supplémentaire que, lorsque'un des radicaux $R_5$ et $R_6$ représente un groupe alcanoyle en $C_2$–$C_4$, l'autre des radicaux $R_5$ et $R_6$ représente un atome d'hydrogène,
ce procédé consistant à:
(a) acyler un composé de formule

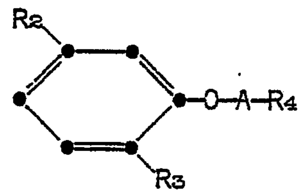

où $R_2$, $R_3$, $R_4$ et A ont les significations définies dans la formule I, dans des conditions de Friedel-Crafts, en vue de fournir un composé de formule I

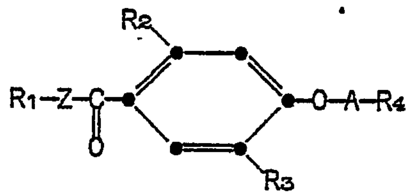 I

ou

(b) alkyler un phénol de formule

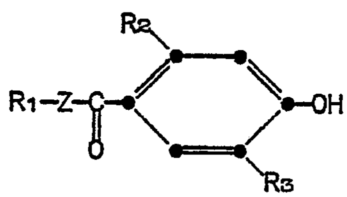 ;

où $R_1$, $R_2$, $R_3$ et Z ont les significations définies dans la revendication 1, en présence d'une base forte suffisante pour générer l'anion du phénol; ou

(c) faire réagir un composé de formule selon la revendication 1, où $R_1$, Z, $R_2$, $R_3$ et A ont les significations définies dans la revendication 1, et $R_4$ représente un groupe de formule:

i) –CN; ou

ii) 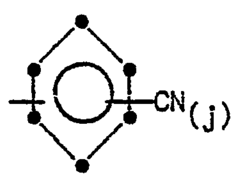 ;

avec un réactif azide pour obtenir un composé selon la revendication 1, où $R_4$ représente un groupe de formule

i) 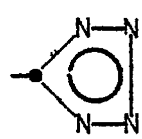

ou

ii) 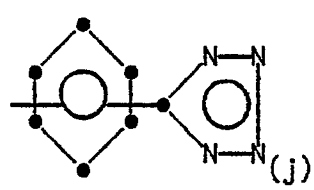 ;

(d) hydrolyser un composé selon la revendication 1, où $R_4$ représente un groupe de formule

i) –CN; ou

ii) 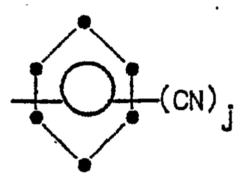 ;

ou

iii) 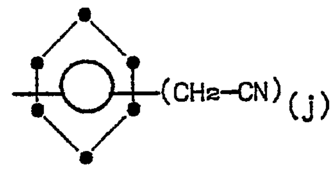 ;

pour obtenir un composé selon la revendication 1, où $R_4$ représente un groupe de formule

i) –COOH; ou

ii) 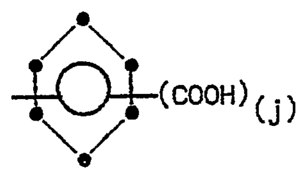

ou

iii) 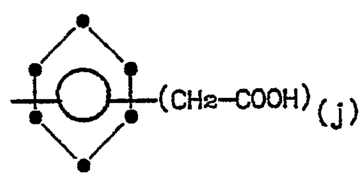

respectivement;

(e) hydrolyser, avec une base aqueuse, un composé selon la revendication 1, où $R_1$, Z, $R_2$, $R_3$, et A ont les significations définies dans la revendication 1 et $R_4$ représente un groupe de formule

i) –CN;

pour obtenir un composé de formule I selon la revendication 1, où $R_4$ représente un groupe amide primaire de formule

i) 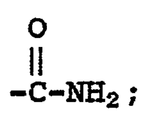 ;

(f) alkyler un composé selon la revendication 1, où $R_1$, Z, $R_2$, $R_3$, et A ont les significations définies selon la revendication 1, et $R_4$ représente un groupe 5-tétrazolyle ou un groupe phényle substitué par un ou deux groupes (5-tétrazolyle); avec un agent d'alkylation de formule

$X{-}(CH_2)_q{-}J$

où X représente un bon groupe qui s'éloigne tel qu'un groupe halo et J représente un atome d'hydrogène ou un groupe de formule –COOR", où R" représente un groupe alkyle en $C_1$–$C_4$, en présence d'un agent fixateur d'acides, pour obtenir un composé selon la revendication 1, où $R_4$ représente un groupe de formule

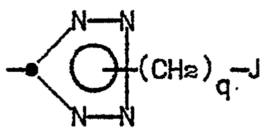

ou

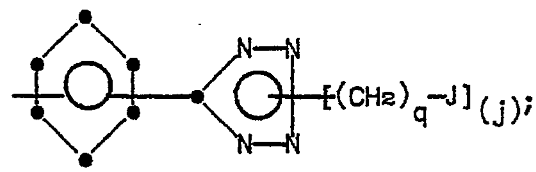

où q a la même signification telle que définie dans la revendication 1, et J représente un ou deux; ou
(g) oxyder un composé selon la revendication 1, où $R_1$, $R_2$, $R_3$, Z et A ont les significations telles que définies dans la revendication 1, et $R_4$ représente un groupe de formule

i) $-S(O)_{p-1}$(alkyle en $C_1$-$C_4$) ; ou

ii) 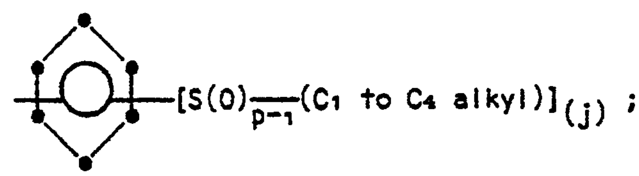

pour obtenir un composé selon la revendication 1, où $R_4$ représente un groupe de formule

i) $-S(O)_p-$(alkyle en $C_1$–$C_4$); ou

ii) 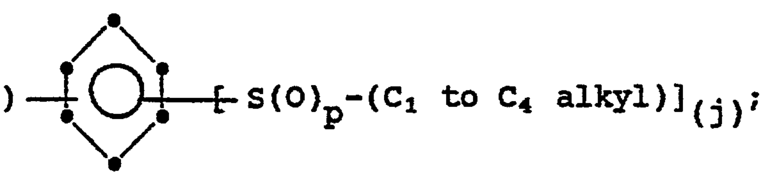

où j représente un ou deux et p représente un ou deux; ou
(h) réduire des composés selon la revendication 1, où $R_1$, Z, $R_2$, $R_3$ et A ont les significations telles que définies par la revendication 1, et $R_4$ représente un groupe azide, pour obtenir les composés correspondants selon la revendication 1, où $R_4$ représente un groupe amino primaire;
i) acyler les composés selon la revendication 1, où $R_1$, Z, $R_2$, $R_3$ et A ont les significations telles que définies dans la revendication 1, et $R_4$ représente un groupe amino primaire ou un groupe amino primaire, avec un agent d'acylation de formule

$CH_3(CH_2)_nCO$–LG

où n représente une valeur de 1 à 3 et "LG" représente un atome de chlore, un atome de brome ou un atome d'iode, ou un groupe formant un anhydride d'acide, en présence d'un agent fixateur d'acides, pour obtenir un composé selon la revendication 1, où $R_4$ représente un groupe (alcanoyl en $C_2$–$C_4$) amino ou un groupe N-(Alcanoyl en $C_2$–$C_4$)amido, respectivement;
(j) désestérifier un composé selon la revendication 1, où $R_1$, Z, $R_2$, $R_3$ et A ont les significations telles que définies dans la revendication 1, et $R_4$ représente un groupe de formule

i) $-COR_7$, où $R_7$ représente un groupe alcoxy en $C_1$–$C_4$;

ii) 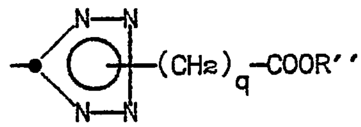

où q a la signification telle que définie dans la revendication 1 et R″ représente un groupe alkyle en $C_1$–$C_4$;

iii) 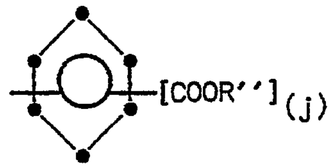 ;

où R″ représente un groupe alkyle en $C_1$–$C_4$ et (j) représente un ou deux; ou

iv) 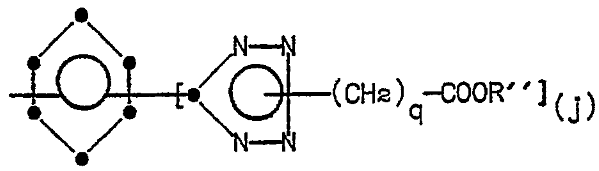

où (j) représente un ou deux, q a la signification telle que définie dans la revendication telle que définie dans la revendication 1 et R″ représente un groupe alkyle en $C_1$–$C_4$;
pour obtenir des composés selon la revendication 1, où $R_4$ représente un groupe de formule

i) –COOH;

ii) 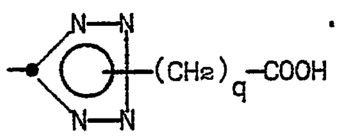

où q représente une valeur de un à quatre;

iii) 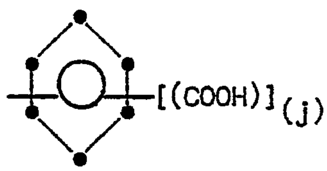 ;

où (j) représente un ou deux; ou

iv) 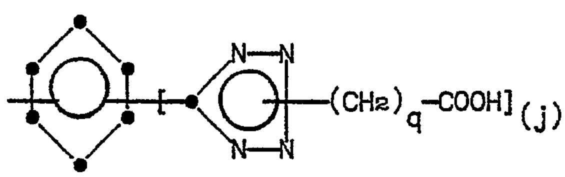

où q représente une valeur de un à quatre (j) représente un ou deux;
(k) estérifier un composé selon la revendication 1, où $R_1$, Z, $R_2$, $R_3$, et A ont les significations telles que définies dans la revendication 1, et $R_4$ représente un groupe de formule
i) –COOH;

ii) 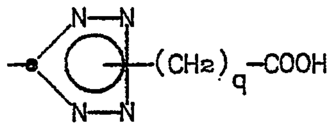 ;

où représente une valeur de un à quatre

iii) 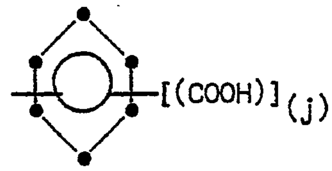 ;

où (j) représente un ou deux: ou

iv) 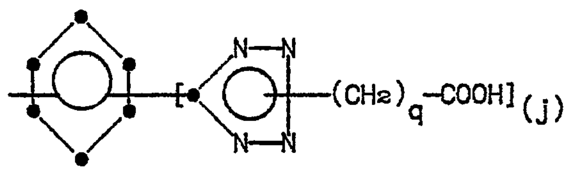

où q représente une valeur de un à quatre et (j) représente un ou deux; en faisant réagir un composé de formule $X-(CH_2)_d-CH_3$ où X représente un bon groupe qui s'éloigne et d représente une valeur de un à trois, de façon à obtenir un composé selon la revendication 1, où $R_4$ représente un groupe de formule:

i) $-COR_7$, où $R_7$ représente un groupe alcoxy en $C_1-C_4$;

ii) 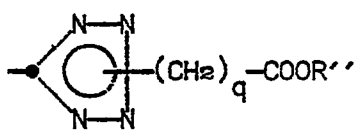

où q a la signification telle que définie par la revendication 1 et R″ représente un groupe alkyle en $C_1-C_4$;

iii) 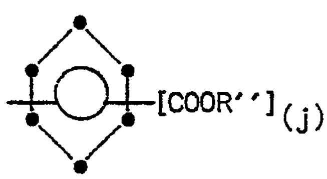 ;

où R″ représente un groupe alkyle en $C_1-C_4$ et (j) représente un ou deux; ou

iv) 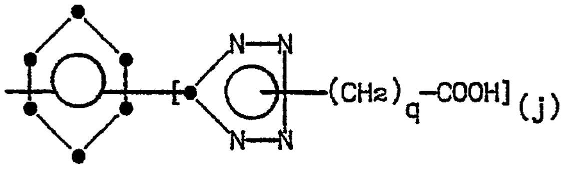

où (j) représente un ou deux, q a la signification telle que définie dans la revendication 1, et R″ représente un groupe alkyle en $C_1-C_4$;
(l) déméthyler un composé selon la revendication 1 où $R_1$, Z, $R_3$, A et $R_4$ ont les significations définies dans la revendication 1 et $R_2$ représente un groupe de formule
$-O-(CH_2)_m-Y$

où m représente un et y représente un atome d'hydrogène, dans des conditions classiques de déméthylation, pour obtenir un composé selon la revendication 1, où $R_2$ représente un groupe hydroxyle; ou (m) faire réagir un composé selon la revendication 1, où $R_1$, $R_2$, $R_3$, Z, et A ont les significations définies dans la revendication 1 et $R_4$ représente un groupe de formule $-COR_7$, où $R_7$ représente un groupe de formule $-COR_7$, où $R_7$ représente un groupe hydroxyle, avec un réactif d'halogénation tel que $POCL_3$, $POBr_3$, $PCL_5$, $PBr_5$, $SOCl_2$, $(O)_3PCH_3I$, et analogues pour obtenir un composé selon la revendication 1, où $R_4$ représente un groupe de formule $-COR_7$, où $R_7$ représente un groupe de formule $-COR_7$, où $R_7$ représente un groupe halo;

(n) faire réagir un composé selon la revendication 1, où $R_1$, Z, $R_2$ et A ont les significations définies dans la revendication 1 et où $R_4$ représente un atome d'halogène, avec:

i) un cyanure de métal alcalin pour obtenir un composé selon la revendication 1, où $R_4$ représente un groupe cyano;

ii) une amine primaire ou secondaire de formule $HNR_5R_6$, pour obtenir un composé selon la revendication 1, où $R_4$ représente un groupe de formule $-NR_5R_6$; ou

iv) un anion thiolate ou un thiol de formule

$T-S(O)_p-$(alkyle en $C_1-C_4$)

où T représente un atome d'hydrogène ou un atome de métal alcalin, pour obtenir un composé selon la revendication 1, où $R_4$ représente un groupe de formule

$-S(O)_p-$(alkyle en $C_1-C_4$)

où p représente zéro; ou

(o) faire réagir un composé selon la revendication 1 où $R_1$, Z, $R_2$, et A ont les significations définies dans la revendication 1, et $R_4$ représente un groupe de formule $-COR_7$, où $R_7$ représente un groupe halo, avec:

i) une amine de formule $HNR_5R_6$ où $R_5$ et $R_6$ ont les significations définies dans la revendication 1, pour obtenir un composé dans lequel $R_7$ représente $NR_5R_6$; ou

ii) une hydroxylamine, pour obtenir un composé selon la revendication 1, où $R_7$ représente un groupe de formule $-NHOH$; ou

iii) un groupe 5-aminotétrazole lié au groupe acyle via l'atome d'azote du groupe amino; ou

iv) un alcanol en $C_1-C_4$ ou un alcoxyde en $C_1-C_4$, pour obtenir un composé selon la revendication 1, où $R_7$ représente un groupe alcoxy en $C_1-C_4$.

2. Procédé de préparation d'un composé selon la revendication 1, de formule Ia

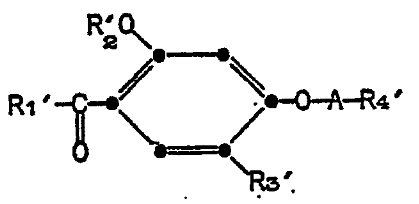

Ia

où un de ses sels pharmaceutiquement acceptables, formule dans laquelle:
$R_1'$ représente un groupe méthyle ou un groupe éthyle;
$R_2'$ représente un atome d'hydrogène ou un groupe méthyle;
$R_3'$ représente un groupe éthyle, un groupe propyle, $-CH_2-S-CH_3$, ou un groupe allyle; et
$R_4'$ représente un groupe hydroxyle, un groupe cyano, $-COOR'$, $-CONR_5R_6$, un groupe $-CO$(alkyl en $C_1-C_3$), un groupe $-S(O)_p-$(alkyl en $C_1-C_4$), un groupe 5-tétrazolyle éventuellement substitué par un groupe alkyle en $C_1-C_4$, ou un groupe phényle éventuellement substitué en position méta par un atome d'halogène, un groupe cyano, un groupe alkyle en $C_1-C_3$, un groupe trifluorométhyle, $-CH_2CN$, $-CH_2Br$, un groupe alcoxy en $C_1-C_4$, un groupe $-S(O)_p$-alkyle en $C_1-C_4$), un groupe acétyle, ou $-COOR'$.

3. Procédé de préparation d'un composé de formule Ia selon la revendication 2, ce composé étant la 2-hydroxy-4-[4-(2-méthyl-2H-tétrazol-5-yl)-butoxy]-5-éthyl-acétophénone, la 2-hydroxy-4-[6-(méthylsulfonyl)-hexyloxy]-5-éthylacétophénone ou l'acide 7-(4-acétyl-2-éthyl-5-hydroxyphénoxy)-2,2-diméthylheptanoïque, ou un de leurs sels pharmaceutiquement acceptables.

4. Procédé de préparation du 3-[(4-acétyl-2-éthyl-5-hydroxyphénoxy)méthyl]benzonitrile.

5. Procédé de préparation de la 5-éthyl-2-hydroxy-4-[6-méthyl-6-(1H-tétrazol-5-yl)-heptyloxy]acétophénone ou d'un de ses sels pharmaceutiquement acceptables.

6. Procédé pour la préparation du composé selon la revendication 5, ce procédé consistant à:
(a) alkyler un diphénol de formule:

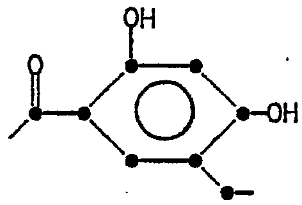

V

avec du 1,5-dibromopentane, pour obtenir un composé de formule

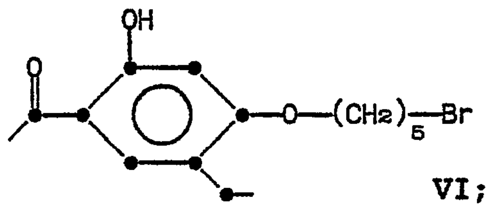

VI;

(b) alkyler le composé de formule VI avec du 2-cyanopropan-2-ate de sodium, pour obtenir un composé cyano de formule

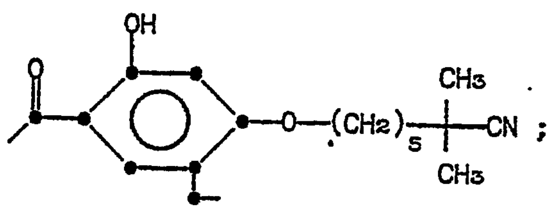

VII

(c) faire réagir le composé cyano de formule VII avec un réactif azide tel que l'azide de tri(n-butyl)étain, pour obtenir un composé tétrazole de formule:

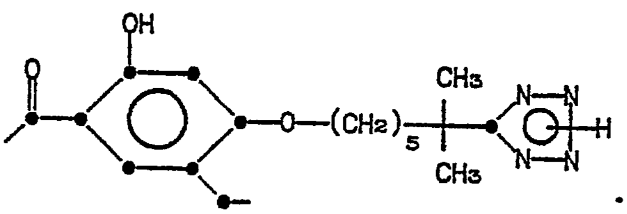

VIII

7. Procédé de préparation d'une formulation pharmaceutique, ce procédé consistant à mélanger un composé de formule I, ou un de ses sels pharmaceutiquement acceptables, tel que défini dans l'une quelconque des revendications 1 à 5, avec un ou plusieurs supports ou excipients pharmaceutiquement acceptables pour ce composé.